(19) Europäisches Patentamt
European Patent Office
Office européen des brevets

(11) EP 3 155 096 B1

(12) # EUROPEAN PATENT SPECIFICATION

(45) Date of publication and mention of the grant of the patent:
**17.03.2021 Bulletin 2021/11**

(21) Application number: **15728011.6**

(22) Date of filing: **12.06.2015**

(51) Int Cl.:
***C12N 9/28*** *(2006.01)* ***C11D 3/386*** *(2006.01)*

(86) International application number:
**PCT/EP2015/063132**

(87) International publication number:
**WO 2015/189370 (17.12.2015 Gazette 2015/50)**

(54) **OXIDATION STABLE ALPHA-AMYLASE VARIANTS**

OXIDATIONSSTABILE ALPHA-AMYLASE

ALPHA-AMYLASE STABLE À L'OXYDATION

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **12.06.2014 US 201462011564 P**
**08.05.2015 EP 15166870**

(43) Date of publication of application:
**19.04.2017 Bulletin 2017/16**

(73) Proprietor: **Novozymes A/S**
**2880 Bagsvaerd (DK)**

(72) Inventors:
- **ANDERSEN, Carsten**
**DK-2880 Bagsvaerd (DK)**
- **HOUG, Anne, Dorte**
**DK-2880 Bagsvaerd (DK)**
- **LARSEN, Signe, Eskildsen**
**DK-2880 Bagsvaerd (DK)**
- **FALLAH-ARAGHI, Ali**
**DK-2880 Bagsvaerd (DK)**

(74) Representative: **NZ EPO Representatives**
**Krogshoejvej 36**
**2880 Bagsvaerd (DK)**

(56) References cited:
**WO-A1-02/42740** **WO-A1-94/18314**
**WO-A1-96/23873** **WO-A1-2009/102854**
**WO-A2-00/60058**

Remarks:
The file contains technical information submitted after the application was filed and not included in this specification

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 155 096 B1

## Description

### Reference to a Sequence Listing

This patent contains a Sequence Listing.

## BACKGROUND OF THE INVENTION

### Field of the Invention

The present invention relates to the use of an alpha-amylase variant of a parent alpha-amylase in a cleaning process; a composition comprising oxidation stable alpha-amylase variants; and methods for removing a stain from a surface using the composition of the invention.

### Description of the Related Art

Alpha-amylases (alpha-1,4-glucan-4-glucanohydrolases, E.C. 3.2.1.1) constitute a group of enzymes, which catalyses hydrolysis of starch and other linear and branched 1,4-gluosidic oligo- and polysaccharides.

Alpha-amylase is a key enzyme for use in detergent compositions and its use has become increasingly important for removal of starchy stains during laundry washing or dishwashing.

Some detergents, in particular dishwashing detergents, comprise bleaching systems, bleach activators, and bleach catalysts which are all very destabilizing for the alpha-amylases due to oxidation of the molecules. Therefore, it is important to find alpha-amylase variants, which are stable, have high wash performance, stain removal effect and/or activity in detergents comprising various bleaching agents.

It is known in the art to stabilize alpha-amylases towards bleaching agents and oxidation by substituting the methionine at position 197 (using the amylase from *B. licheniformis* for numbering) with *e.g.* leucine. This has *e.g.* been disclosed in WO199418314. However, these prior art oxidation stable alpha-amylases have the disadvantage that the alpha-amylase activity is reduced.

WO 00/60058A2 discloses alpha-amlase variants with increased oxidation stability having a modification at position M202, including substitution M202L.

WO 2009/102854A1 concerns cleaning compositions comprising a protease and an alpha-amylase variant, wherein the alpha-amylase variant has the amino acids at positions 183 and 184 deleted and further has N195F and M202L substitutions.

WO 94/18314A1 discloses an oxidation stable alpha-amylase variant for use in detergents, wherein the alpha-amylase variant has a M197L substitution, which corresponds to a M202L substitution in the mature AAI10 alpha-amylase, i.e., SEQ ID NO: 3 of the present patent.

WO 96/23873A1 concerns improving oxidation stable alpha-amylases with an M202 substitution by pairwise deletion of amino acids in the 181-184 region.

Thus, it is an object of the present invention to provide the use of alpha-amylase variants that exhibit a high level of stability in detergents, in particular in dishwashing detergents and other detergents comprising bleaching agents or systems but at the same time have improved wash performance compared to the parent alpha-amylase.

## SUMMARY OF THE INVENTION

The present invention relates to the use of an alpha-amylase variant of a parent alpha-amylase in a cleaning process, wherein a bleaching system is added in a concentration of at least 5 weight%, wherein said parent has an amino acid sequence which is at least 80% identical to the amino acid sequence set forth in SEQ ID NO: 3, wherein said variant comprises a substitution in one or more positions providing oxidation stability of said variant, wherein said variant comprises a substitution in position 202, wherein said position corresponds to the amino acid position of the amino acid sequence as set forth in SEQ ID NO: 3, wherein said substitution is a M202L substitution, wherein said variant has a sequence identity of at least 80%, but less than 100% to the amino acid sequence set forth in SEQ ID NO: 3, wherein said variant has an improvement factor of >1.0 as a measure for wash performance, when compared to said parent alpha-amylase, wherein said variant has alpha-amylase activity.

The present invention also relates to a composition comprising alpha-amylase variants of SEQ ID NO: 3 as defined herein further comprises one or more surfactants and one or more bleaching systems and one or more builders.

Further, the present invention also relates to methods for removing a stain from a surface comprising contacting the surface with a composition of the invention.

## Conventions for Designation of Variants

For purposes of the present invention, the amino acid sequence as set forth in SEQ ID NO: 3 is used to determine the corresponding amino acid residue in another alpha-amylase. The amino acid sequence of another alpha-amylase is aligned with the amino acid sequence set forth in SEQ ID NO: 3, and based on the alignment, the amino acid position number corresponding to any amino acid residue in the amino acid sequence as set forth in SEQ ID NO: 3 is determined using the Needleman-Wunsch algorithm (Needleman and Wunsch, 1970, J. Mol. Biol. 48: 443-453) as implemented in the Needle program of the EMBOSS package (EMBOSS: The European Molecular Biology Open Software Suite, Rice et al., 2000, Trends Genet. 16: 276-277), preferably version 5.0.0 or later. The parameters used are gap open penalty of 10, gap extension penalty of 0.5, and the EBLOSUM62 (EMBOSS version of BLOSUM62) substitution matrix.

Identification of the corresponding amino acid residue in another alpha-amylase may be determined by an alignment of multiple polypeptide sequences using several computer programs including, but not limited to, MUSCLE (multiple sequence comparison by log-expectation; version 3.5 or later; Edgar, 2004, Nucleic Acids Research 32: 1792-1797), MAFFT (version 6.857 or later; Katoh and Kuma, 2002, Nucleic Acids Research 30: 3059-3066; Katoh et al., 2005, Nucleic Acids Research 33: 511-518; Katoh and Toh, 2007, Bioinformatics 23: 372-374; Katoh et al., 2009, Methods in Molecular Biology 537:_39-64; Katoh and Toh, 2010, Bioinformatics 26:_1899-1900), and EMBOSS EMMA employing ClustalW (1.83 or later; Thompson et al., 1994, Nucleic Acids Research 22: 4673-4680), using their respective default parameters.

When the other enzyme has diverged from the amino acid sequence as set forth in SEQ ID NO: 3 such that traditional sequence-based comparison fails to detect their relationship (Lindahl and Elofsson, 2000, J. Mol. Biol. 295: 613-615), other pairwise sequence comparison algorithms can be used. Greater sensitivity in sequence-based searching can be attained using search programs that utilize probabilistic representations of polypeptide families (profiles) to search databases. For example, the PSI-BLAST program generates profiles through an iterative database search process and is capable of detecting remote homologs (Atschul et al., 1997, Nucleic Acids Res. 25: 3389-3402). Even greater sensitivity can be achieved if the family or superfamily for the polypeptide has one or more representatives in the protein structure databases. Programs such as GenTHREADER (Jones, 1999, J. Mol. Biol. 287: 797-815; McGuffin and Jones, 2003, Bioinformatics 19: 874-881) utilize information from a variety of sources (PSI-BLAST, secondary structure prediction, structural alignment profiles, and solvation potentials) as input to a neural network that predicts the structural fold for a query sequence. Similarly, the method of Gough et al., 2000, J. Mol. Biol. 313: 903-919, can be used to align a sequence of unknown structure with the superfamily models present in the SCOP database. These alignments can in turn be used to generate homology models for the polypeptide, and such models can be assessed for accuracy using a variety of tools developed for that purpose.

For proteins of known structure, several tools and resources are available for retrieving and generating structural alignments. For example the SCOP superfamilies of proteins have been structurally aligned, and those alignments are accessible and downloadable. Two or more protein structures can be aligned using a variety of algorithms such as the distance alignment matrix (Holm and Sander, 1998, Proteins 33: 88-96) or combinatorial extension (Shindyalov and Bourne, 1998, Protein Engineering 11: 739-747), and implementation of these algorithms can additionally be utilized to query structure databases with a structure of interest in order to discover possible structural homologs (*e.g.*, Holm and Park, 2000, Bioinformatics 16: 566-567).

In describing the variants, the nomenclature described below is adapted for ease of reference. The accepted IUPAC single letter or three letter amino acid abbreviation is employed.

Substitutions. For an amino acid substitution, the following nomenclature is used: Original amino acid, position, substituted amino acid. Accordingly, the substitution of threonine at position 226 with alanine is designated as "Thr226Ala" or "T226A". Multiple mutations are separated by addition marks ("+"), *e.g.*, "Gly205Arg + Ser411Phe" or "G205R + S411F", representing substitutions at positions 205 and 411 of glycine (G) with arginine (R) and serine (S) with phenylalanine (F), respectively.

Deletions. For an amino acid deletion, the following nomenclature is used: Original amino acid, position, *. Accordingly, the deletion of glycine at position 195 is designated as "Gly195*" or "G195*". Multiple deletions are separated by addition marks ("+"), *e.g.*, "Gly195* + Ser411*" or "G195* + S411*".

Multiple modifcations. Variants comprising multiple modifications are separated by addition marks ("+"), *e.g.*, "Arg170Tyr+Gly195Glu" or "R170Y+G195E" representing a substitution of arginine and glycine at positions 170 and 195 with tyrosine and glutamic acid, respectively.

Different modifications. Where different modifications may be introduced at a position, the different alterations are separated by a comma, *e.g.*, "Arg170Tyr,Glu" represents a substitution of arginine at position 170 with tyrosine or glutamic acid. Thus, "Tyr167Gly,Ala + Arg170Gly,Ala" designates the following variants:
"Tyr167Gly+Arg170Gly", "Tyr167Gly+Arg170Ala", "Tyr167Ala+Arg170Gly", and "Tyr167Ala+Arg170Ala".

## DETAILED DESCRIPTION OF THE INVENTION

The present invention relates to the use of an alpha-amylase variant of a parent alpha-amylase in a cleaning process, wherein a bleaching system is added in a concentration of at least 5 weight%, wherein said parent has an amino acid sequence which is at least 80% identical to the amino acid sequence set forth in SEQ ID NO: 3, wherein said variant comprises a substitution in one or more positions providing oxidation stability of said variant, wherein said variant comprises a substitution in position 202, wherein said position corresponds to the amino acid position of the amino acid sequence as set forth in SEQ ID NO: 3, wherein said substitution is a M202L substitution, wherein said variant has a sequence identity of at least 80%, but less than 100% to the amino acid sequence set forth in SEQ ID NO: 3, wherein said variant has an improvement factor of >1.0 as a measure for wash performance, when compared to said parent alpha-amylase, wherein said variant has alpha-amylase activity.

SEQ ID NO: 1 is the mature nucleotide sequence of an alpha-amylase (AAI10)
SEQ ID NO: 2 is the full-length, *i.e.* including the signal peptide, amino acid sequence of the alpha-amylase (AAI10). The signal peptide is designated as amino acid number -29 to -1, and thus, the mature part of the alpha-amylase is designated as amino acid number 1 to 485.
SEQ ID NO: 3 is the mature amino acid sequence of the alpha-amylase (AAI10)
SEQ ID NO: 4 is the mature amino acid sequence of the alpha-amylase (AAI10) comprising a deletion of the amino acids corresponding to positions 182 and 183 of SEQ ID NO: 3
SEQ ID NO: 5 is the mature amino acid sequence of an alpha-amylase (SP707)
SEQ ID NO: 6 is the mature amino acid sequence of an alpha-amylase (AA560)
SEQ ID NO: 7 is the mature amino acid sequence of an alpha-amylase (K36)
SEQ ID NO: 8 is the mature amino acid sequence of a protease (Savinase)
SEQ ID NO: 9 is the mature amino acid sequence of an alpha-amylase (hybrid polypeptide)
SEQ ID NO: 10 is the mature amino acid sequence of an alpha-amylase (K38)
SEQ ID NO: 11 is the mature amino acid sequence of an alpha-amylase (KSM-AP1378)
SEQ ID NO: 12 is the mature amino acid sequence of an alpha-amylase (SP.7-7)
SEQ ID NO: 13 is the mature amino acid sequence of an alpha-amylase (AAI6)

### Variants

Disclosed are variants of a parent polypeptide used in accordance with the present invention having alpha-amylase activity.

The present inventors have found that a variant comprising an amino acid substitution in one or more positions providing oxidation stability does not compromise the wash performance of the variant, *i.e.* see the results of Example 3.

The term "alpha-amylase variant" as used herein, refers to a polypeptide having alpha-amylase activity comprising a modification, *i.e.,* a substitution, insertion, and/or deletion, at one or more (*e.g.*, several) positions. A substitution means replacement of the amino acid occupying a position with a different amino acid; a deletion means removal of the amino acid occupying a position; and an insertion means adding an amino acid adjacent to and immediately following the amino acid occupying a position, all as defined above. The variants used in accordance with the present invention have at least 20%, *e.g.*, at least 40%, at least 50%, at least 60%, at least 70%, at least 80%, at least 90%, at least 95%, or at least 100% of the alpha-amylase activity of the amino acid sequences set forth in SEQ ID NOs: 3, 4, or the mature amino acid sequence of SEQ ID NO: 2.

The term "alpha-amylase activity" as used herein, refers to the activity of alpha-1,4-glucan-4-glucanohydrolases, E.C. 3.2.1.1, which constitute a group of enzymes, catalyzing hydrolysis of starch and other linear and branched 1,4-glucosidic oligo- and polysaccharides. The terms "alpha-amylase" and "amylase" may be used interchangeably and constitute the same meaning and purpose within the scope of the present invention. For purposes of the present invention, alpha-amylase activity is determined according to the procedure described in the Examples. In one embodiment, the variants of the present invention have at least 20%, *e.g.*, at least 40%, at least 50%, at least 60%, at least 70%, at least 80%, at least 90%, at least 95%, or at least 100% of the alpha-amylase activity of the amino acid sequence as set forth in SEQ ID NOs: 3 or 4; or the mature amino acid sequence as set forth in SEQ ID NO: 2.

The term "parent alpha-amylase" as used herein, refers to an alpha-amylase to which an alteration is made to produce alpha-amylase variants. An alpha-amylase having any of the amino acid sequences set forth in SEQ ID NOs: 3 and 4 may *e.g.* be a parent for the variants of the present invention. Any amino acid sequence having at least 80%, such as at least 85%, such at least 90%, such as at least 95%, such as at least 97%, such as at least 99%, sequence identity to any one of SEQ ID NOs: 3 and 4 may also be a parent alpha-amylase for the variants of the present invention.

The parent polypeptide may be obtained from microorganisms of any genus. For purposes of the present invention, the term "obtained from" as used herein in connection with a given source shall mean that the parent encoded

by a polynucleotide is produced by the source or by a strain in which the polynucleotide from the source has been inserted.

In some aspects, the parent alpha-amylase is *Bacillus* sp. alpha-amylase, *e.g.*, the alpha-amylase of SEQ ID NO: 2, the mature polypeptide thereof, *i.e.* SEQ ID NO: 3 or 4.

In some aspects, the parent alpha-amylase polypeptide is encoded by the nucleic acid sequence as set forth in SEQ ID NO: 1.

It will be understood that the aforementioned species encompasses both the perfect and imperfect states, and other taxonomic equivalents, *e.g.*, anamorphs, regardless of the species name by which they are known. Those skilled in the art will readily recognize the identity of appropriate equivalents.

Strains of this species are readily accessible to the public in a number of culture collections, such as the American Type Culture Collection (ATCC), Deutsche Sammlung von Mikroorganismen und Zellkulturen GmbH (DSMZ), Centraalbureau Voor Schimmelcultures (CBS), and Agricultural Research Service Patent Culture Collection, Northern Regional Research Center (NRRL).

The parent alpha-amylase may be identified and obtained from other sources including microorganisms isolated from nature (*e.g.*, soil, composts, water, etc.) or DNA samples obtained directly from natural materials (*e.g.*, soil, composts, water, etc.) using the above-mentioned probes. Techniques for isolating microorganisms and DNA directly from natural habitats are well known in the art. A polynucleotide encoding a parent polypeptide may then be obtained by similarly screening a genomic DNA or cDNA library of another microorganism or mixed DNA sample. Once a polynucleotide encoding a parent polypeptide has been detected with the probe(s), the polynucleotide can be isolated or cloned by utilizing techniques that are known to those of ordinary skill in the art (see, *e.g.*, Sambrook *et al.,* 1989, *supra*).

In one embodiment, the parent alpha-amylase comprises or consists of the amino acid sequence set forth in SEQ ID NO: 3 or 4. In another embodiment, the parent alpha-amylase comprises or consists of the full-length amino acid sequence as set forth in SEQ ID NO: 2. In particular, the parent alpha-amylase may comprise or consist of amino acids 1 to 485 of SEQ ID NO: 2.

In another embodiment, the parent alpha-amylase is a fragment of the amino acid sequence as set forth in SEQ ID NO: 3 or 4 containing at least 475 amino acid residues, *e.g.*, at least 480 and at least 485 amino acid residues of SEQ ID NO: 3 or 4.

In another embodiment, the parent alpha-amylase is an allelic variant of the amino acid sequence as set forth in SEQ ID NO: 3 or 4. Accordingly, the parent alpha-amylase may comprise the amino acid sequence set forth in SEQ ID NO: 13.

The term "sequence identity" as used herein, refers to the relatedness between two amino acid sequences or between two nucleotide sequences. For purposes of the present invention, the sequence identity between two amino acid sequences is determined using the Needleman-Wunsch algorithm (Needleman and Wunsch, 1970, J. Mol. Biol. 48: 443-453) as implemented in the Needle program of the EMBOSS package (EMBOSS: The European Molecular Biology Open Software Suite, Rice et al., 2000, Trends Genet. 16: 276-277), preferably version 5.0.0 or later. The parameters used are gap open penalty of 10, gap extension penalty of 0.5, and the EBLOSUM62 (EMBOSS version of BLOSUM62) substitution matrix. The output of Needle labeled "longest identity" (obtained using the -nobrief option) is used as the percent identity and is calculated as follows:

(Identical Residues x 100)/(Length of Alignment – Total Number of Gaps in Alignment)

For purposes of the present invention, the sequence identity between two deoxyribonucleotide sequences is determined using the Needleman-Wunsch algorithm (Needleman and Wunsch, 1970, *supra*) as implemented in the Needle program of the EMBOSS package (EMBOSS: The European Molecular Biology Open Software Suite, Rice et al., 2000, *supra*), preferably version 5.0.0 or later. The parameters used are gap open penalty of 10, gap extension penalty of 0.5, and the EDNAFULL (EMBOSS version of NCBI NUC4.4) substitution matrix. The output of Needle labeled "longest identity" (obtained using the -nobrief option) is used as the percent identity and is calculated as follows:

(Identical Deoxyribonucleotides x 100)/(Length of Alignment – Total Number of Gaps in Alignment)

The term "oxidation stability" as used herein, refers to a property of a protein, such as an alpha-amylase variant according to the invention, which is not oxidized, *i.e.* protein degraded or made inactive, due to an active oxidation components of, *e.g.*, a detergent composition comprising such oxidation components. The term "oxidation components" as used herein, refers to bleaching systems as defined elsewhere herein. Thus, a variant which is oxidation stable as the variants of the present invention remains active even in the presence of bleaching systems.

The term "wash performance" as used herein, refers to an enzyme's ability to remove starch or starch-containing

stains present on the object to be cleaned during *e.g.* laundry or hard surface cleaning, such as dish wash. The term "wash performance" includes cleaning in general *e.g.* hard surface cleaning as in dish wash, but also wash performance on textiles such as laundry, and also industrial and institutional cleaning. The wash performance may be quantified by calculating the so-called Intensity value, and results may be displayed as "Improvement Factor" (IF). Wash performance may be determined as in described in the Examples herein.

The term "Intensity value" as used herein, refers to the wash performance measured as the brightness expressed as the intensity of the light reflected from the sample when illuminated with white light. When the sample is stained the intensity of the reflected light is lower, than that of a clean sample. Therefore the intensity of the reflected light can be used to measure wash performance, where a higher intensity value correlates with higher wash performance.

Color measurements are made with a professional flatbed scanner (Kodak iQsmart, Kodak) used to capture an image of the washed textile.

To extract a value for the light intensity from the scanned images, 24-bit pixel values from the image are converted into values for red, green and blue (RGB). The intensity value (Int) is calculated by adding the RGB values together as vectors and then taking the length of the resulting vector:

$$Int = \sqrt{r^2 + g^2 + b^2}$$

The term "does not compromise the wash performance of the variant" as used herein, refers to a property of the variant according to the invention, wherein the modifications made in the variant does not have a negative or any effect on the wash performance.

In one embodiment, the oxidation stability is determined by an Automatic Mechanical Stress Assay (AMSA) wherein the variant is tested at 55°C for 20 min, and wherein a detergent used in the AMSA comprises a bleaching system as described in Example 3.

The term "Automatic Mechanical Stress Assay (AMSA)" as used herein, refers to a specific assay which is used to assess the wash performance of an enzyme, such as an alpha-amylase. With the AMSA test the wash performance of a large quantity of small volume enzyme-detergent solutions can be examined. The AMSA plate has a number of slots for test solutions and a lid firmly squeezing the textile swatch to be washed against all the slot opening. During the washing time, the plate, test solutions, textile and lidt are vigorously shaken to bring the test solution in contact with the textile and apply mechanical stress in a regular, periodic oscilating manner. For specific conditions under which wash performance may be determined, see the Example 3. Furthermore, the skilled person knows how to perform a general AMSA in order to evaluate wash performance of a variant.

The term "detergent" or "detergent solution" as used herein, refers to a composition which is considered applicable for use in detergents, such as laundry detergents. The terms "detergent" and "detergent solution" may be used interchangeably herein, and have the same meaning and purpose, unless otherwise explicitly stated by context.

The term "detergent used in the said AMSA" as used herein, refers to a specific detergent used in the AMSA performed. *I.e.* the detergent used may be such as the Model Z detergent as described in the Example 3.

The term "bleaching system" as used herein, refers to inorganic and organic bleaches suitable as cleaning actives. Inorganic bleaches include perhydrate salts such as perborate, percarbonate, perphosphate, persulfate and persilicate salts. The inorganic perhydrate salts are normally the alkali metal salts. The inorganic perhydrate salt may be included as the crystalline solid without additional protection. Alternatively, the salt can be coated.

Alkali metal percarbonates, particularly sodium percarbonate are preferred perhydrates for use herein. The percarbonate is most preferably incorporated into the products in a coated form which provides in-product stability. A suitable coating material providing in product stability comprises mixed salt of a water-soluble alkali metal sulphate and carbonate. Such coatings together with coating processes have previously been described in GB 1,466,799. The weight ratio of the mixed salt coating material to percarbonate lies in the range from 1:200 to 1:4, more preferably from 1:99 to 1:9, and most preferably from 1:49 to 1:19. Preferably, the mixed salt is of sodium sulphate and sodium carbonate which has the general formula Na2S04.n.Na2CO3 wherein n is from 0.1 to 3, preferably n is from 0.3 to 1.0 and most preferably n is from 0.2 to 0.5.

Another suitable coating material providing in product stability, comprises sodium silicate of $SiO_2$: $Na_2O$ ratio from 1.8:1 to 3.0:1, preferably 1.8:1 to 2.4:1, and/or sodium metasilicate, preferably applied at a level of from 2% to 10%, (normally from 3% to 5%) of SiO2 by weight of the inorganic perhydrate salt. Magnesium silicate can also be included in the coating. Coatings that comprise silicate and borate salts or boric acids or other inorganics are also suitable.

Other coatings which comprising waxes, oils, fatty soaps can also be used advantageously within the present invention.

Potassium peroxymonopersulfate is another inorganic perhydrate salt of utility herein. Typical organic bleaches are organic peroxyacids including diacyl and tetraacylperoxides, especially diperoxydodecanedioc acid, diperoxytetradecanedioc acid, and diperoxyhexadecanedioc acid. Dibenzoyl peroxide is a preferred organic peroxyacid herein. Mono-

and diperazelaic acid, mono- and diperbrassylic acid, and Nphthaloylaminoperoxicaproic acid are also suitable herein. The diacyl peroxide, especially dibenzoyl peroxide, should preferably be present in the form of particles having a weight average diameter of from about 0.1 to about 100 microns, preferably from about 0.5 to about 30 microns, more preferably from about 1 to about 10 microns. Preferably, at least about 25%, more preferably at least about 50%, even more preferably at least about 75%, most preferably at least about 90%, of the particles are smaller than 10 microns, preferably smaller than 6 microns. Diacyl peroxides within the above particle size range have also been found to provide better stain removal especially from plastic dishware, while minimizing undesirable deposition and filming during use in automatic dishwashing machines, than larger diacyl peroxide particles. The preferred diacyl peroxide particle size thus allows the formulator to obtain good stain removal with a low level of diacyl peroxide, which reduces deposition and filming. Conversely, as diacyl peroxide particle size increases, more diacyl peroxide is needed for good stain removal, which increases deposition on surfaces encountered during the dishwashing process. Further typical organic bleaches include the peroxy acids, particular examples being the alkylperoxy acids and the arylperoxy acids. Preferred representatives are (a) peroxybenzoic acid and its ring-substituted derivatives, such as alkylperoxybenzoic acids, but also peroxy-[alpha]-naphthoic acid and magnesium monoperphthalate, (b) the aliphatic or substituted aliphatic peroxy acids, such as peroxylauric acid, peroxystearic acid, [epsilon]-phthalimidoperoxycaproic acid[phthaloiminoperoxyhexanoic acid (PAP)], o-carboxybenzamidoperoxycaproic acid, N-nonenylamidoperadipic acid and N-nonenylamidopersuccinates, and (c) aliphatic and araliphatic peroxydicarboxylic acids, such as 1,12-diperoxycarboxylic acid, 1,9-diperoxyazelaic acid, diperoxysebacic acid, diperoxybrassylic acid, the diperoxyphthalic acids, 2- decyldiperoxybutane-1,4-dioic acid, N,N-terephthaloyldi(6-aminopercaproic acid).

In a particular embodiment, the bleaching system is added in a concentration of at least 5 weight%, such as at least 8 weight%, such as at least 10 weight%, or such as at least 15 weight%.

The term "weight%" as used herein, refers to a component, such as a bleaching system, which is present in a percentage calculated based on weight rather than volume. The term is well-known to the skilled person, who is also able to calculate such weight% based on the knowledge of the components of a solution, such as a detergent solution.

In one embodiment, the bleaching system is sodium percarbonate.

In one embodiment, the variant has an improvement factor of >1.5 wherein the IF has been determined in an AMSA wherein the conditions are a wash cycle of 20 min at 55°C and wherein the variant is tested in the presence of a bleaching system, such as sodium percarbonate, at a concentration of 8 weight%.

In one embodiment, the parent alpha-amylase has an amino acid sequence as set forth in SEQ ID NO: 3, or has an amino acid sequence which is at least 80%, such as at least 85%, such as at least 90%, such as at least 95%, such as at least 98%, identical to the amino acid sequence as set forth in SEQ ID NO: 3.

In one embodiment, the parent alpha-amylase has an amino acid sequence as set forth in SEQ ID NO: 4, or has an amino acid sequence which is at least 80%, such as at least 85%, such as at least 90%, such as at least 95%, such as at least 98%, identical to the amino acid sequence as set forth in SEQ ID NO: 4.

In one embodiment, the parent alpha-amylase has an amino acid sequence as set forth in SEQ ID NO: 13, or has an amino acid sequence which is at least 80%, such as at least 85%, such as at least 90%, such as at least 95%, such as at least 98%, identical to the amino acid sequence as set forth in SEQ ID NO: 13.

In one embodiment, the parent alpha-amylase comprises or consists of the amino acid sequence as set forth in SEQ ID NO: 3.

In one embodiment, the parent alpha-amylase comprises or consists of the amino acid sequence as set forth in SEQ ID NO: 4.

In one embodiment, the parent alpha-amylase comprises or consists of the amino acid sequence as set forth in SEQ ID NO: 13.

In one embodiment, the parent alpha-amylase is a fragment of the polypeptide of SEQ ID NO: 3, wherein the fragment has alpha-amylase activity.

In one embodiment, the parent alpha-amylase is a fragment of the polypeptide of SEQ ID NO: 4, wherein the fragment has alpha-amylase activity.

In one embodiment, the parent alpha-amylase is a fragment of the polypeptide of SEQ ID NO: 13, wherein the fragment has alpha-amylase activity.

The term "fragment" as used herein, refers to a polypeptide having one or more (e.g., several) amino acids absent from the amino and/or carboxyl terminus of a mature polypeptide; wherein the fragment has alpha-amylase activity. In one embodiment, a fragment contains at least 480 amino acid residues, at least 481 amino acid residues, or at least 482 amino acid residues.

In one embodiment, the variant has a sequence identity of at least 80%, such as at least 85%, such as at least 90%, such as at least 95%, such as at least 98%, but less than 100% to the amino acid sequence as set forth in SEQ ID NO: 3.

In one embodiment, the variant has a sequence identity of at least 80%, such as at least 85%, such as at least 90%, such as at least 95%, such as at least 98%, but less than 100% to the amino acid sequence as set forth in SEQ

ID NO: 4.

In one embodiment, the variant has a sequence identity of at least 80%, such as at least 85%, such as at least 90%, such as at least 95%, such as at least 98%, but less than 100% to the amino acid sequence as set forth in SEQ ID NO: 13.

The term "sequence identity" as used herein, refers to any definition already stated herein. Determination of the sequence identity may be performed as described elsewhere herein.

Without being bound by any theory it is contemplated that any amino acid substitution but the naturally-occurring amino acid in the position corresponding to M202 of SEQ ID NO: 3, will provide an oxidation stable variant having an IF of at least 1.0 as a measure for wash performance.

In one embodiment, the variant comprises a deletion in two or more positions corresponding to positions R181, G182, D183, and G184 of the amino acid sequence as set forth in SEQ ID NO: 3.

The term "deletion" as used herein, refers to the removal of an amino acid within a polypeptide, such as an enzyme. Such removal, *i.e.* deletion, of one or more amino acids may be done by site-directed mutagenesis or any other method known in the art and by the skilled person.

A variant according to the present invention comprising a deletion in two or more positions corresponding to positions R181, G182, D183, and G184 of SEQ ID NO: 3, provides stability to the variants as well as contribute to improving the wash performance of the variants. It is well-known that this particular part of alpha-amylases provides stability to alpha-amylase variants, *i.e.* as described in *e.g.* WO 1996/023873. The term "stability" as used in this context, refers to the stability of the alpha-amylase variants during wash. Such stability may be determined by measuring the activity of the variant after a wash cycle of *e.g.* 60 min in a detergent solution at 40-60°C. Providing variants comprising a deletion in two or more positions corresponding to positions R181, G182, D183, and G184 of SEQ ID NO: 3 and a substitution in one or more amino acids providing oxidation stability of a variant, lies within the scope of the present invention.

Thus, in one embodiment, the variant comprises a deletion in the positions corresponding to R181+G182; R181+D183; R181+G184; G182+D183; G182+G184; or D183+G184, wherein the positions correspond to the positions in the amino acid sequence as set forth in SEQ ID NO:3.

The variants may further comprise one or more additional modifications, *e.g.* substitutions, at one or more (*e.g.*, several) other positions.

The amino acid changes may be of a minor nature, that is conservative amino acid substitutions or insertions that do not significantly affect the folding and/or activity of the protein; small deletions, typically of 1-30 amino acids; small amino- or carboxyl-terminal extensions, such as an amino-terminal methionine residue; a small linker peptide of up to 20-25 residues; or a small extension that facilitates purification by changing net charge or another function, such as a polyhistidine tract, an antigenic epitope or a binding domain.

Examples of conservative substitutions are within the groups of basic amino acids (arginine, lysine and histidine), acidic amino acids (glutamic acid and aspartic acid), polar amino acids (glutamine and asparagine), hydrophobic amino acids (leucine, isoleucine and valine), aromatic amino acids (phenylalanine, tryptophan and tyrosine), and small amino acids (glycine, alanine, serine, threonine and methionine). Amino acid substitutions that do not generally alter specific activity are known in the art and are described, for example, by H. Neurath and R.L. Hill, 1979, In, The Proteins, Academic Press, New York. Common substitutions are Ala/Ser, Val/Ile, Asp/Glu, Thr/Ser, Ala/Gly, Ala/Thr, Ser/Asn, Ala/Val, Ser/Gly, Tyr/Phe, Ala/Pro, Lys/Arg, Asp/Asn, Leu/Ile, Leu/Val, Ala/Glu, and Asp/Gly.

Alternatively, the amino acid changes are of such a nature that the physico-chemical properties of the polypeptides are altered. For example, amino acid changes may improve the thermal stability of the polypeptide, alter the substrate specificity, change the pH optimum, and the like.

Essential amino acids in a polypeptide can be identified according to procedures known in the art, such as site-directed mutagenesis or alanine-scanning mutagenesis (Cunningham and Wells, 1989, Science 244: 1081-1085). In the latter technique, single alanine mutations are introduced at every residue in the molecule, and the resultant mutant molecules are tested for alpha-amylase activity to identify amino acid residues that are critical to the activity of the molecule. See also, Hilton et al., 1996, J. Biol. Chem. 271: 4699-4708. The active site of the enzyme or other biological interaction can also be determined by physical analysis of structure, as determined by such techniques as nuclear magnetic resonance, crystallography, electron diffraction, or photoaffinity labeling, in conjunction with mutation of putative contact site amino acids. See, for example, de Vos et al., 1992, Science 255: 306-312; Smith et al., 1992, J. Mol. Biol. 224: 899-904; Wlodaver et al., 1992, FEBS Lett. 309: 59-64. The identity of essential amino acids can also be inferred from an alignment with a related polypeptide.

Thus, in one embodiment, the variant comprises 1 to 40 substitutions, such as 1 to 30, such as 1 to 20, such as 1 to 10, such as 1, 2, 3, 4, 5, 6, 7, 8, 9, 10, 11, 12, 13, 14, 15, 16, 17, 18, 19, 20, 21, 22, 23, 24, 25, 26, 27, 28, 29, or 30 substitutions.

In a particular embodiment, the number of substitutions is 1 to 20, *e.g.*, 1 to 10 and 1 to 5, such as 1, 2, 3, 4, 5, 6, 7, 8, 9 or 10 substitutions.

In one embodiment, the variant further comprises further modifications.

The variants may consist of 430 to 490 amino acids, *e.g.*, 440 to 485, 450 to 485, and 460 to 483 amino acids.

In one embodiment, the variant comprises or consists of the following modifications; G182*+D183*+M202L, wherein numbering is according to SEQ ID NO: 3.

In a particular embodiment, the variant comprises or consists of the following modifications; G182*+D183*+N195F+M202L, wherein numbering is according to SEQ ID NO: 3.

The variant of a parent alpha-amylase comprises or consists of the amino acid sequence having at least 80%, such as at least 85%, such as at least 90%, such as at least 95%, or such as at least 98% sequence identity to an amino acid sequence as set forth in SEQ ID NO: 3, and the variant consists of a M202L substitution in a position corresponding to position M202 of SEQ ID NO: 3.

In another aspect, the parent alpha-amylase consisting of the amino acid sequence as set forth in SEQ ID NO: 3, wherein the variant consists of the modifications G182*+D183*+M202L, wherein numbering is according to SEQ ID NO: 3.

In another aspect, the variant of a parent alpha-amylase consisting of the amino acid sequence as set forth in SEQ ID NO: 3, wherein the variant consists of the modifications G182*+D183*+N195F+M202L, wherein numbering is according to SEQ ID NO: 3.

**Polynucleotides**

Disclosed are polynucleotides encoding a variant used in accordance with the present invention. Thus, in particular, the disclosed polynucleotide encodes a variant comprising a substitution in one or more positions providing oxidation stability.

The term "polynucleotides encoding" as used herein, refers to a polynucleotide that encodes a mature polypeptide having alpha-amylase activity. In one aspect, the polypeptide coding sequence is the nucleotide sequence set forth in SEQ ID NO: 1.

In one embodiment, the polynucleotide encodes a variant used in accordance with the present invention and has at least 80%, such as at least 85%, such as at least 90%, such as at least 95%, such as at least 96%, such as at least 97%, such as at least 98%, such as at least 99% but less than 100% sequence identity to the polynucleotide of SEQ ID NO: 1.

In one embodiment, the polynucleotide encodes a variant comprising a substitution and/or deletion in two, three, or four positions corresponding to positions R181, G182, D183, and G184 of the amino acid sequence as set forth in SEQ ID NO: 3, and a substitution in the position corresponding to position M202 of the amino acid sequence as set forth in SEQ ID NO: 3.

**Nucleic Acid Constructs**

Disclosed are nucleic acid constructs comprising a polynucleotide encoding a variant used in accordance with the present invention operably linked to one or more control sequences that direct the expression of the coding sequence in a suitable host cell under conditions compatible with the control sequences. Disclosed is also a nucleic acid construct comprising a polynucleotide encoding a variant used in accordance with the present invention comprising a substitution in one or more positions providing oxidation stability of the variant, wherein the polynucleotide is operately linked to one or more control sequences.

The term "nucleic acid construct" as used herein, refers to a nucleic acid molecule, either single- or double-stranded, which is isolated from a naturally occurring gene or is modified to comprise segments of nucleic acids in a manner that would not otherwise exist in nature or which is synthetic, which comprises one or more control sequences.

The term "operably linked" as used herein, refers to a configuration in which a control sequence is placed at an appropriate position relative to the coding sequence of a polynucleotide such that the control sequence directs expression of the coding sequence.

The polynucleotide may be manipulated in a variety of ways to provide for expression of a variant. Manipulation of the polynucleotide prior to its insertion into a vector may be desirable or necessary depending on the expression vector. The techniques for modifying polynucleotides utilizing recombinant DNA methods are well known in the art.

The control sequence may be a promoter, a polynucleotide which is recognized by a host cell for expression of the polynucleotide. The promoter comprises transcriptional control sequences that mediate the expression of the variant. The promoter may be any polynucleotide that shows transcriptional activity in the host cell including mutant, truncated, and hybrid promoters, and may be obtained from genes encoding extracellular or intracellular polypeptides either homologous or heterologous to the host cell.

In one embodiment, the nucleic acid construct comprises a polynucleotide encoding a variant comprising a substitution and/or deletion in two, three, or four positions corresponding to positions R181, G182, D183, and G184 of

the amino acid sequence as set forth in SEQ ID NO: 3, and a substitution in the position corresponding to position M202 of the amino acid sequence as set forth in SEQ ID NO: 3, wherein the polynucleotide is operately linked to one or more control sequences.

## Expression Vectors

Disclosed are recombinant expression vectors comprising a polynucleotide encoding a variant used in accordance with the present invention, a promoter, and transcriptional and translational stop signals. Disclosed is an expression vector, optionally a recombinant expression vector, comprising a polynucleotide encoding a variant used in accordance with the present invention comprising a substitution in one or more positions providing oxidation stability of the variant, a promoter, and transcriptional and translational stop signals.

The term "expression vector" as used herein, refers to a linear or circular DNA molecule that comprises a polynucleotide encoding a variant and is operably linked to control sequences that provide for its expression.

In one embodiment, the expression vector comprises a polynucleotide encoding a variant comprising a substitution and/or deletion in two, three, or four positions corresponding to positions R181, G182, D183, and G184 of the amino acid sequence as set forth in SEQ ID NO: 3, and a substitution in the position corresponding to position M202 of the amino acid sequence as set forth in SEQ ID NO: 3, and wherein the expression vector further comprises a promoter, and transcriptional and translational stop signals.

The various nucleotide and control sequences may be joined together to produce a recombinant expression vector that may include one or more convenient restriction sites to allow for insertion or substitution of the polynucleotide encoding the variant at such sites. Alternatively, the polynucleotide may be expressed by inserting the polynucleotide or a nucleic acid construct comprising the polynucleotide into an appropriate vector for expression. In creating the expression vector, the coding sequence is located in the vector so that the coding sequence is operably linked with the appropriate control sequences for expression.

The recombinant expression vector may be any vector (*e.g.*, a plasmid or virus) that can be conveniently subjected to recombinant DNA procedures and can bring about expression of the polynucleotide. The choice of the vector will typically depend on the compatibility of the vector with the host cell into which the vector is to be introduced.

The vector may be an autonomously replicating vector, *i.e.*, a vector that exists as an extrachromosomal entity, the replication of which is independent of chromosomal replication, *e.g.*, a plasmid, an extrachromosomal element, a minichromosome, or an artificial chromosome. The vector may comprise any means for assuring self-replication. Alternatively, the vector may be one that, when introduced into the host cell, is integrated into the genome and replicated together with the chromosome(s) into which it has been integrated. Furthermore, a single vector or plasmid or two or more vectors or plasmids that together contain the total DNA to be introduced into the genome of the host cell, or a transposon, may be used.

The vector preferably comprises one or more selectable markers that permit easy selection of transformed, transfected, transduced, or the like cells. A selectable marker is a gene the product of which provides for biocide or viral resistance, resistance to heavy metals, prototrophy to auxotrophs, and the like.

The skilled person would know which expression vector is the most suitable for specific expression systems. Thus, the present invention is not limited to any specific expression vector, but any expression vector comprising the polynucleotide encoding a variant according to the invention is considered part of the present invention.

## Host Cells

Disclosed are recombinant host cells, comprising a polynucleotide encoding a variant used in accordance with the present invention operably linked to one or more control sequences that direct the production of said variant. Disclosed is a host cell, optionally a recombinant host cell, comprising polynucleotide encoding a variant used in accordance with the present invention comprising a substitution in one or more positions providing oxidation stability of the variant, operably linked to one or more control sequences that direct the production of the variant.

The term "host cell" as used herein, refers to any cell type that is susceptible to transformation, transfection, transduction, or the like with a nucleic acid construct or expression vector comprising a polynucleotide of the present invention. The term "host cell" encompasses any progeny of a parent cell that is not identical to the parent cell due to mutations that occur during replication.

In one embodiment, the host cell comprises a polynucleotide encoding a variant comprising a substitution and/or deletion in two, three, or four positions corresponding to positions R181, G182, D183, and G184 of the amino acid sequence as set forth in SEQ ID NO: 3, and a substitution in the position corresponding to position M202 of the amino acid sequence as set forth in SEQ ID NO: 3, the polynucleotide operately linked to one or more control sequences that direct the production of the variant.

A construct or vector comprising a polynucleotide is introduced into a host cell so that the construct or vector

is maintained as a chromosomal integrant or as a self-replicating extrachromosomal vector as described earlier. The term "host cell" encompasses any progeny of a parent cell that is not identical to the parent cell due to mutations that occur during replication. The choice of a host cell will to a large extent depend upon the gene encoding the variant and its source.

The host cell may be any cell useful in the recombinant production of a variant, *e.g.*, a prokaryote or a eukaryote.

The prokaryotic host cell may be any Gram-positive or Gram-negative bacterium. Gram-positive bacteria include, but are not limited to, *Bacillus, Clostridium, Enterococcus, Geobacillus, Lactobacillus, Lactococcus, Oceanobacillus, Staphylococcus, Streptococcus,* and *Streptomyces.* Gram-negative bacteria include, but are not limited to, *Campylobacter, E. coli, Flavobacterium, Fusobacterium, Helicobacter, Ilyobacter, Neisseria, Pseudomonas, Salmonella,* and *Ureaplasma.*

**Methods of the Invention**

Disclosed are methods of producing a variant used in accordance with the present invention, comprising: (a) cultivating a host cell under conditions suitable for expression of the variant; and (b) recovering the variant. Disclosed is a method of producing a variant used in accordance with the present invention comprising a substitution in one or more positions providing oxidation stability of the variant, wherein the method comprises the steps of a) cultivating the host cell under conditions suitable for expression of the variant, and b) recovering the variant.

The term "conditions suitable for expression" as used herein, refers to which settings the host cell expressing the variant according to the invention, are cultivated in. These conditions (or settings) may depend on the type of host cell. *I.e.* conditions suitable to cultivate yeast host cells are different than from those of cultivate bacterial host cells. It is within the knowledge of the skilled person to determine which conditions are the most optimal, *i.e.* suitable, for the specific host cells.

In one embodiment, the method of producing a variant comprising a substitution and/or deletion in two, three, or four positions corresponding to positions R181, G182, D183, and G184 of the amino acid sequence as set forth in SEQ ID NO: 3, and a substitution in the position corresponding to position M202 of the amino acid sequence as set forth in SEQ ID NO: 3, comprises the steps of a) cultivating the host cell according to the invention under conditions suitable for expression of the variant, and b) recovering the variant.

The host cells are cultivated in a nutrient medium suitable for production of the variant using methods known in the art. For example, the cell may be cultivated by shake flask cultivation, or small-scale or large-scale fermentation (including continuous, batch, fed-batch, or solid state fermentations) in laboratory or industrial fermentors performed in a suitable medium and under conditions allowing the variant to be expressed and/or isolated. The cultivation takes place in a suitable nutrient medium comprising carbon and nitrogen sources and inorganic salts, using procedures known in the art. Suitable media are available from commercial suppliers or may be prepared according to published compositions (*e.g.*, in catalogues of the American Type Culture Collection). If the variant is secreted into the nutrient medium, the variant can be recovered directly from the medium. If the variant is not secreted, it can be recovered from cell lysates.

The variant may be detected using methods known in the art that are specific for the variants having alpha-amylase activity. These detection methods include, but are not limited to, use of specific antibodies, formation of an enzyme product, or disappearance of an enzyme substrate. For example, an enzyme assay may be used to determine the activity of the variant.

The variant may be recovered using methods known in the art. For example, the variant may be recovered from the nutrient medium by conventional procedures including, but not limited to, collection, centrifugation, filtration, extraction, spray-drying, evaporation, or precipitation.

The variant may be purified by a variety of procedures known in the art including, but not limited to, chromatography (*e.g.*, ion exchange, affinity, hydrophobic, chromatofocusing, and size exclusion), electrophoretic procedures (*e.g.*, preparative isoelectric focusing), differential solubility (*e.g.*, ammonium sulfate precipitation), SDS-PAGE, or extraction (see, *e.g.,* Protein Purification, Janson and Ryden, editors, VCH Publishers, New York, 1989) to obtain substantially pure variants.

In an alternative aspect, the variant is not recovered, but rather a host cell of the present invention expressing the variant is used as a source of the variant.

Disclosed are also methods for obtaining a variant, comprising introducing into a parent alpha-amylase a substitution in position M202 wherein the position correspond to the position of the amino acid sequence as set forth in SEQ ID NO: 3; b) optionally, introducing a deletion in two or more positions corresponding to positions R181, G182, D183, and G184 of the amino acid sequence as set forth in SEQ ID NO: 3; and c) recovering the variant.

The variants can be prepared using any mutagenesis procedure known in the art, such as site-directed mutagenesis, synthetic gene construction, semi-synthetic gene construction, random mutagenesis, shuffling, etc.

Site-directed mutagenesis is a technique in which one or more (*e.g.*, several) mutations are introduced at one or more defined sites in a polynucleotide encoding the parent.

Site-directed mutagenesis can be accomplished *in vitro* by PCR involving the use of oligonucleotide primers containing the desired mutation. Site-directed mutagenesis can also be performed *in vitro* by cassette mutagenesis involving the cleavage by a restriction enzyme at a site in the plasmid comprising a polynucleotide encoding the parent and subsequent ligation of an oligonucleotide containing the mutation in the polynucleotide. Usually the restriction enzyme that digests the plasmid and the oligonucleotide is the same, permitting sticky ends of the plasmid and the insert to ligate to one another. See, e.g., Scherer and Davis, 1979, Proc. Natl. Acad. Sci. USA 76: 4949-4955; and Barton et al., 1990, Nucleic Acids Res. 18: 7349-4966.

Site-directed mutagenesis can also be accomplished *in vivo* by methods known in the art. See, *e.g.*, U.S. Patent Application Publication No. 2004/0171154; Storici et al., 2001, Nature Biotechnol. 19: 773-776; Kren et al., 1998, Nat. Med. 4: 285-290; and Calissano and Macino, 1996, Fungal Genet. Newslett. 43: 15-16.

Any site-directed mutagenesis procedure can be used in the present invention. There are many commercial kits available that can be used to prepare variants.

Synthetic gene construction entails *in vitro* synthesis of a designed polynucleotide molecule to encode a polypeptide of interest. Gene synthesis can be performed utilizing a number of techniques, such as the multiplex microchip-based technology described by Tian et al. (2004, Nature 432: 1050-1054) and similar technologies wherein oligonucleotides are synthesized and assembled upon photo-programmable microfluidic chips.

Single or multiple amino acid substitutions, deletions, and/or insertions can be made and tested using known methods of mutagenesis, recombination, and/or shuffling, followed by a relevant screening procedure, such as those disclosed by Reidhaar-Olson and Sauer, 1988, Science 241: 53-57; Bowie and Sauer, 1989, Proc. Natl. Acad. Sci. USA 86: 2152-2156; WO 95/17413; or WO 95/22625. Other methods that can be used include error-prone PCR, phage display (*e.g.*, Lowman et al., 1991, Biochemistry 30: 10832-10837; U.S. Patent No. 5,223,409; WO 92/06204) and region-directed mutagenesis (Derbyshire et al., 1986, Gene 46: 145; Ner et al., 1988, DNA 7: 127).

Mutagenesis/shuffling methods can be combined with high-throughput, automated screening methods to detect activity of cloned, mutagenized polypeptides expressed by host cells (Ness et al., 1999, Nature Biotechnology 17: 893-896). Mutagenized DNA molecules that encode active polypeptides can be recovered from the host cells and rapidly sequenced using standard methods in the art. These methods allow the rapid determination of the importance of individual amino acid residues in a polypeptide.

Semi-synthetic gene construction is accomplished by combining aspects of synthetic gene construction, and/or site-directed mutagenesis, and/or random mutagenesis, and/or shuffling. Semi-synthetic construction is typified by a process utilizing polynucleotide fragments that are synthesized, in combination with PCR techniques. Defined regions of genes may thus be synthesized *de novo*, while other regions may be amplified using site-specific mutagenic primers, while yet other regions may be subjected to error-prone PCR or non-error prone PCR amplification. Polynucleotide subsequences may then be shuffled.

Disclosed is also a method of improving oxidation stability of a parent alpha-amylase having the amino acid sequence of SEQ ID NO: 3 or 4, or having at least 80% sequence identity thereto, wherein the method comprises the steps of;

a) introducing a substitution in one or more positions providing oxidation stability in the parent alpha-amylase; and
b) optionally, introducing a substitution and/or deletion of two, three, or four positions corresponding to positions R181, G182, D183, and G184 of the amino acid sequence as set forth in SEQ ID NO: 3,

wherein the variant has at least 80%, such as at least 85%, such as at least 90%, such as at least 95%, such as at least 98%, such as at least 99%, but less than 100% sequence identity with the amino acid sequence as set forth in SEQ ID NO: 3, and wherein the variant has alpha-amylase activity and improved oxidation stability compared to the parent alpha-amylase.

In one embodiment, the method of improving oxidation stability of a parent alpha-amylase having the amino acid sequence of SEQ ID NO: 3 or 4, or having at least 80% sequence identity thereto, wherein the method comprises the steps of;

a) introducing a substitution in the position corresponding to position M202 of the amino acid sequence as set forth in SEQ ID NO: 3; and
b) introducing a substitution and/or deletion of two, three, or four positions corresponding to positions R181, G182, D183, and G184 of the amino acid sequence as set forth in SEQ ID NO: 3,

wherein the variant has at least 80%, such as at least 85%, such as at least 90%, such as at least 95%, such as at least 98%, such as at least 99%, but less than 100% sequence identity with the amino acid sequence as set forth in SEQ ID NO: 3, and wherein the variant has alpha-amylase activity and improved oxidation stability compared to the parent alpha-amylase.

In one embodiment, the variant has at least 50%, such as at least 60%, or at least 70%, or at least 80%, or at least 90%, or at least 100% of the activity of the parent polypeptide having the amino acid sequence of SEQ ID NO: 4.

In another embodiment, the variant has at least 50%, such as at least 60%, or at least 70%, or at least 80%, or at least 90%, or at least 100% of the activity of the parent polypeptide having the amino acid sequence of SEQ ID NO: 3.

In one embodiment, the activity is determined according to a Phadebas assay.

The alpha-amylase activity may be determined by a method using the Phadebas substrate (from for example Magle Life Sciences, Lund, Sweden). A Phadebas tablet includes interlinked starch polymers that are in the form of globular microspheres that are insoluble in water. A blue dye is covalently bound to these microspheres. The interlinked starch polymers in the microsphere are degraded at a speed that is proportional to the alpha-amylase activity. When the alpha-amylase degrades the starch polymers, the released blue dye is water soluble and concentration of dye can be determined by measuring absorbance at 620nm. The concentration of blue is proportional to the alpha-amylase activity in the sample.

The variant sample to be analyzed is diluted in activity buffer with the desired pH. Two substrate tablets are suspended in 5mL activity buffer and mixed on magnetic stirrer. During mixing of substrate transfer 150μl to microtiter plate (MTP) or PCR-MTP. Add 30μl diluted amylase sample to 150μl substrate and mix. Incubate for 15 minutes at 37°C. The reaction is stopped by adding 30μl 1M NaOH and mix. Centrifuge MTP for 5 minutes at 4000xg. Transfer 100μl to new MTP and measure absorbance at 620nm.

The alpha-amylase sample should be diluted so that the absorbance at 620nm is between 0 and 2.2, and is within the linear range of the activity assay.

Thus, in one embodiment, the activity is determined by a method comprising the steps of;

a) incubating an alpha-amylase variant according to the invention with a dyed amylose substrate for 15 minute at 37°C; and
b) measuring the absorption at OD 620 nm.

In a further embodiment, the activity is determined by a method comprising the steps of;

a) incubating an alpha-amylase variant according to the invention with a dyed amylose substrate for 15 minute at 37°C; and
b) centrifuging the sample;
c) transferring the supernatant to reader plate, and

measuring the absorption at OD 620 nm.

In another embodiment, the activity is determined according to the pNP-G7 assay as described in Example 2.

**Fermentation Broth Formulations or Cell Compositions**

Disclosed is a fermentation broth formulation or a cell composition comprising a variant used in accordance with the present invention. Disclosed is the fermentation broth formulation or cell composition comprising a variant comprising a substitution in one or more positions providing oxidation stability of the variant. In another embodiment, the fermentation broth formulation or cell composition comprises a polynucleotide encoding a variant comprising a substitution in one or more positions providing oxidation stability of the variant, nucleic acid construct encoding a variant comprising a substitution in one or more positions providing oxidation stability of the variant, or an expression vector encoding a variant comprising a substitution in one or more positions providing oxidation stability of the variant. The fermentation broth product may further comprise additional ingredients used in the fermentation process, such as, for example, cells (including, the host cells containing the gene encoding the polypeptide of the present invention which are used to produce the polypeptide of interest), cell debris, biomass, fermentation media and/or fermentation products. In some embodiments, the composition is a cell-killed whole broth containing organic acid(s), killed cells and/or cell debris, and culture medium.

The term "fermentation broth" as used herein refers to a preparation produced by cellular fermentation that undergoes no or minimal recovery and/or purification. For example, fermentation broths are produced when microbial cultures are grown to saturation, incubated under carbon-limiting conditions to allow protein synthesis (*e.g.*, expression of enzymes by host cells) and secretion into cell culture medium. The fermentation broth may comprise unfractionated or fractionated contents of the fermentation materials derived at the end of the fermentation. Typically, the fermentation broth is unfractionated and comprises the spent culture medium and cell debris present after the microbial cells (*e.g.*, filamentous fungal cells) are removed, *e.g.*, by centrifugation. In some embodiments, the fermentation broth comprises spent cell culture medium, extracellular enzymes, and viable and/or nonviable microbial cells.

In one embodiment, the fermentation broth formulation and cell compositions comprise a first organic acid

component comprising at least one 1-5 carbon organic acid and/or a salt thereof and a second organic acid component comprising at least one 6 or more carbon organic acid and/or a salt thereof. In a particular embodiment, the first organic acid component is acetic acid, formic acid, propionic acid, a salt thereof, or a mixture of two or more of the foregoing and the second organic acid component is benzoic acid, cyclohexanecarboxylic acid, 4-methylvaleric acid, phenylacetic acid, a salt thereof, or a mixture of two or more of the foregoing.

In one embodiment, the composition comprises an organic acid(s), and optionally further comprises killed cells and/or cell debris. In one embodiment, the killed cells and/or cell debris are removed from a cell-killed whole broth to provide a composition that is free of these components.

The fermentation broth formulations or cell compositions may further comprise a preservative and/or anti-microbial (*e.g.*, bacteriostatic) agent, including, but not limited to, sorbitol, sodium chloride, potassium sorbate, and others known in the art.

The cell-killed whole broth or composition may comprise the unfractionated contents of the fermentation materials derived at the end of the fermentation. Typically, the cell-killed whole broth or composition comprises the spent culture medium and cell debris present after the microbial cells (*e.g.*, filamentous fungal cells) are grown to saturation, incubated under carbon-limiting conditions to allow protein synthesis. In some embodiments, the cell-killed whole broth or composition comprises the spent cell culture medium, extracellular enzymes, and killed filamentous fungal cells. In some embodiments, the microbial cells present in the cell-killed whole broth or composition may be permeabilized and/or lysed using methods known in the art.

A whole broth or cell composition as described herein is typically a liquid, but may comprise insoluble components, such as killed cells, cell debris, culture media components, and/or insoluble enzyme(s). In some embodiments, insoluble components may be removed to provide a clarified liquid composition.

The whole broth formulations and cell compositions of the present invention may be produced by a method described in WO 90/15861 or WO 2010/096673.

## Compositions

The present invention also relates to compositions comprising a variant used in accordance with the invention further comprises one or more surfactants and one or more bleaching systems and one or more builders. Preferably, the compositions are enriched in such a variant. The term "enriched" means that the alpha-amylase activity of the composition has been increased, *e.g.*, with an enrichment factor of 1.1.

In one embodiment, the composition comprises a variant comprising a M202L substitution in the position corresponding to position M202 of the amino acid sequence as set forth in SEQ ID NO: 3, and a substitution and/or deletion of two, three, orfour positions corresponding to positions R181, G182, D183, and G184 of the amino acid sequence as set forth in SEQ ID NO: 3.

The compositions may be prepared in accordance with methods known in the art and may be in the form of a liquid or a dry composition. For instance, the composition may be in the form of a granulate or a microgranulate. The variant may be stabilized in accordance with methods known in the art.

Accordingly, in one embodiment, the composition is a liquid laundry or liquid dish wash composition, such as an Automatic Dish Wash (ADW) liquid detergent composition, or a powder laundry, such as a soap bar, or powder dish wash composition, such as an ADW detergent composition.

In one embodiment, the composition further comprises one or more surfactants, one or more sulfonated polymers, one or more chelators, one or more bleaching systems, and one or more builders.

The choice of additional components is within the skills of the skilled person in the art and includes conventional ingredients, including the exemplary non-limiting components set forth below. The choice of components may include, for fabric care, the consideration of the type of fabric to be cleaned, the type and/or degree of soiling, the temperature at which cleaning is to take place, and the formulation of the detergent product. Although components mentioned below are categorized by general header according to a particular functionality, this is not to be construed as a limitation, as a component may comprise additional functionalities as will be appreciated by the skilled artisan.

In one embodiment of the present invention, the variant of the present invention may be added to a detergent composition in an amount corresponding to 0.001-100 mg of protein, such as 0.01-100 mg of protein, preferably 0.005-50 mg of protein, more preferably 0.01-25 mg of protein, even more preferably 0.05-10 mg of protein, most preferably 0.05-5 mg of protein, and even most preferably 0.01-1 mg of protein per liter of wash liquor. The term "protein" in this context is contemplated to be understood to include a variant according to the present invention.

A composition for use in automatic dish wash (ADW), for example, may include 0.0001%-50%, such as 0.001%-20%, such as 0.01%-10%, such as 0.05-5% of enzyme protein by weight of the composition.

A composition for use in laundry granulation, for example, may include 0.0001%-50%, such as 0.001%-20%, such as 0.01%-10%, such as 0.05%-5% of enzyme protein by weight of the composition.

A composition for use in laundry liquid, for example, may include 0.0001%-10%, such as 0.001-7%, such as

0.1%-5% of enzyme protein by weight of the composition.

The variants of the invention as well as the further active components, such as additional enzymes, may be stabilized using conventional stabilizing agents, *e.g.*, a polyol such as propylene glycol or glycerol, a sugar or sugar alcohol, lactic acid, boric acid, or a boric acid derivative, *e.g.*, an aromatic borate ester, or a phenyl boronic acid derivative such as 4-formylphenyl boronic acid, and the composition may be formulated as described in, for example, WO92/19709 and WO92/19708.

In certain markets different wash conditions and, as such, different types of detergents are used. This is disclosed in *e.g.* EP 1 025 240. For example, in Asia (Japan) a low detergent concentration system is used, while the United States uses a medium detergent concentration system, and Europe uses a high detergent concentration system.

A low detergent concentration system includes detergents where less than about 800 ppm of detergent components are present in the wash water. Japanese detergents are typically considered low detergent concentration system as they have approximately 667 ppm of detergent components present in the wash water.

A medium detergent concentration includes detergents where between about 800 ppm and about 2000ppm of detergent components are present in the wash water. North American detergents are generally considered to be medium detergent concentration systems as they have approximately 975 ppm of detergent components present in the wash water.

A high detergent concentration system includes detergents where greater than about 2000 ppm of detergent components are present in the wash water. European detergents are generally considered to be high detergent concentration systems as they have approximately 4500-5000 ppm of detergent components in the wash water.

Latin American detergents are generally high suds phosphate builder detergents and the range of detergents used in Latin America can fall in both the medium and high detergent concentrations as they range from 1500 ppm to 6000 ppm of detergent components in the wash water. Such detergent compositions are all embodiments of the invention.

A variant of the present invention may also be incorporated in the detergent formulations disclosed in WO97/07202.

Examples are given herein of preferred uses of the compositions of the present invention. The dosage of the composition and other conditions under which the composition is used may be determined on the basis of methods known in the art.

In one embodiment, the composition further comprises a bleaching system.

The term "bleaching system" as used herein, refers to inorganic and organic bleaches suitable cleaning actives. The terms "bleaching system" and "bleaches" may be used interchangeably herein and constitute the same meaning and purpose, unless explicitly stated otherwise. Inorganic bleaches include perhydrate salts such as perborate, percarbonate, perphosphate, persulfate and persilicate salts. The inorganic perhydrate salts are normally the alkali metal salts. The inorganic perhydrate salt may be included as the crystalline solid without additional protection. Alternatively, the salt can be coated.

Alkali metal percarbonates, particularly sodium percarbonate are preferred perhydrates for use herein. The percarbonate is most preferably incorporated into the products in a coated form which provides in-product stability. A suitable coating material providing in product stability comprises mixed salt of a water-soluble alkali metal sulphate and carbonate. Such coatings together with coating processes have previously been described in GB 1,466,799. The weight ratio of the mixed salt coating material to percarbonate lies in the range from 1:200 to 1:4, more preferably from 1:99 to 1:9, and most preferably from 1:49 to 1:19. Preferably, the mixed salt is of sodium sulphate and sodium carbonate which has the general formula Na2S04.n.Na2CO3 wherein n is from 0.1 to 3, preferably n is from 0.3 to 1.0 and most preferably n is from 0.2 to 0.5.

Another suitable coating material providing in product stability, comprises sodium silicate of $SiO_2$:$Na_2O$ ratio from 1.8:1 to 3.0:1, preferably 1.8:1 to 2.4:1, and/or sodium metasilicate, preferably applied at a level of from 2% to 10%, (normally from 3% to 5%) of SiO2 by weight of the inorganic perhydrate salt. Magnesium silicate can also be included in the coating. Coatings that comprise silicate and borate salts or boric acids or other inorganics are also suitable.

Other coatings which comprise waxes, oils, fatty soaps can also be used advantageously within the present invention.

Potassium peroxymonopersulfate is another inorganic perhydrate salt of utility herein. Typical organic bleaches are organic peroxyacids including diacyl and tetraacylperoxides, especially diperoxydodecanedioc acid, diperoxytetradecanedioc acid, and diperoxyhexadecanedioc acid. Dibenzoyl peroxide is a preferred organic peroxyacid herein. Mono- and diperazelaic acid, mono- and diperbrassylic acid, and Nphthaloylaminoperoxicaproic acid are also suitable herein. The diacyl peroxide, especially dibenzoyl peroxide, should preferably be present in the form of particles having a weight average diameter of from about 0.1 to about 100 microns, preferably from about 0.5 to about 30 microns, more preferably from about 1 to about 10 microns. Preferably, at least about 25%, more preferably at least about 50%, even more preferably at least about 75%, most preferably at least about 90%, of the particles are smaller than 10 microns, preferably smaller than 6 microns. Diacyl peroxides within the above particle size range have also been found to provide better stain removal especially from plastic dishware, while minimizing undesirable deposition and filming during use in automatic dishwashing machines, than larger diacyl peroxide particles. The preferred diacyl peroxide particle size thus allows the formulator to obtain good stain removal with a low level of diacyl peroxide, which reduces deposition and filming. Conversely, as diacyl peroxide particle size increases, more diacyl peroxide is needed for good stain removal, which increases deposition on surfaces encountered during the dishwashing process.

Further typical organic bleaches include the peroxy acids, particular examples being the alkylperoxy acids and the arylperoxy acids. Preferred representatives are (a) peroxybenzoic acid and its ring-substituted derivatives, such as alkylperoxybenzoic acids, but also peroxy-[alpha]-naphthoic acid and magnesium monoperphthalate, (b) the aliphatic or substituted aliphatic peroxy acids, such as peroxylauric acid, peroxystearic acid, [epsilon]-phthalimidoperoxycaproic acid[phthaloiminoperoxyhexanoic acid (PAP)], o-carboxybenzamidoperoxycaproic acid, N-nonenylamidoperadipic acid and N-nonenylamidopersuccinates, and (c) aliphatic and araliphatic peroxydicarboxylic acids, such as 1,12-diperoxycarboxylic acid, 1,9-diperoxyazelaic acid, diperoxysebacic acid, diperoxybrassylic acid, the diperoxyphthalic acids, 2-decyldiperoxybutane-1,4-dioic acid, N,N-terephthaloyldi(6-aminopercaproic acid).

In one embodiment, the composition further comprises a bleach activator, bleach catalyst, silicate and/or metal care agent(s).

The term "bleach activators" as used herein, refers to a typically organic peracid precursor which enhances the bleaching action in the course of cleaning at temperatures of 60°C and below. Bleach activators suitable for use herein include compounds which, under perhydrolysis conditions, give aliphatic peroxoycarboxylic acids having preferably from 1 to 10 carbon atoms, in particular from 2 to 4 carbon atoms, and/or optionally substituted perbenzoic acid. Suitable substances bear O-acyl and/or N-acyl groups of the number of carbon atoms specified and/or optionally substituted benzoyl groups. Preference is given to polyacylated alkylenediamines, in particular tetraacetylethylenediamine (TAED), acylated triazine derivatives, in particular 1,5-diacetyl-2,4- dioxohexahydro-1,3,5-triazine (DADHT), acylated glycolurils, in particular tetraacetylglycoluril (TAGU), N-acylimides, in particular N-nonanoylsuccinimide (NOSI), acylated phenolsulfonates, in particular n-nonanoyl- or isononanoyloxybenzenesulfonate (n- or iso-NOBS), carboxylic anhydrides, in particular phthalic anhydride, acylated polyhydric alcohols, in particular triacetin, ethylene glycol diacetate and 2,5-diacetoxy-2,5-dihydrofuran and also triethylacetyl citrate (TEAC). Bleach activators if included in the compositions of the invention are in a level of from about 0.1 to about 10%, preferably from about 0.5 to about 2% by weight of the composition.

The term "bleach catalysts" as used herein, refers to manganese triazacyclononane and related complexes (US-A-4246612, US-A-5227084); Co, Cu, Mn and Fe bispyridylamine and related complexes (US-A-5114611); and pentamine acetate cobalt(III) and related complexes (US-A- 4810410). A complete description of bleach catalysts suitable for use herein can be found in WO 99/06521, pages 34, line 26 to page 40, line 16. Bleach catalyst if included in the compositions of the invention are in a level of from about 0.1 to about 10%, preferably from about 0.5 to about 2% by weight of the composition.

Oxidoreductases, for example oxidases, oxygenases, catalases, peroxidases such as halo-, chloro-, bromo-, lignin, glucose, or manganese peroxidases, dioxygenases, or laccases (phenoloxidases, polyphenoloxidases), can also be used according to the present invention to intensify the bleaching effect. Advantageously, preferably organic, particularly preferably aromatic compounds that interact with the enzymes are additionally added in order to enhance the activity of the relevant oxidoreductases (enhancers) or, if there is a large difference in redox potentials between the oxidizing enzymes and the stains, to ensure electron flow (mediators).

The term "silicates" as used herein, refers to sodium silicates such as sodium disilicate, sodium metasilicate and crystalline phyllosilicates. Silicates if present are at a level of from about 1 to about 20%, preferably from about 5 to about 15% by weight of composition.

The term "metal care agents" as used herein, refers to prevention or reduction of tarnishing, corrosion or oxidation of metals, including aluminium, stainless steel and non-ferrous metals, such as silver and copper. Suitable examples include one or more of the following:

(a) benzatriazoles, including benzotriazole or bis-benzotriazole and substituted derivatives thereof. Benzotriazole derivatives are those compounds in which the available substitution sites on the aromatic ring are partially or completely substituted. Suitable substituents include linear or branch-chain Ci-C20- alkyl groups and hydroxyl, thio, phenyl or halogen such as fluorine, chlorine, bromine and iodine.

(b) metal salts and complexes chosen from the group consisting of zinc, manganese, titanium, zirconium, hafnium, vanadium, cobalt, gallium and cerium salts and/or complexes, the metals being in one of the oxidation states II, III, IV, V or VI. In one aspect, suitable metal salts and/or metal complexes may be chosen from the group consisting of Mn(II) sulphate, Mn(II) citrate, Mn(II) stearate, Mn(II) acetylacetonate, K^TiF6, K^ZrF6, CoSO4, Co(NOs)2 and Ce(NOs)3, zinc salts, for example zinc sulphate, hydrozincite or zinc acetate.;

(c) silicates, including sodium or potassium silicate, sodium disilicate, sodium metasilicate, crystalline phyllosilicate and mixtures thereof.

Further suitable organic and inorganic redox-active substances that act as silver/copper corrosion inhibitors are disclosed in WO 94/26860 and WO 94/26859. Preferably the composition of the invention comprises from 0.1 to 5% by weight of the composition of a metal care agent, preferably the metal care agent is a zinc salt.

In one embodiment, the composition further comprises a surfactant.

The term "surfactant" as used herein, refers in particular to a dish washing component, which is a non-ionic compound. Suitable nonionic surfactants include, but are not limited to low-foaming nonionic (LFNI) surfactants. A LFNI surfactant is most typically used in an automatic dishwashing composition because of the improved water- sheeting action (especially from glassware) which they confer to the automatic dishwashing composition. They also may encompass non-silicone, phosphate or nonphosphate polymeric materials which are known to defoam food soils encountered in automatic dishwashing. The LFNI surfactant may have a relatively low cloud point and a high hydrophilic-lipophilic balance (HLB). Cloud points of 1% solutions in water are typically below about 32°C. and alternatively lower, e.g., 0°C, for optimum control of sudsing throughout a full range of water temperatures. If desired, a biodegradable LFNI surfactant having the above properties may be used.

An LFNI surfactant may include, but is not limited to: alkoxylated surfactants, especially ethoxylates derived from primary alcohols, and blends thereof with more sophisticated surfactants, such as the polyoxypropylene/polyoxyethylene/polyoxypropylene reverse block polymers. Suitable block polyoxyethylene-polyoxypropylene polymeric compounds that meet the requirements may include those based on ethylene glycol, propylene glycol, glycerol, trimethylolpropane and ethylenediamine, and mixtures thereof. Polymeric compounds made from a sequential ethoxylation and propoxylation of initiator compounds with a single reactive hydrogen atom, such as C 12- is aliphatic alcohols, do not generally provide satisfactory suds control in Automatic dishwashing compositions. However, certain of the block polymer surfactant compounds designated as PLURONIC(R) and TETRONIC(R) by the BASF-Wyandotte Corp., Wyandotte, Mich., are suitable in Automatic dishwashing compositions. The LFNI surfactant can optionally include a propylene oxide in an amount up to about 15% by weight. Other LFNI surfactants can be prepared by the processes described in U.S. Pat. No. 4,223,163. The LFNI surfactant may also be derived from a straight chain fatty alcohol containing from about 16 to about 20 carbon atoms (C16-C20 alcohol), alternatively a Ci8 alcohol, condensed with an average of from about 6 to about 15 moles, or from about 7 to about 12 moles, and alternatively, from about 7 to about 9 moles of ethylene oxide per mole of alcohol. The ethoxylated nonionic surfactant so derived may have a narrow ethoxylate distribution relative to the average.

In certain embodiments, a LFNI surfactant having a cloud point below 30°C. may be present in an amount from about 0.01% to about 60%, or from about 0.5% to about 10% by weight, and alternatively, from about 1% to about 5% by weight of the composition

In preferred embodiments, the surfactant is a non-ionic surfactant or a non-ionic surfactant system having a phase inversion temperature, as measured at a concentration of 1% in distilled water, between 40 and 70°C, preferably between 45 and 65°C. By a "non-ionic surfactant system" is meant herein a mixture of two or more non-ionic surfactants. Preferred for use herein are non-ionic surfactant systems. They seem to have improved cleaning and finishing properties and stability in product than single non-ionic surfactants. Suitable nonionic surfactants include: i) ethoxylated non-ionic surfactants prepared by the reaction of a monohydroxy alkanol or alkyphenol with 6 to 20 carbon atoms with preferably at least 12 moles particularly preferred at least 16 moles, and still more preferred at least 20 moles of ethylene oxide per mole of alcohol or alkylphenol; ii) alcohol alkoxylated surfactants having a from 6 to 20 carbon atoms and at least one ethoxy and propoxy group. Preferred for use herein are mixtures of surfactants i) and ii).

Another suitable non-ionic surfactants are epoxy-capped poly(oxyalkylated) alcohols represented by the formula:

$$R_1O[CH_2CH(CH_3)O]_x[CH_2CH_2O]_y[CH_2CH(OH)R_2] \qquad (I)$$

wherein $R_1$ is a linear or branched, aliphatic hydrocarbon radical having from 4 to 18 carbon atoms; $R_2$ is a linear or branched aliphatic hydrocarbon radical having from 2 to 26 carbon atoms; x is an integer having an average value of from 0.5 to 1.5, more preferably about 1; and y is an integer having a value of at least 15, more preferably at least 20.

Preferably, the surfactant of formula I has at least about 10 carbon atoms in the terminal epoxide unit $[CH_2CH(OH)R_2]$. Suitable surfactants of formula I are Olin Corporation's POLY-TERGENT(R) SLF- 18B nonionic surfactants, as described, for example, in WO 94/22800, published October 13, 1994 by Olin Corporation.

Preferably non-ionic surfactants and/or system herein have a Draves wetting time of less than 360 seconds, preferably less than 200 seconds, more preferably less than 100 seconds and especially less than 60 seconds as measured by the Draves wetting method (standard method ISO 8022 using the following conditions; 3-g hook, 5-g cotton skein, 0.1% by weight aqueous solution at a temperature of 25 °C). Amine oxides surfactants are also useful in the present invention as anti-redeposition surfactants include linear and branched compounds having the formula:

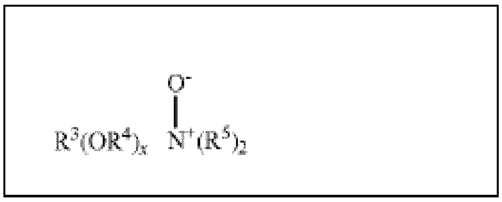

wherein $R^3$ is selected from an alkyl, hydroxyalkyl, acylamidopropoyl and alkyl phenyl group, or mixtures thereof, containing from 8 to 26 carbon atoms, preferably 8 to 18 carbon atoms; $R^4$ is an alkylene or hydroxyalkylene group containing from 2 to 3 carbon atoms, preferably 2 carbon atoms, or mixtures thereof; x is from 0 to 5, preferably from 0 to 3; and each $R^5$ is an alkyl or hydroxyalkyl group containing from 1 to 3, preferably from 1 to 2 carbon atoms, or a polyethylene oxide group containing from 1 to 3, preferable 1, ethylene oxide groups. The $R^5$ groups can be attached to each other, e.g., through an oxygen or nitrogen atom, to form a ring structure.

These amine oxide surfactants in particular include $C_{10}$-$C_{18}$ alkyl dimethyl amine oxides and $C_8$-$C_{18}$ alkoxy ethyl dihydroxyethyl amine oxides. Examples of such materials include dimethyloctylamine oxide, diethyldecylamine oxide, bis-(2-hydroxyethyl)dodecylamine oxide, dimethyldodecylamine oxide, dipropyltetradecylamine oxide, methylethylhexadecylamine oxide, dodecylamidopropyl dimethylamine oxide, cetyl dimethylamine oxide, stearyl dimethylamine oxide, tallow dimethylamine oxide and dimethyl-2-hydroxyoctadecylamine oxide. Preferred are $C_{10}$-$C_{18}$ alkyl dimethylamine oxide, and $C_{10}$-$C_{18}$ acylamido alkyl dimethylamine oxide. Surfactants and especially non-ionic surfactants may be present in amounts from 0 to 10% by weight, preferably from 0.1% to 10%, and most preferably from 0.25% to 6%.

In one embodiment, the composition further comprises a sulfonated polymer.

The term "sulfonated polymer" as used herein, refers to polymers containing sulfonic acid or sulfonate functional groups.

The polymer, if used, is used in any suitable amount from about 0.1% to about 20%, preferably from 1% to about 15%, more preferably from 2% to 10% by weight of the composition. Sulfonated/carboxylated polymers are particularly suitable for the compositions contained in the pouch of the invention.

Suitable sulfonated/carboxylated polymers described herein may have a weight average molecular weight of less than or equal to about 100,000 Da, or less than or equal to about 75,000 Da, or less than or equal to about 50,000 Da, or from about 3,000 Da to about 50,000, preferably from about 5,000 Da to about 45,000 Da.

As noted herein, the sulfonated/carboxylated polymers may comprise (a) at least one structural unit derived from at least one carboxylic acid monomer having the general formula (I):

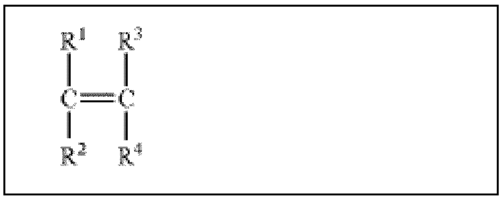

wherein $R^1$ to $R^4$ are independently hydrogen, methyl, carboxylic acid group or $CH_2COOH$ and wherein the carboxylic acid groups can be neutralized; (b) optionally, one or more structural units derived from at least one nonionic monomer having the general formula (II):

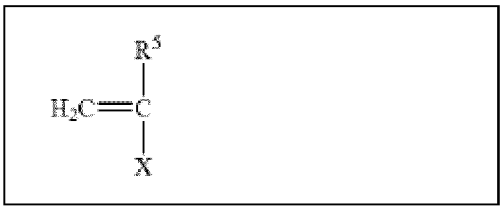

wherein $R^5$ i is hydrogen, $C_1$ to $C_6$ alkyl, or $C_1$ to $C_6$ hydroxyalkyl, and X is either aromatic (with $R^5$ being hydrogen or methyl when X is aromatic) or X is of the general formula (III):

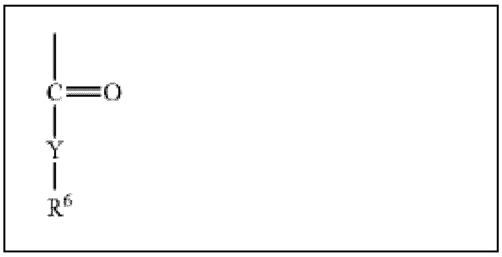

wherein $R^6$ is (independently of $R^5$) hydrogen, $C_1$ to $C_6$ alkyl, or $C_1$ to $C_6$ hydroxyalkyl, and Y is O or N; and at least one structural unit derived from at least one sulfonic acid monomer having the general formula (IV):

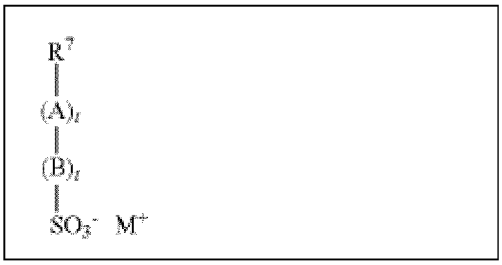

wherein $R^7$ is a group comprising at least one $sp^2$ bond, A is O, N, P, S or an amido or ester linkage, B is a mono- or polycyclic aromatic group or an aliphatic group, each t is independently 0 or 1 , and $M^+$ is a cation. In one aspect, $R^7$ is a $C_2$ to $C_6$alkene. In another aspect, $R^7$ is ethene, butene or propene.

Preferred carboxylic acid monomers include one or more of the following: acrylic acid, maleic acid, itaconic acid, methacrylic acid, or ethoxylate esters of acrylic acids, acrylic and methacrylic acids being more preferred. Preferred sulfonated monomers include one or more of the following: sodium (meth) allyl sulfonate, vinyl sulfonate, sodium phenyl (meth) allyl ether sulfonate, or 2-acrylamido-methyl propane sulfonic acid. Preferred non-ionic monomers include one or more of the following: methyl (meth) acrylate, ethyl (meth) acrylate, t-butyl (meth) acrylate, methyl (meth) acrylamide, ethyl (meth) acrylamide, t-butyl (meth) acrylamide, styrene, or [alpha]-methyl styrene.

Preferably, the polymer comprises the following levels of monomers: from about 40 to about 90%, preferably from about 60 to about 90% by weight of the polymer of one or more carboxylic acid monomer; from about 5 to about 50%, preferably from about 10 to about 40% by weight of the polymer of one or more sulfonic acid monomer; and optionally from about 1 % to about 30%, preferably from about 2 to about 20% by weight of the polymer of one or more non-ionic monomer. An especially preferred polymer comprises about 70% to about 80% by weight of the polymer of at least one carboxylic acid monomer and from about 20% to about 30% by weight of the polymer of at least one sulfonic acid monomer.

The carboxylic acid is preferably (meth)acrylic acid. The sulfonic acid monomer is preferably one of the following: 2-acrylamido methyl- 1-propanesulfonic acid, 2-methacrylamido-2-methyl- 1-propanesulfonic acid, 3-methacrylamido-2-hydroxypropanesulfonic acid, allysulfonic acid, methallysulfonic acid, allyloxybenzenesulfonic acid, methallyloxybenzensulfonic acid, 2-hydroxy-3-(2-propenyloxy)propanesulfonic acid, 2-methyl-2-propene-1-sulfonic acid, styrene sulfonic acid, vinylsulfonic acid, 3-sulfopropyl acrylate, 3-sulfopropyl methacrylate, sulfomethylacrylamid, sulfomethylmethacrylamide, and water soluble salts thereof. The unsaturated sulfonic acid monomer is most preferably 2-acrylamido-2-propanesulfonic acid (AMPS).

Preferred commercial available polymers include: Alcosperse 240, Aquatreat AR 540 and Aquatreat MPS supplied by Alco Chemical; Acumer 3100, Acumer2000, Acusol 587G and Acusol 588G supplied by Rohm & Haas; Goodrich K-798, K-775 and K-797 supplied by BF Goodrich; and ACP 1042 supplied by ISP technologies Inc. Particularly preferred polymers are Acusol 587G and Acusol 588G supplied by Rohm & Haas.

In the polymers, all or some of the carboxylic or sulfonic acid groups may be present in neutralized form, *i.e.* the acidic hydrogen atom of the carboxylic and/or sulfonic acid group in some or all acid groups can be replaced with metal ions, preferably alkali metal ions and in particular with sodium ions.

In one embodiment, the composition further comprises a hydrotrope.

The term "hydrotrope" as used herein, refers to a compound that solubilises hydrophobic compounds in aqueous solutions (or oppositely, polar substances in a non-polar environment). Typically, hydrotropes have both hydrophilic and a hydrophobic character (so-called amphiphilic properties as known from surfactants); however the molecular structure of hydrotropes generally do not favor spontaneous self-aggregation, see *e.g.* review by Hodgdon and Kaler (2007),

Current Opinion in Colloid & Interface Science 12: 121-128. Hydrotropes do not display a critical concentration above which self-aggregation occurs as found for surfactants and lipids forming miceller, lamellar or other well defined mesophases. Instead, many hydrotropes show a continuous-type aggregation process where the sizes of aggregates grow as concentration increases. However, many hydrotropes alter the phase behavior, stability, and colloidal properties of systems containing substances of polar and non-polar character, including mixtures of water, oil, surfactants, and polymers. Hydrotropes are classically used across industries from pharma, personal care, food, to technical applications. Use of hydrotropes in detergent compositions allow for example more concentrated formulations of surfactants (as in the process of compacting liquid detergents by removing water) without inducing undesired phenomena such as phase separation or high viscosity.

The detergent may contain 0-10% by weight, for example 0-5% by weight, such as about 0.5 to about 5%, or about 3% to about 5%, of a hydrotrope. Any hydrotrope known in the art for use in detergents may be utilized. Non-limiting examples of hydrotropes include sodium benzenesulfonate, sodium p-toluene sulfonate (STS), sodium xylene sulfonate (SXS), sodium cumene sulfonate (SCS), sodium cymene sulfonate, amine oxides, alcohols and polyglycolethers, sodium hydroxynaphthoate, sodium hydroxynaphthalene sulfonate, sodium ethylhexyl sulfate, and combinations thereof.

In particular, a composition according to the present invention further comprises a chelator.

The term "chelator" as used herein, refers to chemicals which form molecules with certain metal ions, inactivating the ions so that they cannot react with other elements. Thus, a chelator may be defined as a binding agent that suppresses chemical activity by forming chelates. Chelation is the formation or presence of two or more separate bindings between a ligand and a single central atom. The ligand may be any organic compound, a silicate or a phosphate. In the present context the term "chelating agents" comprises chelants, chelating agent, chelating agents, complexing agents, or sequestering agents that forms water-soluble complexes with metal ions such as calcium and magnesium. The chelate effect describes the enhanced affinity of chelating ligands for a metal ion compared to the affinity of a collection of similar non-chelating ligands for the same metal. Chelating agents having binding capacity with metal ions, in particular calcium (Ca2+) ions, and has been used widely in detergents and compositions in general for wash, such as laundry or dish wash. Chelating agents have however shown themselves to inhibit enzymatic activity. The term chelating agent is used in the present application interchangeably with "complexing agent" or "chelating agent" or "chelant".

Since most alpha-amylases are calcium sensitive the presence of chelating agents these may impair the enzyme activity. The calcium sensitivity of alpha-amylases may be determined by incubating a given alpha-amylase in the presence of a strong chelating agent and analyze the impact of this incubation on the activity of the alpha-amylase in question. A calcium sensitive alpha-amylase will lose a major part or all of its activity during the incubation. Chelating agent may be present in the composition in an amount from 0.0001 wt% to 20wt%, preferably from 0.01 to 10 wt%, more preferably from 0.1 to 5wt%.

Non-limiting examples of chelating agents are; EDTA, DTMPA, HEDP, EDDS, and citrate. Thus, in one embodiment, the composition comprises a variant according to the invention and a chelating agent, such as EDTA, DTMPA, HEDP, EDDS, or citrate.

The term "EDTA" as used herein, refers to ethylene-diamine-tetra-acetic acid which falls under the definition of "strong chelating agents".

The term "DTMPA" as used herein, refers to diethylenetriamine penta(methylene phosphonic acid). DTMPA can inhibit the scale formation of carbonate, sulfate and phosphate.

The term "HEDP" as used herein, refers to hydroxy-ethane diphosphonic acid, which falls under the definition of "strong chelating agents".

The term "EDDS" as used herein, refers to an aminopolycarboxylic acid, which falls under the definition of "strong chelating agents".

The chelate effect or the chelating effect describes the enhanced affinity of chelating ligands for a metal ion compared to the affinity of a collection of similar nonchelating ligands for the same metal. However, the strength of this chelate effect can be determined by various types of assays or measure methods thereby differentiating or ranking the chelating agents according to their chelating effect (or strength).

In an assay the chelating agents may be characterized by their ability to reduce the concentration of free calcium ions (Ca2+) from 2.0 mM to 0.10 mM or less at pH 8.0, e.g. by using a test based on the method described by M.K.Nagarajan et al., JAOCS, Vol. 61, no. 9 (September 1984), pp. 1475-1478.

For reference, a chelator having the same ability to reduce the concentration of free calcium ions (Ca2+) from 2.0 mM to 0.10 mM at pH as EDTA at equal concentrations of the chelator are said to be strong chelators.

The composition of the present invention may be in any convenient form, e.g., a bar, a homogenous tablet, a tablet having two or more layers, a pouch having one or more compartments, a regular or compact powder, a granule, a paste, a gel, or a regular, compact or concentrated liquid. There are a number of detergent formulation forms such as layers (same or different phases), pouches, as well as forms for machine dosing unit.

Pouches can be configured as single or multicompartments. It can be of any form, shape and material which

is suitable for hold the composition, *e.g.* without allowing the release of the composition from the pouch prior to water contact. The pouch is made from water soluble film which encloses an inner volume. Said inner volume can be divided into compartments of the pouch. Preferred films are polymeric materials preferably polymers which are formed into a film or sheet. Preferred polymers, copolymers or derivatives thereof are selected polyacrylates, and water soluble acrylate copolymers, methyl cellulose, carboxy methyl cellulose, sodium dextrin, ethyl cellulose, hydroxyethyl cellulose, hydroxypropyl methyl cellulose, malto dextrin, poly methacrylates, most preferably polyvinyl alcohol copolymers and, hydroxyprpyl methyl cellulose (HPMC). Preferably the level of polymer in the film for example PVA is at least about 60%. Preferred average molecular weight will typically be about 20,000 to about 150,000. Films can also be of blend compositions comprising hydrolytically degradable and water soluble polymer blends such as polyactide and polyvinyl alcohol (known under the Trade reference M8630 as sold by Chris Craft In. Prod. Of Gary, Ind., US) plus plasticisers like glycerol, ethylene glycerol, Propylene glycol, sorbitol and mixtures thereof. The pouches can comprise a solid laundry cleaning composition or part components and/or a liquid cleaning composition or part components separated by the water soluble film. The compartment for liquid components can be different in composition than compartments containing solids. Ref: (US2009/0011970 A1).

Detergent ingredients may be separated physically from each other by compartments in water dissolvable pouches or in different layers of tablets. Thereby negative storage interaction between components may be avoided. Different dissolution profiles of each of the compartments can also give rise to delayed dissolution of selected components in the wash solution.

A liquid or gel detergent, which is not unit dosed, may be aqueous, typically containing at least 20% by weight and up to 95% water, such as up to about 70% water, up to about 65% water, up to about 55% water, up to about 45% water, up to about 35% water. Other types of liquids, including without limitation, alkanols, amines, diols, ethers and polyols may be included in an aqueous liquid or gel. An aqueous liquid or gel detergent may contain from 0-30% organic solvent. A liquid or gel detergent may be non-aqueous.

Another form of composition is in the form of a soap bar, such as a laundry soap bar, and may be used for hand washing laundry, fabrics and/or textiles. The term "soap bar" as used herein, refers to includes laundry bars, soap bars, combo bars, syndet bars and detergent bars. The types of bar usually differ in the type of surfactant they contain, and the term laundry soap bar includes those containing soaps from fatty acids and/or synthetic soaps. The laundry soap bar has a physical form which is solid and not a liquid, gel or a powder at room temperature. The term "solid" as used herein, refers to a physical form which does not significantly change over time, *i.e.* if a solid object (*e.g.* laundry soap bar) is placed inside a container, the solid object does not change to fill the container it is placed in. The bar is a solid typically in bar form but can be in other solid shapes such as round or oval.

The soap bar may also comprise complexing agents like EDTA and HEDP, perfumes and/or different type of fillers, surfactants *e.g.* anionic synthetic surfactants, builders, polymeric soil release agents, detergent chelators, stabilizing agents, fillers, dyes, colorants, dye transfer inhibitors, alkoxylated polycarbonates, suds suppressers, structurants, binders, leaching agents, bleaching activators, clay soil removal agents, anti-redeposition agents, polymeric dispersing agents, brighteners, fabric softeners, perfumes and/or other compounds known in the art.

The soap bar may be processed in conventional laundry soap bar making equipment such as but not limited to: mixers, plodders, *e.g.* a two stage vacuum plodder, extruders, cutters, logo-stampers, cooling tunnels and wrappers. The invention is not limited to preparing the soap bars by any single method. The premix of the invention may be added to the soap at different stages of the process. For example, the premix comprising a soap, an enzyme, optionally one or more additional enzymes, a protease inhibitor, and a salt of a monovalent cation and an organic anion may be prepared and the mixture may then plodded. The enzyme and optional additional enzymes may be added at the same time as an enzyme inhibitor, e.g. a protease inhibitor, for example in liquid form. Besides the mixing step and the plodding step, the process may further comprise the steps of milling, extruding, cutting, stamping, cooling and/or wrapping.

In one embodiment, the composition comprises a builder and/or a co-builder.

The term "builder" as used herein, refers to specific type of a chelating agent. Accordingly, the composition may comprise about 0-65% by weight, such as about 5% to about 50% of a detergent builder or co-builder, or a mixture thereof. In a dish wash detergent, the level of builder is typically 40-65%, particularly 50-65%. The builder and/or co-builder may particularly be a chelating agent that forms water-soluble complexes with Ca and Mg. Any builder and/or co-builder known in the art for use in ADW detergents may be utilized. Non-limiting examples of builders include zeolites, diphosphates (pyrophosphates), triphosphates such as sodium triphosphate (STP or STPP), carbonates such as sodium carbonate, soluble silicates such as sodium metasilicate, layered silicates (*e.g.*, SKS-6 from Hoechst), ethanolamines such as 2-aminoethan-1-ol (MEA), diethanolamine (DEA, also known as 2,2'-iminodiethan-1-ol), triethanolamine (TEA, also known as 2,2',2"-nitrilotriethan-1-ol), and (carboxymethyl)inulin (CMI), and combinations thereof.

The detergent composition may also comprise 0-50% by weight, such as about 5% to about 30%, of a detergent co-builder. The detergent composition may include a co-builder alone, or in combination with a builder, for example a zeolite builder. Non-limiting examples of co-builders include homopolymers of polyacrylates or copolymers thereof, such as poly(acrylic acid) (PAA) or copoly(acrylic acid/maleic acid) (PAA/PMA). Further non-limiting examples include citrate,

chelators such as aminocarboxylates, aminopolycarboxylates and phosphonates, and alkyl- or alkenylsuccinic acid. Additional specific examples include 2,2',2"-nitrilotriacetic acid (NTA), ethylenediaminetetraacetic acid (EDTA), diethylenetriaminepentaacetic acid (DTPA), iminodisuccinic acid (IDS), ethylenediamine-*N*,*N*'-disuccinic acid (EDDS), methylglycinediacetic acid (MGDA), glutamic acid-*N*,*N*-diacetic acid (GLDA), 1-hydroxyethane-1,1-diphosphonic acid (HEDP), ethylenediaminetetra(methylenephosphonic acid) (EDTMPA), diethylenetriaminepentakis(methylenephosphonic acid) (DTMPA or DTPMPA), *N*-(2-hydroxyethyl)iminodiacetic acid (EDG), aspartic acid-N-monoacetic acid (ASMA), aspartic acid-*N*,*N*-diacetic acid (ASDA), aspartic acid-*N*-monopropionic acid (ASMP), iminodisuccinic acid (IDA), *N*-(2-sulfomethyl)-aspartic acid (SMAS), *N*-(2-sulfoethyl)-aspartic acid (SEAS), *N*-(2-sulfomethyl)-glutamic acid (SMGL), *N*-(2-sulfoethyl)-glutamic acid (SEGL), *N*-methyliminodiacetic acid (MIDA), α-alanine-*N*,*N*-diacetic acid (a-ALDA), serine-*N*,*N*-diacetic acid (SEDA), isoserine-*N*,*N*-diacetic acid (ISDA), phenylalanine-*N*,*N*-diacetic acid (PHDA), anthranilic acid-*N*,*N*-diacetic acid (ANDA), sulfanilic acid-*N*,*N*-diacetic acid (SLDA), taurine-*N*,*N*-diacetic acid (TUDA) and sulfomethyl-*N*,*N*-diacetic acid (SMDA), *N*-(2-hydroxyethyl)ethylenediamine-*N*,*N*',*N*"-triacetic acid (HEDTA), diethanolglycine (DEG), diethylenetriamine penta(methylenephosphonic acid) (DTPMP), aminotris(methylenephosphonic acid) (ATMP), and combinations and salts thereof. Further exemplary builders and/or co-builders are described in, *e.g*., WO 09/102854, US 5977053.

In one embodiment, the composition comprises a polymer. The composition may comprise 0-10% by weight, such as 0.5-5%, 2-5%, 0.5-2% or 0.2-1% of a polymer. Any polymer known in the art for use in detergents may be utilized. The polymer may function as a co-builder as mentioned above, or may provide antiredeposition, fiber protection, soil release, dye transfer inhibition, grease cleaning and/or anti-foaming properties. Some polymers may have more than one of the above-mentioned properties and/or more than one of the below-mentioned motifs. Exemplary polymers include (carboxymethyl)cellulose (CMC), poly(vinyl alcohol) (PVA), poly(vinylpyrrolidone) (PVP), poly(ethyleneglycol) or poly(ethylene oxide) (PEG), ethoxylated poly(ethyleneimine), carboxymethyl inulin (CMI), and polycarboxylates such as PAA, PAA/PMA, poly-aspartic acid, and lauryl methacrylate/acrylic acid copolymers , hydrophobically modified CMC (HM-CMC) and silicones, copolymers of terephthalic acid and oligomeric glycols, copolymers of poly(ethylene terephthalate) and poly(oxyethene terephthalate) (PET-POET), PVP, poly(vinylimidazole) (PVI), poly(vinylpyridine-*N*-oxide) (PVPO or PVPNO) and polyvinylpyrrolidone-vinylimidazole (PVPVI). Further exemplary polymers include sulfonated polycarboxylates, polyethylene oxide and polypropylene oxide (PEO-PPO) and diquaternium ethoxy sulfate. Other exemplary polymers are disclosed in, *e.g*., WO 2006/130575. Salts of the above-mentioned polymers are also contemplated.

The compositions according to the present invention may comprise a variant of the present invention as the major enzymatic component, *e.g*., a mono-component composition. In another embodiment, the composition comprises at least one additional enzyme, such as a protease, lipase, cellulose, pectate lyase, and/or mannanase. Thus, the compositions may comprise multiple enzymatic activities, such as one or more (*e.g*., several) enzymes selected from the group consisting of hydrolase, isomerase, ligase, lyase, oxidoreductase, or transferase, *e.g*., an alpha-galactosidase, alpha-glucosidase, aminopeptidase, amylase, beta-galactosidase, beta-glucosidase, beta-xylosidase, carbohydrase, carboxypeptidase, catalase, cellobiohydrolase, cellulase, chitinase, cutinase, cyclodextrin glycosyltransferase, deoxyribonuclease, endoglucanase, esterase, glucoamylase, invertase, laccase, lipase, mannosidase, mutanase, oxidase, pectinolytic enzyme, peroxidase, phytase, polyphenoloxidase, proteolytic enzyme, ribonuclease, transglutaminase, or xylanase.

In a particular embodiment, the composition further comprises one or more additional enzymes selected from the following group:

(i) an alpha-amylase comprising a modifications in the following position: 202 as compared with the alpha-amylase in SEQ ID NO:5;
(ii) an alpha-amylase comprising one or more modifications in the following positions: 9, 118, 149, 182, 186, 195, 202, 257, 295, 299, 320, 323, 339, 345, and 458 as compared with the alpha-amylase in SEQ ID NO:6;
(iii) an alpha-amylase comprising one or more modification in the following positions: 405, 421, 422, and 428 as compared with the alpha-amylase in SEQ ID NO: 9;
(iv) an alpha-amylase comprising any one the amino acid sequences set forth in SEQ ID NO: 7, 10, 11, and 12; and/or
(v) a protease comprising one or more modifications in the following positions: 32, 33, 48-54, 58-62, 94-107, 116, 123-133, 150, 152-156, 158-161, 164, 169, 175-186, 197, 198, 203-216 as compared with the protease in SEQ ID NO:8.

In general the properties of the selected enzyme(s) should be compatible with the selected detergent, (*i.e*., pH-optimum, compatibility with other enzymatic and non-enzymatic ingredients, etc.), and the enzyme(s) should be present in effective amounts.

In one embodiment, the composition comprises or consists of an alpha-amylase variant comprising or consisting of the following modifications; G182*+D183*+M202L, wherein numbering is according to SEQ ID NO: 3 and an alpha-amylase comprising a modification in the following position: 202 as compared with the alpha-amylase in SEQ ID NO:5.

In one embodiment, the composition comprises or consists of an alpha-amylase variant comprising or consisting

of the following modifications; G182*+D183*+N195F+M202L, wherein numbering is according to SEQ ID NO: 3 and an alpha-amylase comprising a modifications in the following position: 202 as compared with the alpha-amylase in SEQ ID NO:5.

In one embodiment, the composition comprises or consists of an alpha-amylase variant comprising or consisting of the following modifications; G182*+D183*+M202L, wherein numbering is according to SEQ ID NO: 3 and an alpha-amylase comprising one or more modifications in the following positions: 9, 118, 149, 182, 186, 195, 202, 257, 295, 299, 320, 323, 339, 345, and 458 as compared with the alpha-amylase in SEQ ID NO:6.

In one embodiment, the composition comprises or consists of an alpha-amylase variant comprising or consisting of the following modifications; G182*+D183*+N195F+M202L, wherein numbering is according to SEQ ID NO: 3 and an alpha-amylase comprising one or more modifications in the following positions: 9, 118, 149, 182, 186, 195, 202, 257, 295, 299, 320, 323, 339, 345, and 458 as compared with the alpha-amylase in SEQ ID NO:6.

In one embodiment, the composition comprises or consists of an alpha-amylase variant comprising or consisting of the following modifications; G182*+D183*+M202L, wherein numbering is according to SEQ ID NO: 3 and an alpha-amylase comprising one or more modification in the following positions: 405, 421, 422, and 428 as compared with the alpha-amylase in SEQ ID NO: 9.

In one embodiment, the composition comprises or consists of an alpha-amylase variant comprising or consisting of the following modifications; G182*+D183*+N195F+M202L, wherein numbering is according to SEQ ID NO: 3 and an alpha-amylase comprising one or more modification in the following positions: 405, 421, 422, and 428 as compared with the alpha-amylase in SEQ ID NO: 9.

In one embodiment, the composition comprises or consists of an alpha-amylase variant comprising or consisting of the following modifications; G182*+D183*+M202L, wherein numbering is according to SEQ ID NO: 3 and an alpha-amylase comprising any one the amino acid sequences set forth in SEQ ID NO: 7, 10, 11, and 12.

In one embodiment, the composition comprises or consists of an alpha-amylase variant comprising or consisting of the following modifications; G182*+D183*+N195F+M202L, wherein numbering is according to SEQ ID NO: 3 and an alpha-amylase comprising any one the amino acid sequences set forth in SEQ ID NO: 7, 10, 11, and 12.

In one embodiment, the composition comprises or consists of an alpha-amylase variant comprising or consisting of the following modifications; G182*+D183*+M202L, wherein numbering is according to SEQ ID NO: 3 and a protease comprising one or more modifications in the following positions: 32, 33, 48-54, 58-62, 94-107, 116, 123-133, 150, 152-156, 158-161, 164, 169, 175-186, 197, 198, 203-216 as compared with the protease in SEQ ID NO:8.

In one embodiment, the composition comprises or consists of an alpha-amylase variant comprising or consisting of the following modifications; G182*+D183*+N195F+M202L, wherein numbering is according to SEQ ID NO: 3 and a protease comprising one or more modifications in the following positions: 32, 33, 48-54, 58-62, 94-107, 116, 123-133, 150, 152-156, 158-161, 164, 169, 175-186, 197, 198, 203-216 as compared with the protease in SEQ ID NO:8.

Suitable cellulases include those of bacterial or fungal origin. Chemically modified or protein engineered mutants are included. Suitable cellulases include cellulases from the genera *Bacillus, Pseudomonas, Humicola, Fusarium, Thielavia, Acremonium, e.g.,* the fungal cellulases produced from *Humicola insolens, Myceliophthora thermophila* and *Fusarium oxysporum* disclosed in US 4,435,307, US 5,648,263, US 5,691,178, US 5,776,757 and WO 89/09259.

Especially suitable cellulases are the alkaline or neutral cellulases having color care benefits. Examples of such cellulases are cellulases described in EP 0 495 257, EP 0 531 372, WO 96/11262, WO 96/29397, WO 98/08940. Other examples are cellulase variants such as those described in WO 94/07998, EP 0 531 315, US 5,457,046, US 5,686,593, US 5,763,254, WO 95/24471, WO 98/12307 and PCT/DK98/00299.

Example of cellulases exhibiting endo-beta-1,4-glucanase activity (EC 3.2.1.4) are those having described in WO02/099091.

Other examples of cellulases include the family 45 cellulases described in WO96/29397, and especially variants thereof having substitution, insertion and/or deletion at one or more of the positions corresponding to the following positions in SEQ ID NO: 8 of WO 02/099091: 2, 4, 7, 8, 10, 13, 15, 19, 20, 21, 25, 26, 29, 32, 33, 34, 35, 37, 40, 42, 42a, 43, 44, 48, 53, 54, 55, 58, 59, 63, 64, 65, 66, 67, 70, 72, 76, 79, 80, 82, 84, 86, 88, 90, 91, 93, 95, 95d, 95h, 95j, 97, 100, 101, 102, 103, 113, 114, 117, 119, 121, 133, 136, 137, 138, 139, 140a, 141, 143a, 145, 146, 147, 150e, 150j, 151, 152, 153, 154, 155, 156, 157, 158, 159, 160c, 160e, 160k, 161, 162, 164, 165, 168, 170, 171, 172, 173, 175, 176, 178, 181, 183, 184, 185, 186, 188, 191, 192, 195, 196, 200, and/or 20, preferably selected among P19A, G20K, Q44K, N48E, Q119H or Q146 R.

Commercially available cellulases include Celluzyme, and Carezyme (Novozymes A/S), Clazinase, and Puradax HA (Genencor International Inc.), and KAC-500(B) (Kao Corporation).

Suitable lipases and cutinases include those of bacterial or fungal origin. Chemically modified or protein engineered mutant enzymes are included. Examples include lipase from Thermomyces, e.g. from T. lanuginosus (previously named Humicola lanuginosa) as described in EP258068 and EP305216, cutinase from Humicola, e.g. H. insolens (WO96/13580), lipase from strains of Pseudomonas (some of these now renamed to Burkholderia), *e.g. P. alcaligenes* or *P. pseudoalcaligenes* (EP218272), *P. cepacia* (EP331376), *P.* sp. strain SD705 (WO95/06720 & WO96/27002), *P.*

*wisconsinensis* (WO96/12012), GDSL-type Streptomyces lipases (WO10/065455), cutinase from *Magnaporthe grisea* (WO10/107560), cutinase from *Pseudomonas mendocina* (US5,389,536), lipase from *Thermobifida fusca* (WO11/084412), *Geobacillus stearothermophilus* lipase (WO11/084417), lipase from *Bacillus subtilis* (WO11/084599), and lipase from *Streptomyces griseus* (WO11/150157) and *S. pristinaespiralis* (WO12/137147).

Further examples are lipases sometimes referred to as acyltransferases or perhydrolases, *e.g.* acyltransferases with homology to *Candida antarctica* lipase A (WO10/111143), acyltransferase from *Mycobacterium smegmatis* (WO05/56782), perhydrolases from the CE 7 family (WO09/67279), and variants of the *M. smegmatis* perhydrolase in particular the S54V variant used in the commercial product Gentle Power Bleach from Huntsman Textile Effects Pte Ltd (WO10/100028).

Other examples are lipase variants such as those described in EP407225, WO92/05249, WO94/01541, WO94/25578, WO95/14783, WO95/30744, WO95/35381, WO95/22615, WO96/00292, WO97/04079, WO97/07202, WO00/34450, WO00/60063, WO01/92502, WO07/87508 and WO09/109500.

Preferred commercial lipase products include include LipolaseTM, Lipex™; LipolexTM and LipocleanTM (Novozymes A/S), Lumafast (originally from Genencor) and Lipomax (originally from Gist-Brocades).

Suitable peroxidases/oxidases include those of plant, bacterial orfungal origin. Chemically modified or protein engineered mutants are included. Examples of useful peroxidases include peroxidases from Coprinus, *e.g.*, from C. cinereus, and variants thereof as those described in WO 93/24618, WO 95/10602, and WO 98/15257.

Commercially available peroxidases include Guardzyme (Novozymes A/S).

The detergent enzyme(s) may be included in a detergent composition by adding separate additives containing one or more enzymes, or by adding a combined additive comprising all of these enzymes. A detergent additive of the invention, *i.e.,* a separate additive or a combined additive, can be formulated, for example, as a granulate, liquid, slurry, etc. Preferred detergent additive formulations are granulates, in particular non-dusting granulates, liquids, in particular stabilized liquids, or slurries.

Non-dusting granulates may be produced, *e.g.*, as disclosed in US 4,106,991 and 4,661,452 and may optionally be coated by methods known in the art. Examples of waxy coating materials are poly(ethylene oxide) products (polyethyleneglycol, PEG) with mean molar weights of 1000 to 20000; ethoxylated nonylphenols having from 16 to 50 ethylene oxide units; ethoxylated fatty alcohols in which the alcohol contains from 12 to 20 carbon atoms and in which there are 15 to 80 ethylene oxide units; fatty alcohols; fatty acids; and mono- and di- and triglycerides of fatty acids. Examples of film-forming coating materials suitable for application by fluid bed techniques are given in GB 1483591. Liquid enzyme preparations may, for instance, be stabilized by adding a polyol such as propylene glycol, a sugar or sugar alcohol, lactic acid or boric acid according to established methods. Protected enzymes may be prepared according to the method disclosed in EP 238,216.

The compositions may be prepared in accordance with methods known in the art and may be in the form of a liquid or a dry composition. The compositions may be stabilized in accordance with methods known in the art.

Any detergent components known in the art for use in ADW detergents may also be utilized. Other optional detergent components include anti-corrosion agents, anti-shrink agents, anti-soil redeposition agents, anti-wrinkling agents, bactericides, binders, corrosion inhibitors, disintegrants/disintegration agents, dyes, enzyme stabilizers (including boric acid, borates, CMC, and/or polyols such as propylene glycol), fabric conditioners including clays, fillers/processing aids, fluorescent whitening agents/optical brighteners, foam boosters, foam (suds) regulators, perfumes, soil-suspending agents, softeners, suds suppressors, tarnish inhibitors, and wicking agents, either alone or in combination. Any ingredient known in the art for use ADW detergents may be utilized. The choice of such ingredients is well within the skill of the artisan.

The compositions of the present invention may also comprise dispersants. In particular powdered detergents may comprise dispersants. Suitable water-soluble organic materials include the homo- or co-polymeric acids or their salts, in which the polycarboxylic acid comprises at least two carboxyl radicals separated from each other by not more than two carbon atoms. Suitable dispersants are for example described in Powdered Detergents, Surfactant science series volume 71, Marcel Dekker, Inc.

The compositions of the present invention may also comprise one or more dye transfer inhibiting agents. Suitable polymeric dye transfer inhibiting agents include, but are not limited to, polyvinylpyrrolidone polymers, polyamine N-oxide polymers, copolymers of *N*-vinylpyrrolidone and *N*-vinylimidazole, polyvinyloxazolidones and polyvinylimidazoles or mixtures thereof. When present in a subject composition, the dye transfer inhibiting agents may be present at levels from about 0.0001 % to about 10%, from about 0.01% to about 5% or even from about 0.1% to about 3% by weight of the composition.

The compositions of the present invention will preferably also contain additional components that may tint articles being cleaned, such as fluorescent whitening agent or optical brighteners. Where present the brightener is preferably at a level of about 0.01% to about 0.5%. Any fluorescent whitening agent suitable for use in a laundry detergent composition may be used in the composition of the present invention. The most commonly used fluorescent whitening agents are those belonging to the classes of diaminostilbene-sulfonic acid derivatives, diarylpyrazoline derivatives and bisphenyl-distyryl derivatives. Examples of the diaminostilbene-sulfonic acid derivative type of fluorescent whitening

agents include the sodium salts of: 4,4'-bis-(2-diethanolamino-4-anilino-s-triazin-6-ylamino) stilbene-2,2'-disulfonate, 4,4'-bis-(2,4-dianilino-s-triazin-6-ylamino) stilbene-2.2'-disulfonate, 4,4'-bis-(2-anilino-4-(*N*-methyl-*N*-2-hydroxy-ethylamino)-s-triazin-6-ylamino) stilbene-2,2'-disulfonate, 4,4'-bis-(4-phenyl-1,2,3-triazol-2-yl)stilbene-2,2'-disulfonate and sodium 5-(2*H*-naphtho[1,2-*d*][1,2,3]triazol-2-yl)-2-[(*E*)-2-phenylvinyl]benzenesulfonate. Preferred fluorescent whitening agents are Tinopal DMS and Tinopal CBS available from Ciba-Geigy AG, Basel, Switzerland. Tinopal DMS is the disodium salt of 4,4'-bis-(2-morpholino-4-anilino-s-triazin-6-ylamino) stilbene-2,2'-disulfonate. Tinopal CBS is the disodium salt of 2,2'-bis-(phenyl-styryl)-disulfonate. Also preferred are fluorescent whitening agents is the commercially available Parawhite KX, supplied by Paramount Minerals and Chemicals, Mumbai, India. Other fluorescers suitable for use in the invention include the 1-3-diaryl pyrazolines and the 7-alkylaminocoumarins.

Suitable fluorescent brightener levels include lower levels of from about 0.01, from 0.05, from about 0.1 or even from about 0.2 wt % to upper levels of 0.5 or even 0.75 wt%.

**Uses**

The present invention relates to the use of an alpha-amylase variant of a parent alpha-amylase in a cleaning process, wherein a bleaching system is added in a concentration of at least 5 weight%, wherein said parent has an amino acid sequence which is at least 80% identical to the amino acid sequence set forth in SEQ ID NO: 3, wherein said variant comprises a substitution in one or more positions providing oxidation stability of said variant, wherein said variant comprises a substitution in position 202, wherein said position corresponds to the amino acid position of the amino acid sequence as set forth in SEQ ID NO: 3, wherein said substitution is a M202L substitution, wherein said variant has a sequence identity of at least 80%, but less than 100% to the amino acid sequence set forth in SEQ ID NO: 3, wherein said variant has an improvement factor of >1.0 as a measure for wash performance, when compared to said parent alpha-amylase, wherein said variant has alpha-amylase activity. The cleaning process may befor laundry or hard surface cleaning including automated dish wash and industrial cleaning. The soils and stains that are important for cleaning are composed of many different substances, and a range of different enzymes, all with different substrate specificities, have been developed for use in detergents both in relation to laundry and hard surface cleaning, such as dishwashing. These enzymes are considered to provide an enzyme detergency benefit, since they specifically improve stain removal in the cleaning process that they are used in, compared to the same process without enzymes. Stain removing enzymes that are known in the art include enzymes such as proteases, amylases, lipases, cutinases, cellulases, endoglucanases, xyloglucanases, pectinases, pectin lyases, xanthanases, peroxidaes, haloperoxygenases, catalases and mannanases.

In one embodiment, the invention relates the use of variants of the present invention in detergent compositions, for use in cleaning hard-surfaces, such as dish wash, or in laundering or for stain removal. In another embodiment, the invention relates to the use of a variant according to the invention in a cleaning process such as laundry or hard surface cleaning including, but not limited to, dish wash and industrial cleaning. Thus, in one embodiment, the invention relates to the use of a variant comprising a substitution in one or more positions providing oxidation stability of the variant.

In a particular embodiment, the variant comprising a substitution in the position corresponding to position M202 of the amino acid sequence as set forth in SEQ ID NO: 3, and a substitution and/or deletion of two, three, or four positions corresponding to positions R181, G182, D183, and G184 of the amino acid sequence as set forth in SEQ ID NO: 3.

In one embodiment of the invention relates the use of a composition according to the invention comprising a variant of the present invention together with one or more surfactants and optionally one or more detergent components, selected from the list comprising of hydrotropes, builders and co-builders, bleaching systems, polymers, fabric hueing agents and adjunct materials, or any mixture thereof in detergent compositions and in detergent applications.

A further embodiment is the use of the composition according to the invention comprising a variant of the present invention together with one or more surfactants, and one or more additional enzymes selected from the group comprising of proteases, lipases, cutinases, cellulases, endoglucanases, xyloglucanases, pectinases, pectin lyases, xanthanases, peroxidaes, haloperoxygenases, catalases and mannanases, or any mixture thereof in detergent compositions and in detergent applications.

In another aspect, the invention relates to a laundering process which may be for household laundering as well as industrial laundering. Furthermore, the invention relates to a process for the laundering of textiles (*e.g.* fabrics, garments, cloths etc.) where the process comprises treating the textile with a washing solution containing a detergent composition and an alpha-amylase of the present invention. The laundering can for example be carried out using a household or an industrial washing machine or be carried out by hand using a detergent composition containing a glucoamylase of the invention.

In another aspect, the invention relates to a dish wash process which may be for household dish wash as well as industrial dish wash. The term "dish wash" as used herein, refers to both manual dish wash and automated dish wash. Furthermore, the invention relates to a process for the washing of hard surfaces (*e.g.* cutlery such as knives, forks, spoons; crockery such as plates, glasses, bowls; and pans) where the process comprises treating the hard surface

with a washing solution containing a detergent composition and an alpha-amylase variant of the present invention. The hard surface washing can for example be carried out using a household or an industrial dishwasher or be carried out by hand using a detergent composition containing an alpha-amylase of the invention, optionally together with one or more further enzymes selected from the group comprising of proteases, amylases, lipases, cutinases, cellulases, endoglucanases, xyloglucanases, pectinases, pectin lyases, xanthanases, peroxidaes, haloperoxygenases, catalases, mannanases, or any mixture thereof.

In a further aspect, the invention relates to a method for removing a stain from a surface comprising contacting the surface with a composition comprising an alpha-amylase used in accordance with the present invention together with one or more surfactants and optionally one or more detergent components selected from the list comprising of hydrotropes, builders and co-builders, bleaching systems, polymers, fabric hueing agents and adjunct materials, or any mixture thereof in detergent compositions and in detergent applications. A further aspect is a method for removing a stain from a surface comprising contacting the surface with a composition comprising an alpha-amylase variant of the present invention together with one or more surfactants, one or more additional enzymes selected from the group comprising of proteases, lipases, cutinases, cellulases, endoglucanases, xyloglucanases, pectinases, pectin lyases, xanthanases, peroxidaes, haloperoxygenases, catalases and mannanases, or any mixture thereof in detergent compositions and in detergent applications.

The present invention is further described by the following.

## EXAMPLES

### Example 1 - Generation of variants

Using the parent alpha-amylase having the amino acid sequence as set forth in SEQ ID NO: 4, the variants of the present invention were constructed. The variants were prepared by standard procedures, which in brief is; introducing an amino acid substitution in the position corresponding to position M200 of the amino acid sequence as set forth in SEQ ID NO: 4 (if numbering is according to SEQ ID NO: 3, the amino acid position is M202), by site-directed mutagenesis into the gene, transforming *Bacillus subtilis* host cells with the mutated gene, fermenting the transformed cells (*e.g.* as described in Example 1 of WO 2004/111220), and purifying the variants from the fermentation broth. The reference amylase, *i.e.* the parent alpha-amylase, having the amino acid sequence as set forth in SEQ ID NO: 3 or 4, respectively, were produced recombinantly in *Bacillus subtilis* in a similar manner.

### Example 2 - Determination of amylolytic activity (alpha-amylase activity)

The variants generated as described in Example 1 were tested for alpha-amylase activity by a pNP-G7 assay.

The alpha-amylase activity was determined by employing the pNP-G7 substrate (PNP-G7 the abbreviation for 4,6-ethylidene(G7)-p-nitrophenyl(G1)-$\alpha$,D-maltoheptaoside, a blocked oligosaccharide which is cleaved by an endo-amylase, such as an alpha-amylase).

An antibody was diluted in Phosphate buffered saline (PBS) (0.010 M Phosphate buffer pH7.4, 0.0027M KCl, 0.14M NaCl) buffer to concentration of 10$\mu$g/ml. A maxisorp microtiter plate was coated with antibody by adding 100$\mu$l diluted antibody (10$\mu$g/ml) to each well and incubated for 1h at room temperature (RT) and mixing at 800 rpm. After incubation the microtiter plate was washed (using Bio-Tek ELx405 ELISA washer) with 3x 200$\mu$l Phosphate buffered saline with 0.05% Tween (PBST) (0.010 M Phosphate buffer pH7.4, 0.0027M KCl, 0.14M NaCl, 0.05% Tween 20) buffer.

Microtiter plates with the alpha-amylase variants culture broths were spun down and supernatants transferred to new microtiter plates and diluted 4x in PBST buffer. 100$\mu$l diluted supernatant was transferred to antibody coated maxisorp microtiter plate and incubated for 1h at RT and mixing at 800rpm. After incubation microtiter plates were washed in PBST buffer (3x 200$\mu$l, ELISA washer).

Upon the cleavage of the pNP-G7 substrate, the alpha-Glucosidase included in the kit used is digested and the hydrolysed substrate liberates a free pNP molecule which has a yellow color and thus can be measured by visible spectophometry at Abs=405nm (400-420 nm.). Kits containing pNP-G7 substrate and alpha-Glucosidase are manufactured by Roche/Hitachi (cat. No.11876473). 100$\mu$l pNP-G7 substrate was added to all wells and mixed for 1 minute before measuring absorbance at 405nm. The slope (absorbance per minute) is determined and only the linear range of curve is used.

Results were compared to a reference sample and samples with an activity on par or higher were considered to have a maintained alpha-amylase activity. Such variants were further evaluated for oxidation stability, as described in Example 3.

The specific alpha-amylase activity may also be determined by other activity assays, such as amylazyme activity assay, Phadebas activity assay, or reducing sugar activity assay as described below.

Amylazyme activity assay (from Megazyme, Ireland): An Amylazyme tablet includes interlinked amylose polymers that are in the form of globular microspheres that are insoluble in water. A blue dye is covalently bound to these microspheres. The interlinked amylose polymers in the microsphere are degraded at a speed that is proportional to the alpha-amylase activity. When the alpha-amylase degrades the amylose polymers, the released blue dye is water soluble and concentration of dye can be determined by measuring absorbance at 650nm. The concentration of blue is proportional to the alpha-amylase activity in the sample.

The amylase sample to be analysed is diluted in activity buffer with the desired pH. One substrate tablet is suspended in 5mL activity buffer and mixed on magnetic stirrer. During mixing of substrate transfer 150μl to microtiter plate (MTP). Add 30μl diluted amylase sample to 150μl substrate and mix. Incubate for 15 minutes at 37°C. The reaction is stopped by adding 30μl 1M NaOH and mix. Centrifuge MTP for 5 minutes at 4000xg. Transfer 100μl to new MTP and measure absorbance at 620nm.

The amylase sample should be diluted so that the absorbance at 650nm is between 0 and 2.2, and is within the linear range of the activity assay.

Phadebas activity assay (from for example Magle Life Sciences, Lund, Sweden): A Phadebas tablet includes interlinked starch polymers that are in the form of globular microspheres that are insoluble in water. A blue dye is covalently bound to these microspheres. The interlinked starch polymers in the microsphere are degraded at a speed that is proportional to the alpha-amylase activity. When the alpha-amylase degrades the starch polymers, the released blue dye is water soluble and concentration of dye can be determined by measuring absorbance at 650nm. The concentration of blue is proportional to the alpha-amylase activity in the sample.

The amylase sample to be analysed is diluted in activity buffer with the desired pH. One substrate tablet is suspended in 5mL activity buffer and mixed on magnetic stirrer. During mixing of substrate transfer 150μl to microtiter plate (MTP). Add 30μl diluted amylase sample to 150μl substrate and mix. Incubate for 15 minutes at 37°C. The reaction is stopped by adding 30μl 1M NaOH and mix. Centrifuge MTP for 5 minutes at 4000xg. Transfer 100μl to new MTP and measure absorbance at 620nm.

The amylase sample should be diluted so that the absorbance at 650nm is between 0 and 2.2, and is within the linear range of the activity assay.

Reducing sugar activity assay: The number of reducing ends formed by the alpha-amylase hydrolysing the alpha-1,4-glycosidic linkages in starch is determined by reaction with p-Hydroxybenzoic acid hydrazide (PHBAH). After reaction with PHBAH the number of reducing ends can be measured by absorbance at 405nm and the concentration of reducing ends is proportional to the alpha-amylase activity in the sample.

The corns starch substrate (3mg/ml) is solubilised by cooking for 5 minutes in milliQ water and cooled down before assay. For the stop solution prepare a Ka-Na-tartrate/NaOH solution (K-Na-tartrate (Merck 8087) 50g/l, NaOH 20g/l) and prepare freshly the stop solution by adding p-Hydroxybenzoic acid hydrazide (PHBAH, Sigma H9882) to Ka-Na-tartrate/NaOH solution to 15mg/ml.

In PCR-MTP 50μl activity buffer is mixed with 50μl substrate. Add 50μl diluted enzyme and mix. Incubate at the desired temperature in PCR machine for 5 minutes. Reaction is stopped by adding 75μl stop solution (Ka-Na-tartrate/NaOH/PHBAH). Incubate in PCR machine for 10 minutes at 95°C. Transfer 150μl to new MTP and measure absorbance at 405nm.

The amylase sample should be diluted so that the absorbance at 405nm is between 0 and 2.2, and is within the linear range of the activity assay.

### Example 3 - Oxidation stability verified by AMSA

In order to assess the oxidation stability of the variants generated as described in Example 1, the wash performance of the variants in a detergent composition comprising an oxidizing agent, such as a bleaching system, was determined by using Automatic Mechanical Stress Assay (AMSA). A variant which is oxidation stable will have a maintained wash performance, and even may have an improved wash performance.

The variants of the present invention were, thus, tested for the wash performance. By use of the AMSA test the wash performance of a large quantity of small volume enzyme-detergent solutions can be examined. The AMSA plate has a number of slots for test solutions and a lid firmly squeezing the textile swatch to be washed against all the slot openings. During the washing time, the plate, test solutions, textile and lid are vigorously shaken to bring the test solution in contact with the textile and apply mechanical stress in a regular, periodic oscillating manner. For further description see WO 02/42740, especially the paragraph "Special method embodiments" at page 23-24.

General wash performance description

The detergent solution used for the AMSA test comprising water (15°dH), 8.67 g/L Model Z detergent composition and the enzyme of the invention at concentration of 0, 0.1 or 0.2 mg enzyme protein/L, was prepared. Fabrics stained with starch (CS-28 from Center For Test materials BV, P.O. Box 120, 3133 KT, Vlaardingen, The Netherlands) was

added and washed for 20 minutes at 55°C. After thorough rinse under running tap water and drying in the dark, the light intensity values of the stained fabrics were subsequently measured as a measure for wash performance. The test with 0 mg enzyme protein/L is used as a blank and corresponds to the contribution from the detergent compostion. The mechanical action was applied during the wash step, e.g. in the form of shaking, rotating or stirring the wash solution with the fabrics. The AMSA wash performance experiments were conducted under the experimental conditions specified below:

Table 1: Experimental condition

| | |
|---|---|
| Detergent | Model detergent Z (see Table 2) |
| Detergent dosage | 8.67 g/L |
| Test solution volume | 160 micro L |
| pH | pH 10.4-10.5 |
| Wash time | 20 minutes |
| Temperature | 55°C |
| Water hardness | 15°dH |
| Enzyme concentration in test | 0.1 or 0.2 mg/L |
| Test material | CS-28 (Rice starch cotton) |

Table 2: Model detergent Z with bleach

| Compound | Content of compound (% w/w) | % active component (% w/w) |
|---|---|---|
| LAS, sodium salt | 7.03 | 6.0 |
| Soap | 1.08 | 1.0 |
| AEO* | 1.51 | 1.5 |
| Sodium carbonate | 20.10 | 20.0 |
| Sodium (di)silicate | 9.99 | 8.0 |
| Zeolite A | 5.00 | 4.0 |
| HEDP-Na4 | 0.24 | 0.2 |
| Sodium citrate | 2.01 | 2.0 |
| Polycarboxylate (PCA) | 1.09 | 1.0 |
| Sodium paercarbonate | 9.33 | 8.0 |
| TAED | 1.09 | 1.0 |
| $Na_2SO_4$ | 41.54 | 41.5 |
| *AEO is added separately before wash. | | |

Notice the balance up to 100 % and extra contributions to sodium carbonate and sodium sulphate. The balance is water ≤ ca. 2.6 % (from LAS ca. 0.1 %; from Sodium (di)silicate ca. 1.6 %; from Zeolite A ca. 0.8 %; from Polycarboxylate ca. 0.1 %); sodium sulfate ≤ ca. 0.9 % (from LAS, sodium salt probably ca. 0.8 %; from Zeolite A ca. 0.1 %); sodium carbonate probably ca. 1.1 % from Sodium percarbonate; and CMC (carboxymethylcellulose, sodium salt), ca. 0.2 % from TAED. Water hardness was adjusted to 15°dH by addition of $CaCl_2$, $MgCl_2$, and $NaHCO_3$ ($Ca^{2+}$:$Mg^{2+}$:$HCO^{3-}$ = 4:1:7.5) to the test system. After washing the textiles were flushed in tap water and dried.

Relative Performance to Parent alpha-amylase in AMSA 20 min 55°C model detergent Z with bleach

| Mutations | 0.1 mg enzyme/L | 0.2 mg enzyme/L |
|---|---|---|
| Blank | -0.2 | 0.0 |

(continued)

| Mutations | 0.1 mg enzyme/L | 0.2 mg enzyme/L |
|---|---|---|
| G182*+D183* | 1.0 | 1.0 |
| G182*+D183*+N195F | 0.9 | 1.1 |
| G182*+D183*+N195F+M202L | 1.8 | 1.6 |

Specifically, the variants of the present invention is considered to be particularly efficient in the ADW use. Thus, in order to further evaluate this, an AMSA for ADW performance may be performed. Such an AMSA for ADW may be performed under the following conditions;

A test solution comprising water (15°dH), 8.67 g/L detergent, *e.g.* Liquid model detergent containing phosphate, as described below, and the variants of the invention at concentration of 0 or 0.5 mg enzyme protein/L, may be prepared. Melamine plates stained with mixed starch (DM-177 from Center For Test materials BV, P.O. Box 120, 3133 KT, Vlaardingen, The Netherlands) can be added and washed for 20 minutes at 55°C. After short rinse under running tap water and drying in the dark, the light intensity values of the stained plates are subsequently measured as a measure for wash performance. The test with 0 mg enzyme protein/L may be used as a blank and corresponds to the contribution from the detergent. Preferably mechanical action is applied during the wash step, *e.g.* in the form of shaking, rotating or stirring the wash solution with the plates. The AMSA automatic dish wash performance experiments may be conducted under the experimental conditions specified below:

Table 3: Experimental condition

| | |
|---|---|
| Detergent | Liquid model detergent containing phosphate (see Table 4) |
| Detergent dosage | 5 mL/L |
| Test solution volume | 160 micro L |
| pH | ~8 |
| Wash time | 20 minutes |
| Temperature | 50°C |
| Water hardness | 21°dH |
| Enzyme concentration in test | 0.01- 0.24 mg/L |
| Test material | Melamine plates (Mixed Starch) |

Table 4: Liquid model automatic dish wash detergent containing phosphate

| **Compound** | **Content of compound (% w/w)** |
|---|---|
| STPP | 50.0 |
| Sodium carbonate | 20.0 |
| Sodium percarbonate | 10.0 |
| Sodium disilicate | 5.0 |
| TAED | 2.0 |
| Sokalan CP5 (39,5%) | 5.0 |
| Surfac 23-6.5 (100%) | 2.0 |
| Sodium Sulfate | 6.0 |

Water hardness was adjusted to 21°dH by addition of $CaCl_2$, $MgCl_2$, and $NaHCO_3$ ($Ca^{2+}$:$Mg^{2+}$:$HCO_3^-$ = 2:1:10) to the test system. After washing the plates were flushed in tap water and dried.

Table 5: Experimental condition

| | |
|---|---|
| Detergent | Powder model detergent A (see Table 6) |
| Detergent dosage | 3.94 g/L |
| Test solution volume | 160 micro L |
| pH | 9.9 |
| Wash time | 20 minutes |
| Temperature | 50°C |
| Water hardness | 21°dH |
| Enzyme concentration in test | 0.01-0.24 mg/L |
| Test material | Melamine plates (Mixed Starch) |

Table 6: Powder automatic dish wash model detergent

| **Compound** | **Content of ingredient (% w/w)** | % active component (% w/w) |
|---|---|---|
| MGDA | 28,9 | 20 |
| Sodium citrate | 17,1 | 20 |
| Sodium carbonate | 17,1 | 20 |
| Sodium percarbonate | 9,7 | 10 |
| Sodium Silicate | 5,3 | 5 |
| Sodium sulfate | 10,2 | 12 |
| Acusol 588G | 4,6 | 5 |
| TAED | 2,8 | 3 |
| Surfac 23-6.5 (liq) | 4,3 | 5 |
| * Surfac 23-6.5 (liq) is added separately before wash | | |

Water hardness was adjusted to 21°dH by addition of $CaCl_2$, $MgCl_2$, and $NaHCO_3$ ($Ca^{2+}$:$Mg^{2+}$:$HCO_3^-$ = 2:1:10) to the test system. After washing the plates were flushed in tap water and dried.

Evaluation of wash performance

The wash performance is measured as the brightness expressed as the intensity of the light reflected from the sample when illuminated with white light. When the sample is stained the intensity of the reflected light is lower, than that of a clean sample. Therefore the intensity of the reflected light can be used to measure wash performance.

Color measurements are made with a professional flatbed scanner (EPSON EXPRESSION 10000XL) used to capture an image of the washed textile and with a controlled digital imaging system (ColorVectorAnalyzer) for capture an image of the washed melamine plates.

To extract a value for the light intensity from the scanned images, from the image are converted into values for red, green and blue (RGB). The intensity value (Int) is calculated by adding the RGB values together as vectors and then taking the length of the resulting vector:

$$Int = \sqrt{r^2 + g^2 + b^2}$$

Table of sequences referred to in the present application

| SEQ ID NO: | Sequence |
|---|---|
| 1 | CATCACGATGGGACGAACGGAACGATTATGCAGTATTTTGAATGGAACGTT<br>CCGAATGATGGACAACATTGGAACCGCTTACACAACAACGCTCAAAATTTAA<br>AAAATGCCGGAATTACAGCAATCTGGATTCCACCTGCGTGGAAAGGAACGA<br>GCCAAAATGATGTAGGCTACGGTGCGTATGACCTTTATGACCTTGGTGAAT<br>TTAACCAAAAAGGAACGGTCCGTACGAAATATGGAACAAAAGCAGAATTAG<br>AACGAGCGATTCGTTCGTTAAAGGCGAACGGGATTCAAGTGTATGGCGATG<br>TTGTTATGAACCATAAAGGCGGAGCTGATTTCACCGAGCGTGTTCAAGCGG<br>TTGAAGTGAACCCGCAAAACCGAAACCAAGAAGTGTCTGGCACTTATCAAA<br>TCGAAGCATGGACAGGGTTCAATTTTCCTGGACGTGGCAATCAACATTCTT<br>CGTTTAAATGGCGCTGGTATCATTTCGATGGGACGGATTGGGACCAGTCTC<br>GCCAACTCGCAAATCGTATTTATAAGTTTAGAGGAGACGGAAAAGCATGGG<br>ACTGGGAAGTTGACACTGAAAATGGGAACTATGATTACTTAATGTATGCAGA<br>CGTTGACATGGATCATCCAGAAGTGATTAACGAACTAAACCGTTGGGGCGT<br>CTGGTACGCGAATACCCTTAATTTAGACGGCTTCCGACTGGATGCAGTGAA<br>ACATATTAAATTTAGCTTCATGCGTGATTGGTTAGGGCATGTTCGCGGGCAA<br>ACGGGCAAGAATCTTTTTGCCGTTGCAGAGTATTGGAAGAATGACCTAGGG<br>GCTTTAGAAAATTATTTAAGCAAAACAAATTGGACGATGAGCGCCTTTGATG<br>TTCCGCTTCATTACAACCTTTATCAAGCGTCAAATAGTAGCGGAAATTACGA<br>CATGAGAAACTTGTTAAATGGAACACTCGTTCAACGTCATCCGAGCCATGC<br>GGTTACGTTTGTCGATAACCACGACACACAGCCTGGAGAAGCCCTCGAATC<br>GTTCGTTCAAGGCTGGTTTAAACCACTAGCTTATGCAACGATTCTTACGAGA<br>GAGCAAGGCTACCCACAAGTGTTTTACGGCGATTATTATGGCATCCCAAGT<br>GACGGTGTTCCAAGCTACCGTCAACAGATCGACCCACTTTTAAAAGCTCGT<br>CAACAATATGCTTATGGTAGACAGCACGATTACTTTGATCATTGGGATGTAA<br>TTGGCTGGACACGTGAAGGAAACGCATCTCACCCGAACTCAGGACTTGCAA<br>CCATTATGTCTGATGGTCCAGGTGGATCAAAATGGATGTATGTTGGCCGTC<br>AGAAAGCTGGCGAAGTGTGGCATGACATGACTGGAAACCGCAGTGGCACT<br>GTGACAATTAATCAAGACGGCTGGGGACACTTTTTTGTCAACGGCGGCTCT<br>GTCTCCGTATGGGTGAAACGATAA |
| 2 | MNRWKAAFSWMLSLALVFTLFYTPSSASAHHDGTNGTIMQYFEWNVPNDGQ<br>HWNRLHNNAQNLKNAGITAIWIPPAWKGTSQNDVGYGAYDLYDLGEFNQKGT |

(continued)

| SEQ ID NO: | Sequence |
|---|---|
| | VRTKYGTKAELERAIRSLKANGIQVYGDVVMNHKGGADFTERVQAVEVNPQN RNQEVSGTYQIEAWTGFNFPGRGNQHSSFKWRWYHFDGTDWDQSRQLANRI YKFRGDGKAWDWEVDTENGNYDYLMYADVDMDHPEVINELNRWGVWYANT LNLDGFRLDAVKHIKFSFMRDWLGHVRGQTGKNLFAVAEYWKNDLGALENYL SKTNWTMSAFDVPLHYNLYQASNSSGNYDMRNLLNGTLVQRHPSHAVTFVDN HDTQPGEALESFVQGWFKPLAYATILTREQGYPQVFYGDYYGIPSDGVPSYRQ QIDPLLKARQQYAYGRQHDYFDHWDVIGWTREGNASHPNSGLATIMSDGPGG SKWMYVGRQKAGEVWHDMTGNRSGTVTINQDGWGHFFVNGGSVSVWVKR |
| 3 | HHDGTNGTIMQYFEWNVPNDGQHWNRLHNNAQNLKNAGITAIWIPPAWKG TSQNDVGYGAYDLYDLGEFNQKGTVRTKYGTKAELERAIRSLKANGIQVYGDV VMNHKGGADFTERVQAVEVNPQNRNQEVSGTYQIEAWTGFNFPGRGNQHSS FKWRWYHFDGTDWDQSRQLANRIYKFRGDGKAWDWEVDTENGNYDYLMYA DVDMDHPEVINELNRWGVWYANTLNLDGFRLDAVKHIKFSFMRDWLGHVRG QTGKNLFAVAEYWKNDLGALENYLSKTNWTMSAFDVPLHYNLYQASNSSGNY DMRNLLNGTLVQRHPSHAVTFVDNHDTQPGEALESFVQGWFKPLAYATILTRE QGYPQVFYGDYYGIPSDGVPSYRQQIDPLLKARQQYAYGRQHDYFDHWDVIG WTREGNASHPNSGLATIMSDGPGGSKWMYVGRQKAGEVWHDMTGNRSGTV TINQDGWGHFFVNGGSVSVWVKR |
| 4 | HHDGTNGTIMQYFEWNVPNDGQHWNRLHNNAQNLKNAGITAIWIPPAWKGTS QNDVGYGAYDLYDLGEFNQKGTVRTKYGTKAELERAIRSLKANGIQVYGDVVM NHKGGADFTERVQAVEVNPQNRNQEVSGTYQIEAWTGFNFPGRGNQHSSFK WRWYHFDGTDWDQSRQLANRIYKFRGKAWDWEVDTENGNYDYLMYADVDM DHPEVINELNRWGVWYANTLNLDGFRLDAVKHIKFSFMRDWLGHVRGQTGKN LFAVAEYWKNDLGALENYLSKTNWTMSAFDVPLHYNLYQASNSSGNYDMRNL LNGTLVQRHPSHAVTFVDNHDTQPGEALESFVQGWFKPLAYATILTREQGYPQ VFYGDYYGIPSDGVPSYRQQIDPLLKARQQYAYGRQHDYFDHWDVIGWTREG NASHPNSGLATIMSDGPGGSKWMYVGRQKAGEVWHDMTGNRSGTVTINQD GWGHFFVNGGSVSVWVKR |

(continued)

| SEQ ID NO: | Sequence |
|---|---|
| 5 | HHNGTNGTMMQYFEWYLPNDGNHWNRLNSDASNLKSKGITAVWIPPAWK GASQNDVGYGAYDLYDLGEFNQKGTVRTKYGTRSQLQAAVTSLKNNGIQVYG DVVMNHKGGADATEMVRAVEVNPNNRNQEVTGEYTIEAWTRFDFPGRGNT HSSFKWRWYHFDGVDWDQSRRLNNRIYKFRGHGKAWDWEVDTENGNYDY LMYADIDMDHPEVVNELRNWGVWYTNTLGLDGFRIDAVKHIKYSFTRDWIN HVRSATGKNMFAVAEFWKNDLGAIENYLQKTNWNHSVFDVPLHYNLYNASK SGGNYDMRNIFNGTVVQRHPSHAVTFVDNHDSQPEEALESFVEEWFKPLAY ALTLTREQGYPSVFYGDYYGIPTHGVPAMRSKIDPILEARQKYAYGKQNDYLD H HNIIGWTREGNTAHPNSGLATIMSDGAGGSKWMFVGRNKAGQVWSDITGN RTGTVTINADGWGNFSVNGGSVSIWVNK |
| 6 | HHNGTNGTMMQYFEWYLPNDGNHWNRLRSDASNLKDKGISAVWIPPAW KGASQNDVGYGAYDLYDLGEFNQKGTIRTKYGTRNQLQAAVNALKSNGIQV YGDVVMNHKGGADATEMVRAVEVNPNNRNQEVSGEYTIEAWTKFDFPGR GNTHSNFKWRWYHFDGVDWDQSRKLNNRIYKFRGDGKGWDWEVDTEN GNYDYLMYADIDMDHPEVVNELRNWGVWYTNTLGLDGFRIDAVKHIKYSF TRDWINHVRSATGKNMFAVAEFWKNDLGAIENYLNKTNWNHSVFDVPLH YNLYNASKSGGNYDMRQIFNGTVVQRHPMHAVTFVDNHDSQPEEALESFV EEWFKPLAYALTLTREQGYPSVFYGDYYGIPTHGVPAMKSKIDPILEARQKYA YGRQNDYLDHHNIIGWTREGNTAHPNSGLATIMSDGAGGNKWMFVGRN KAGQVWTDITGNRAGTVTINADGWGNFSVNGGSVSIWVNK |
| 7 | MKRWVVAMLAVLFLFPSVVVADGLNGTMMQYYEWHLENDGQHWNRLHDDA EALSNAGITAIWIPPAYKGNSQADVGYGAYDLYDLGEFNQKGTVRTKYGTKAQ LERAIGSLKSNDINVYGDVVMNHKLGADFTEAVQAVQVNPSNRWQDISGVYTI DAWTGFDFPGRNNAYSDFKWRWFHFNGVDWDQRYQENHLFRFANTNWNW RVDEENGNYDYLLGSNIDFSHPEVQEELKDWGSWFTDELDLDGYRLDAIKHIP FWYTSDWVRHQRSEADQDLFVVGEYWKDDVGALEFYLDEMNWEMSLFDVPL NYNFYRASKQGGSYDMRNILRGSLVEAHPIHAVTFVDNHDTQPGESLESWVA DWFKPLAYATILTREGGYPNVFYGDYYGIPNDNISAKKDMIDELLDARQNYAYG TQHDYFDHWDIVGWTREGTSSRPNSGLATIMSNGPGGSKWMYVGQQHAGQ TWTDLTGNHAASVTINGDGWGEFFTNGGSVSVYVNQ |

(continued)

| SEQ ID NO: | Sequence |
|---|---|
| 8 | AQSVPWGISRVQAPAAHNRGLTGSGVKVAVLDTGISTHPDLNIRGGASFVPGE PSTQDGNGHGTHVAGTIAALNNSIGVLGVAPSAELYAVKVLGASGSGSVSSIA QGLEWAGNNGMHVANLSLGSPSPSATLEQAVNSATSRGVLVVAASGNSGAG SISYPARYANAMAVGATDQNNNRASFSQYGAGLDIVAPGVNVQSTYPGSTYA SLNGTSMATPHVAGAAALVKQKNPSWSNVQIRNHLKNTATSLGSTNLYGSGLV NAEAATR |
| 9 | HHNGTNGTMMQYFEWYLPNDGNHWNRLNSDASNLKSKGITAVWIPPAWK GASQNDVGYGAYDLYDLGEFNQKGTVRTKYGTRSQLQAAVTSLKNNGIQVYG DVVMNHKGGADATEMVRAVEVNPNNRNQEVTGEYTIEAWTRFDFPGRGNT HSSFKWRWYHFDGVDWDQSRRLNNRIYKFRGKAWDWEVDTENGNYDYLM YADIDMDHPEVVNELRNWGVWYTNTLGLDGFRIDAVKHIKYSFTRDWINHV RSATGKNMFAVAEFWKNDLGAIENYLQKTNWNHSVFDVPLHYNLYNASKSG GNYDMRNIFNGTVVQRHPSHAVTFVDNHDSQPEEALESFVEEWFKPLAYALT LTREQGYPSVFYGDYYGIPTHGVPAMRSKIDPILEARQKYAYGPQHDYLDHPD VIGWTREGDSSHPKSGLATLITDGPGGSKRMYAGLKNAGETWYDITGNRSDT VKIGSDGWGEFHVNDGSVSIYVQK |
| 10 | DGLNGTMMQYYEWHLENDGQHWNRLHDDAAALSDAGITAIWIPPAYKGNSQA DVGYGAYDLYDLGEFNQKGTVRTKYGTKAQLERAIGSLKSNDINVYGD VVMNHKMGADFTEAVQAVQVNPTNRWQDISGAYTIDAWTGFDFSGRNNAYS DFKWRWFHFNGVDWDQRYQENHIFRFANTNWNWRVDEENGNYDYLLGSN IDFSHPEVQDELKDWGSWFTDELDLDGYRLDAIKHIPFWYTSDWVRHQRNEA DQDLFVVGEYWKDDVGALEFYLDEMNWEMSLFDVPLNYNFYRASQQGG SYDMRNILRGSLVEAHPMHAVTFVDNHDTQPGESLESWVADWFKPLAYATILT REGGYPNVFYGDYYGIPNDNISAKKDMIDELLDARQNYAYGTQHDYF DHWDVVGWTREGSSSRPNSGLATIMSNGPGGSKWMYVGRQNAGQTWTDLT GNNGASVTINGDGWGEFFTNGGSVSVYVNQ |

(continued)

| SEQ ID NO: | Sequence |
|---|---|
| 11 | HHNGTNGTMMQYFEWHLPNDGNHWNRLRDDAANLKSKGITAVWIPPAWKGT SQNDVGYGAYDLYDLGEFNQKGTVRTKYGTRSQLQGAVTSLKNNGIQVY GDVVMNHKGGADGTEMVNAVEVNRSNRNQEISGEYTIEAWTKFDFPGRGNT HSNFKWRWYHFDGTDWDQSRQLQNKIYKFRGTGKAWDWEVDIENGNYDY LMYADIDMDHPEVINELRNWGVWYTNTLNLDGFRIDAVKHIKYSYTRDWLTHV RNTTGKPMFAVAEFWKNDLAAIENYLNKTSWNHSVFDVPLHYNLYNA SNSGGYFDMRNILNGSVVQKHPIHAVTFVDNHDSQPGEALESFVQSWFKPLA YALILTREQGYPSVFYGDYYGIPTHGVPSMKSKIDPLLQARQTYAYGT QHDYFDHHDIIGWTREGDSSHPNSGLATIMSDGPGGNKWMYVGKHKAGQVW RDITGNRSGTVTINADGWGNFTVNGGAVSVWVKQ |
| 12 | HHNGTNGTMMQYFEWYLPNDGNHWNRLRSDASNLKDKGITAVWIPPAW KGASQNDVGYGAYDLYDLGEFNQKGTVRTKYGTRNQLQAAVTALKSNGIQV YGDVVMNHKGGADATEWVRAVEVNPSNRNQEVSGDYTIEAWTKFDFPGR GNTHSNFKWRWYHFDGVDWDQSRQLQNRIYKFRGDGKGWDWEVDTEN GNYDYLMYADIDMDHPEVVNELRNWGVWYTNTLGLDGFRIDAVKHIKYSF TRDWLTHVRNTTGKNMFAVAEFWKNDIGAIENYLSKTNWNHSVFDVPLHY NLYNASRSGGNYDMRQIFNGTVVQRHPTHAVTFVDNHDSQPEEALESFVE EWFKPLACALTLTRDQGYPSVFYGDYYGIPTHGVPAMKSKIDPILEARQKYAY GKQNDYLDHHNMIGWTREGNTAHPNSGLATIMSDGPGGNKWMYVGRNK AGQVWRDITGNRSGTVTINADGWGNFSVNGGSVSIWVNN |
| 13 | HHDGTNGTIMQYFEWNVPNDGQHWNRLHNNAQNLKNAGITAIWIPPAWKGTS QNDVGYGAYDLYDLGEFNQKGTVRTKYGTKAELERAIRSLKANGIQVYGDVVM NHKGGADFTERVQAVEVNPQNRNQEVSGTYEIEAWTGFNFPGRGNQHSSFK WRWYHFDGTDWDQSRQLSNRIYKFRGDGKAWDWEVDTENGNYDYLMYADV DMNHPEVINELNRWGVWYANTLNLDGFRLDAVKHIQFSFMRNWLGHVRGQT GKNLFAVAEYWKNDLGALENYLSKTNWTMSAFDVPLHYNLYQASNSGGNYD MRNLLNGTLVQRHPSHAVTFVDNHDTQPGEALESFVQGWFKPLAYATILTREQ GYPQVFYGDYYGIPSDGVPSYRQQIDPLLKARQQYAYGRQHDYFDHWDVIGW TREGNASHPNSGLATIMSDGPGGSKWMYVGRQKAGEVWHDITGNRSGTVTIN QDGWGQFFVNGGSVSVWVKR |

## Claims

1. Use of an alpha-amylase variant of a parent alpha-amylase in a cleaning process, wherein a bleaching system is added in a concentration of at least 5 weight%, wherein said parent has an amino acid sequence which is at least 80% identical to the amino acid sequence set forth in SEQ ID NO: 3, wherein said variant comprises a substitution in one or more positions providing oxidation stability of said variant, wherein said variant comprises a substitution in position 202, wherein said position corresponds to the amino acid position of the amino acid sequence as set forth in SEQ ID NO: 3, wherein said substitution is a M202L substitution, wherein said variant has a sequence identity of at least 80%, but less than 100% to the amino acid sequence set forth in SEQ ID NO: 3, wherein said variant has an improvement factor of ≥1.0 as a measure for wash performance, when compared to said parent alpha-amylase, wherein said variant has alpha-amylase activity.

2. The use of a variant according to the preceding claim, wherein said parent alpha-amylase has an amino acid sequence as set forth in SEQ ID NO: 3, or has an amino acid sequence which is at least 85%, such as at least 90%, such as at least 95%, such as at least 98%, identical to the amino acid sequence as set forth in SEQ ID NO: 3.

3. The use of a variant according to any one of the preceding claims, wherein said parent alpha-amylase comprises or consists of the amino acid sequence as set forth in SEQ ID NO: 3.

4. The use of a variant according to any one of the preceding claims, wherein said variant has a sequence identity of at least 85%, such as at least 90%, such as at least 95%, such as at least 98%, but less than 100% to the amino acid sequence as set forth in SEQ ID NO: 3.

5. The use of a variant according to any one of the preceding claims, wherein said variant comprises a deletion in two or more positions corresponding to positions R181, G182, D183, and G184 of the amino acid sequence as set forth in SEQ ID NO: 3.

6. A composition comprising a variant as defined in any one of the preceding claims, wherein said composition further comprises one or more surfactants and one or more bleaching systems and one or more builders.

7. The composition according to claim 6, wherein said composition is a detergent composition, such as a liquid laundry or liquid dish wash composition, such as an Automatic Dish Wash (ADW) liquid detergent composition, or a powder laundry, such as a soap bar, or powder dish wash composition, such as an ADW detergent composition.

8. The composition according to any one of claims 6 and 7, wherein said composition further comprises one or more sulfonated polymers, one or more chelators and/or one or more builders.

9. A method for removing a stain from a surface comprising contacting the surface with a composition of any of claims 6-8.

## Patentansprüche

1. Verwendung einer alpha-Amylasevariante einer parentalen alpha-Amylase in einem Reinigungsverfahren, wobei ein Bleichsystem in einer Konzentration von mindestens 5 Gew.-% zugegeben wird, wobei das Parentale eine Aminosäuresequenz aufweist, die zur in SEQ ID NO: 3 dargestellten Aminosäuresequenz mindestens 80% identisch ist, wobei die Variante eine Substitution in einer oder mehreren Positionen umfasst, die Oxidationsstabilität der Variante bereitstellen, wobei die Variante eine Substitution in Position 202 umfasst, wobei die Position der Aminosäureposition der wie in SEQ ID NO: 3 dargestellten Aminosäuresequenz entspricht, wobei die Substitution eine M202L-Substitution ist, wobei die Variante eine Sequenzidentität von mindestens 80%, aber weniger als 100% zur in SEQ ID NO: 3 dargestellten Aminosäuresequenz aufweist, wobei die Variante einen Verbesserungsfaktor von ≥1,0 als ein Maß für die Waschleistung aufweist, wenn sie mit der parentalen alpha-Amylase verglichen wird, wobei die Variante alpha-Amylaseaktivität aufweist.

2. Verwendung einer Variante nach dem voranstehenden Anspruch, wobei die parentale alpha-Amylase eine wie in SEQ ID NO: 3 dargestellte Aminosäuresequenz aufweist, oder eine Aminosäuresequenz aufweist, die mindestens 85%, wie mindestens 90%, wie mindestens 95%, wie mindestens 98% identisch zur wie in SEQ ID NO: 3 dargestellten Aminosäuresequenz ist.

3. Verwendung einer Variante nach einem beliebigen der voranstehenden Ansprüche, wobei die parentale alpha-Amylase die wie in SEQ ID NO: 3 dargestellte Aminosäuresequenz umfasst oder daraus besteht.

4. Verwendung einer Variante nach einem beliebigen der voranstehenden Ansprüche, wobei die Variante eine Sequenzidentität von mindestens 85%, wie mindestens 90%, wie mindestens 95%, wie mindestens 98%, aber weniger als 100% zur wie in SEQ ID NO: 3 dargestellten Aminosäuresequenz aufweist.

5. Verwendung einer Variante nach einem beliebigen der voranstehenden Ansprüche, wobei die Variante eine Deletion in zwei oder mehr Positionen umfasst, die den Positionen R181, G182, D183 und G184 der wie in SEQ ID NO: 3 dargestellten Aminosäuresequenz entsprechen.

6. Zusammensetzung, die eine wie in einem beliebigen der voranstehenden Ansprüche definierte Variante umfasst, wobei die Zusammensetzung des Weiteren ein oder mehrere oberflächenaktive Mittel und ein oder mehrere Bleichsysteme und einen oder mehrere Gerüststoffe umfasst.

7. Zusammensetzung nach Anspruch 6, wobei die Zusammensetzung eine Detergenszusammensetzung ist, wie eine flüssige Wäsche- oder flüssige Geschirrspülzusammensetzung, wie eine flüssige Detergenszusammensetzung zum automatisierten Geschirrspülen *(Automatic Dish Wash (ADW)),* oder eine pulverförmige Wäsche-, wie ein Seifenstück, oder pulverförmige Geschirrspülzusammensetzung, wie eine ADW-Detergenszusammensetzung.

8. Zusammensetzung nach einem beliebigen der Ansprüche 6 und 7, wobei die Zusammensetzung des Weiteren ein oder mehrere sulfonierte Polymere, einen oder mehrere Chelatbildner und/oder einen oder mehrere Gerüststoffe umfasst.

9. Verfahren zum Entfernen eines Flecks von einer Oberfläche, das Inkontaktbringen der Oberfläche mit einer Zusammensetzung gemäß einem beliebigen der Ansprüche 6-8 umfasst.

## Revendications

1. Utilisation d'un variant d'alpha-amylase d'une alpha-amylase parente dans un procédé de nettoyage, dans laquelle un système de blanchiment est ajouté à une concentration d'au moins 5 % en poids, dans laquelle ledit parent a une séquence d'acides aminés qui est identique à au moins 80 % à la séquence d'acides aminés présentée dans la SEQ ID NO : 3, dans laquelle ledit variant comprend une substitution à une ou plusieurs positions conférant audit variant une stabilité à l'oxydation, dans laquelle ledit variant comprend une substitution à la position 202, dans laquelle ladite position correspond à la position d'acide aminé de la séquence d'acides aminés telle que présentée dans la SEQ ID NO : 3, dans laquelle ladite substitution est une substitution M202L, dans laquelle ledit variant a une identité de séquence d'au moins 80 %, mais inférieure à 100 %, avec la séquence d'acides aminés présentée dans la SEQ ID NO : 3, dans laquelle ledit variant a un facteur d'amélioration $\geq$ 1,0 en tant que mesure de performance de lavage, en comparaison avec ladite alpha-amylase parente, dans laquelle ledit variant a une activité d'alpha-amylase.

2. Utilisation d'un variant selon la revendication précédente, dans laquelle ladite alpha-amylase parente a une séquence d'acides aminés telle que présentée dans la SEQ ID NO : 3, ou a une séquence d'acides aminés qui est identique à au moins 85 %, telle qu'au moins 90 %, telle qu'au moins 95 %, telle qu'au moins 98 %, à la séquence d'acides aminés telle que présentée dans la SEQ ID NO : 3.

3. Utilisation d'un variant selon l'une quelconque des revendications précédentes, dans laquelle ladite alpha-amylase parente comprend ou consiste en la séquence d'acides aminés telle que présentée dans la SEQ ID NO : 3.

4. Utilisation d'un variant selon l'une quelconque des revendications précédentes, dans laquelle ledit variant a une identité de séquence d'au moins 85 %, telle qu'au moins 90 %, telle qu'au moins 95 %, telle qu'au moins 98 %, mais inférieure à 100 %, avec la séquence d'acides aminés telle que présentée dans la SEQ ID NO : 3.

5. Utilisation d'un variant selon l'une quelconque des revendications précédentes, dans laquelle ledit variant comprend une délétion à deux ou plus de deux positions correspondant aux positions R181, G182, D183 et G184 de la séquence d'acides aminés telle que présentée dans la SEQ ID NO : 3.

6. Composition comprenant un variant tel que défini dans l'une quelconque des revendications précédentes, laquelle composition comprend en outre un ou plusieurs tensioactifs et un ou plusieurs systèmes de blanchiment et un ou plusieurs adjuvants.

7. Composition selon la revendication 6, laquelle composition est une composition détergente, telle qu'une composition liquide pour le linge ou une composition liquide pour la vaisselle, telle qu'une composition détergente liquide pour lave-vaisselle, ou une poudre pour le linge, telle qu'un pain de savon, ou une composition en poudre pour la vaisselle, telle qu'une composition détergente pour lave-vaisselle.

8. Composition selon l'une quelconque des revendications 6 et 7, laquelle composition comprend en outre un ou plusieurs polymères sulfonés, un ou plusieurs chélatants et/ou un ou plusieurs adjuvants.

9. Méthode pour retirer une tache sur une surface, comprenant la mise en contact de la surface avec une composition de l'une quelconque des revendications 6 à 8.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 199418314 A
- WO 0060058 A2
- WO 2009102854 A1
- WO 9418314 A1
- WO 9623873 A1
- GB 1466799 A
- WO 1996023873 A
- US 20040171154
- WO 9517413 A
- WO 9522625 A
- US 5223409 A
- WO 9206204 A
- WO 9015861 A
- WO 2010096673 A
- WO 9219709 A
- WO 9219708 A
- EP 1025240 A
- WO 9707202 A
- US 4246612 A
- US 5227084 A
- US 5114611 A
- US 4810410 A
- WO 9906521 A
- WO 9426860 A
- WO 9426859 A
- US 4223163 A
- WO 9422800 A
- US 20090011970 A1
- WO 09102854 A
- US 5977053 A
- WO 2006130575 A
- US 4435307 A
- US 5648263 A
- US 5691178 A
- US 5776757 A
- WO 8909259 A
- EP 0495257 A
- EP 0531372 A
- WO 9611262 A
- WO 9629397 A
- WO 9808940 A
- WO 9407998 A
- EP 0531315 A
- US 5457046 A
- US 5686593 A
- US 5763254 A
- WO 9524471 A
- WO 9812307 A
- DK 9800299 W
- WO 02099091 A
- EP 258068 A
- EP 305216 A
- WO 9613580 A
- EP 218272 A
- EP 331376 A
- WO 9506720 A
- WO 9627002 A
- WO 9612012 A
- WO 10065455 A
- WO 10107560 A
- US 5389536 A
- WO 11084412 A
- WO 11084417 A
- WO 11084599 A
- WO 11150157 A
- WO 12137147 A
- WO 10111143 A
- WO 0556782 A
- WO 0967279 A
- WO 10100028 A
- EP 407225 A
- WO 9205249 A
- WO 9401541 A
- WO 9425578 A
- WO 9514783 A
- WO 9530744 A
- WO 9535381 A
- WO 9522615 A
- WO 9600292 A
- WO 9704079 A
- WO 0034450 A
- WO 0060063 A
- WO 0192502 A
- WO 0787508 A
- WO 09109500 A
- WO 9324618 A
- WO 9510602 A
- WO 9815257 A
- US 4106991 A
- US 4661452 A
- GB 1483591 A
- EP 238216 A
- WO 2004111220 A
- WO 0242740 A

**Non-patent literature cited in the description**


- **NEEDLEMAN ; WUNSCH.** *J. Mol. Biol.,* 1970, vol. 48, 443-453
- **RICE et al.** EMBOSS: The European Molecular Biology Open Software Suite. *Trends Genet.,* 2000, vol. 16, 276-277
- **EDGAR.** *Nucleic Acids Research,* 2004, vol. 32, 1792-1797
- **KATOH ; KUMA.** *Nucleic Acids Research,* 2002, vol. 30, 3059-3066
- **KATOH et al.** *Nucleic Acids Research,* 2005, vol. 33, 511-518
- **KATOH ; TOH.** *Bioinformatics,* 2007, vol. 23, 372-374
- **KATOH et al.** *Methods in Molecular Biology,* 2009, vol. 537, 39-64
- **KATOH ; TOH.** *Bioinformatics,* 2010, vol. 26, 1899-1900
- **THOMPSON et al.** *Nucleic Acids Research,* 1994, vol. 22, 4673-4680
- **LINDAHL ; ELOFSSON.** *J. Mol. Biol.,* 2000, vol. 295, 613-615
- **ATSCHUL et al.** *Nucleic Acids Res.,* 1997, vol. 25, 3389-3402
- **JONES.** *J. Mol. Biol.,* 1999, vol. 287, 797-815
- **MCGUFFIN ; JONES.** *Bioinformatics,* 2003, vol. 19, 874-881
- **GOUGH et al.** *J. Mol. Biol.,* 2000, vol. 313, 903-919
- **HOLM ; SANDER.** *Proteins,* 1998, vol. 33, 88-96
- **SHINDYALOV ; BOURNE.** *Protein Engineering,* 1998, vol. 11, 739-747
- **HOLM ; PARK.** *Bioinformatics,* 2000, vol. 16, 566-567
- **RICE et al.** *EMBOSS: The European Molecular Biology Open Software Suite,* 2000
- **H. NEURATH ; R.L. HILL.** The Proteins. Academic Press, 1979
- **CUNNINGHAM ; WELLS.** *Science,* 1989, vol. 244, 1081-1085
- **HILTON et al.** *J. Biol. Chem.,* 1996, vol. 271, 4699-4708
- **DE VOS et al.** *Science,* 1992, vol. 255, 306-312
- **SMITH et al.** *J. Mol. Biol.,* 1992, vol. 224, 899-904
- **WLODAVER et al.** *FEBS Lett.,* 1992, vol. 309, 59-64
- Protein Purification. VCH Publishers, 1989
- **SCHERER ; DAVIS.** *Proc. Natl. Acad. Sci. USA,* 1979, vol. 76, 4949-4955
- **BARTON et al.** *Nucleic Acids Res.,* 1990, vol. 18, 7349-4966
- **STORICI et al.** *Nature Biotechnol.,* 2001, vol. 19, 773-776
- **KREN et al.** *Nat. Med.,* 1998, vol. 4, 285-290
- **CALISSANO ; MACINO.** *Fungal Genet. Newslett.,* 1996, vol. 43, 15-16
- **TIAN et al.** *Nature,* 2004, vol. 432, 1050-1054
- **REIDHAAR-OLSON ; SAUER.** *Science,* 1988, vol. 241, 53-57
- **BOWIE ; SAUER.** *Proc. Natl. Acad. Sci. USA,* 1989, vol. 86, 2152-2156
- **LOWMAN et al.** *Biochemistry,* 1991, vol. 30, 10832-10837
- **DERBYSHIRE et al.** *Gene,* 1986, vol. 46, 145
- **NER et al.** *DNA,* 1988, vol. 7, 127
- **NESS et al.** *Nature Biotechnology,* 1999, vol. 17, 893-896
- **HODGDON ; KALER.** *Current Opinion in Colloid & Interface Science,* 2007, vol. 12, 121-128
- **M.K.NAGARAJAN et al.** *JAOCS,* September 1984, vol. 61 (9), 1475-1478
- Powdered Detergents, Surfactant science series. Marcel Dekker, Inc, vol. 71

SEQUENCE LISTING

<110> Novozymes A/S

<120> OXIDATION STABLE ALPHA-AMYLASE VARIANTS

<130> 13076-WO-PCT

<160> 13

<170> PatentIn version 3.5

<210> 1
<211> 1458
<212> DNA
<213> Bacillus sp.

<400> 1

```
catcacgatg ggacgaacgg aacgattatg cagtattttg aatggaacgt tccgaatgat      60
ggacaacatt ggaaccgctt acacaacaac gctcaaaatt taaaaaatgc cggaattaca     120
gcaatctgga ttccacctgc gtggaaagga acgagccaaa atgatgtagg ctacggtgcg     180
tatgaccttt atgaccttgg tgaatttaac caaaaaggaa cggtccgtac gaaatatgga     240
acaaaagcag aattagaacg agcgattcgt tcgttaaagg cgaacgggat tcaagtgtat     300
ggcgatgttg ttatgaacca taaaggcgga gctgatttca ccgagcgtgt tcaagcggtt     360
gaagtgaacc cgcaaaaccg aaaccaagaa gtgtctggca cttatcaaat cgaagcatgg     420
acagggttca attttcctgg acgtggcaat caacattctt cgtttaaatg gcgctggtat     480
catttcgatg ggacggattg ggaccagtct cgccaactcg caaatcgtat ttataagttt     540
agaggagacg gaaaagcatg ggactgggaa gttgacactg aaaatgggaa ctatgattac     600
ttaatgtatg cagacgttga catggatcat ccagaagtga ttaacgaact aaaccgttgg     660
ggcgtctggt acgcgaatac ccttaattta gacggcttcc gactggatgc agtgaaacat     720
attaaattta gcttcatgcg tgattggtta gggcatgttc gcgggcaaac gggcaagaat     780
ctttttgccg ttgcagagta ttggaagaat gacctagggg ctttagaaaa ttatttaagc     840
aaaacaaatt ggacgatgag cgcctttgat gttccgcttc attacaacct ttatcaagcg     900
tcaaatagta gcggaaatta cgacatgaga aacttgttaa atggaacact cgttcaacgt     960
catccgagcc atgcggttac gtttgtcgat aaccacgaca cacagcctgg agaagccctc    1020
gaatcgttcg ttcaaggctg gtttaaacca ctagcttatg caacgattct tacgagagag    1080
caaggctacc cacaagtgtt ttacggcgat tattatggca tcccaagtga cggtgttcca    1140
agctaccgtc aacagatcga cccactttta aaagctcgtc aacaatatgc ttatggtaga    1200
cagcacgatt actttgatca ttgggatgta attggctgga cacgtgaagg aaacgcatct    1260
cacccgaact caggacttgc aaccattatg tctgatggtc caggtggatc aaaatggatg    1320
tatgttggcc gtcagaaagc tggcgaagtg tggcatgaca tgactggaaa ccgcagtggc    1380

actgtgacaa ttaatcaaga cggctgggga cactttttgg tcaacggcgg ctctgtctcc    1440
gtatgggtga aacgataa                                                  1458
```

<210> 2
<211> 514
<212> PRT
<213> Bacillus sp.

<220>
<221> SIGNAL
<222> (1) .. (29)

<220>
<221> mat_peptide
<222> (30) .. (514)

<400> 2

```
Met Asn Arg Trp Lys Ala Ala Phe Ser Trp Met Leu Ser Leu Ala Leu
                -25                 -20                 -15

Val Phe Thr Leu Phe Tyr Thr Pro Ser Ser Ala Ser Ala His His Asp
            -10                 -5              -1  1

Gly Thr Asn Gly Thr Ile Met Gln Tyr Phe Glu Trp Asn Val Pro Asn
    5                   10                  15

Asp Gly Gln His Trp Asn Arg Leu His Asn Asn Ala Gln Asn Leu Lys
20                  25                  30                  35

Asn Ala Gly Ile Thr Ala Ile Trp Ile Pro Pro Ala Trp Lys Gly Thr
                40                  45                  50

Ser Gln Asn Asp Val Gly Tyr Gly Ala Tyr Asp Leu Tyr Asp Leu Gly
            55                  60                  65

Glu Phe Asn Gln Lys Gly Thr Val Arg Thr Lys Tyr Gly Thr Lys Ala
        70                  75                  80

Glu Leu Glu Arg Ala Ile Arg Ser Leu Lys Ala Asn Gly Ile Gln Val
    85                  90                  95

Tyr Gly Asp Val Val Met Asn His Lys Gly Gly Ala Asp Phe Thr Glu
100                 105                 110                 115

Arg Val Gln Ala Val Glu Val Asn Pro Gln Asn Arg Asn Gln Glu Val
                120                 125                 130

Ser Gly Thr Tyr Gln Ile Glu Ala Trp Thr Gly Phe Asn Phe Pro Gly
```

135 140 145

Arg Gly Asn Gln His Ser Ser Phe Lys Trp Arg Trp Tyr His Phe Asp
150 155 160

Gly Thr Asp Trp Asp Gln Ser Arg Gln Leu Ala Asn Arg Ile Tyr Lys
165 170 175

Phe Arg Gly Asp Gly Lys Ala Trp Asp Trp Glu Val Asp Thr Glu Asn
180 185 190 195

Gly Asn Tyr Asp Tyr Leu Met Tyr Ala Asp Val Asp Met Asp His Pro
200 205 210

Glu Val Ile Asn Glu Leu Asn Arg Trp Gly Val Trp Tyr Ala Asn Thr
215 220 225

Leu Asn Leu Asp Gly Phe Arg Leu Asp Ala Val Lys His Ile Lys Phe
230 235 240

Ser Phe Met Arg Asp Trp Leu Gly His Val Arg Gly Gln Thr Gly Lys
245 250 255

Asn Leu Phe Ala Val Ala Glu Tyr Trp Lys Asn Asp Leu Gly Ala Leu
260 265 270 275

Glu Asn Tyr Leu Ser Lys Thr Asn Trp Thr Met Ser Ala Phe Asp Val
280 285 290

Pro Leu His Tyr Asn Leu Tyr Gln Ala Ser Asn Ser Ser Gly Asn Tyr
295 300 305

Asp Met Arg Asn Leu Leu Asn Gly Thr Leu Val Gln Arg His Pro Ser
310 315 320

His Ala Val Thr Phe Val Asp Asn His Asp Thr Gln Pro Gly Glu Ala
325 330 335

Leu Glu Ser Phe Val Gln Gly Trp Phe Lys Pro Leu Ala Tyr Ala Thr
340 345 350 355

Ile Leu Thr Arg Glu Gln Gly Tyr Pro Gln Val Phe Tyr Gly Asp Tyr
360 365 370

Tyr Gly Ile Pro Ser Asp Gly Val Pro Ser Tyr Arg Gln Gln Ile Asp
375 380 385

Pro Leu Leu Lys Ala Arg Gln Gln Tyr Ala Tyr Gly Arg Gln His Asp
390 395 400

Tyr Phe Asp His Trp Asp Val Ile Gly Trp Thr Arg Glu Gly Asn Ala
405 410 415

Ser His Pro Asn Ser Gly Leu Ala Thr Ile Met Ser Asp Gly Pro Gly
420 425 430 435

Gly Ser Lys Trp Met Tyr Val Gly Arg Gln Lys Ala Gly Glu Val Trp
440 445 450

His Asp Met Thr Gly Asn Arg Ser Gly Thr Val Thr Ile Asn Gln Asp
455 460 465

Gly Trp Gly His Phe Phe Val Asn Gly Gly Ser Val Ser Val Trp Val
470 475 480

Lys Arg
485

<210> 3
<211> 485
<212> PRT
<213> Bacillus sp.

<400> 3

His His Asp Gly Thr Asn Gly Thr Ile Met Gln Tyr Phe Glu Trp Asn
1 5 10 15

Val Pro Asn Asp Gly Gln His Trp Asn Arg Leu His Asn Asn Ala Gln
20 25 30

Asn Leu Lys Asn Ala Gly Ile Thr Ala Ile Trp Ile Pro Pro Ala Trp
35 40 45

Lys Gly Thr Ser Gln Asn Asp Val Gly Tyr Gly Ala Tyr Asp Leu Tyr
50 55 60

Asp Leu Gly Glu Phe Asn Gln Lys Gly Thr Val Arg Thr Lys Tyr Gly
65 70 75 80

Thr Lys Ala Glu Leu Glu Arg Ala Ile Arg Ser Leu Lys Ala Asn Gly
85 90 95

Ile Gln Val Tyr Gly Asp Val Val Met Asn His Lys Gly Gly Ala Asp
100 105 110

```
Phe Thr Glu Arg Val Gln Ala Val Glu Val Asn Pro Gln Asn Arg Asn
        115                 120                 125

Gln Glu Val Ser Gly Thr Tyr Gln Ile Glu Ala Trp Thr Gly Phe Asn
    130                 135                 140

Phe Pro Gly Arg Gly Asn Gln His Ser Ser Phe Lys Trp Arg Trp Tyr
145                 150                 155                 160

His Phe Asp Gly Thr Asp Trp Asp Gln Ser Arg Gln Leu Ala Asn Arg
                165                 170                 175

Ile Tyr Lys Phe Arg Gly Asp Gly Lys Ala Trp Asp Trp Glu Val Asp
            180                 185                 190

Thr Glu Asn Gly Asn Tyr Asp Tyr Leu Met Tyr Ala Asp Val Asp Met
        195                 200                 205

Asp His Pro Glu Val Ile Asn Glu Leu Asn Arg Trp Gly Val Trp Tyr
    210                 215                 220

Ala Asn Thr Leu Asn Leu Asp Gly Phe Arg Leu Asp Ala Val Lys His
225                 230                 235                 240

Ile Lys Phe Ser Phe Met Arg Asp Trp Leu Gly His Val Arg Gly Gln
                245                 250                 255

Thr Gly Lys Asn Leu Phe Ala Val Ala Glu Tyr Trp Lys Asn Asp Leu
            260                 265                 270

Gly Ala Leu Glu Asn Tyr Leu Ser Lys Thr Asn Trp Thr Met Ser Ala
        275                 280                 285

Phe Asp Val Pro Leu His Tyr Asn Leu Tyr Gln Ala Ser Asn Ser Ser
    290                 295                 300

Gly Asn Tyr Asp Met Arg Asn Leu Leu Asn Gly Thr Leu Val Gln Arg
305                 310                 315                 320

His Pro Ser His Ala Val Thr Phe Val Asp Asn His Asp Thr Gln Pro
                325                 330                 335

Gly Glu Ala Leu Glu Ser Phe Val Gln Gly Trp Phe Lys Pro Leu Ala
            340                 345                 350

Tyr Ala Thr Ile Leu Thr Arg Glu Gln Gly Tyr Pro Gln Val Phe Tyr
        355                 360                 365
```

Gly Asp Tyr Tyr Gly Ile Pro Ser Asp Gly Val Pro Ser Tyr Arg Gln
370 375 380

Gln Ile Asp Pro Leu Leu Lys Ala Arg Gln Gln Tyr Ala Tyr Gly Arg
385 390 395 400

Gln His Asp Tyr Phe Asp His Trp Asp Val Ile Gly Trp Thr Arg Glu
405 410 415

Gly Asn Ala Ser His Pro Asn Ser Gly Leu Ala Thr Ile Met Ser Asp
420 425 430

Gly Pro Gly Gly Ser Lys Trp Met Tyr Val Gly Arg Gln Lys Ala Gly
435 440 445

Glu Val Trp His Asp Met Thr Gly Asn Arg Ser Gly Thr Val Thr Ile
450 455 460

Asn Gln Asp Gly Trp Gly His Phe Phe Val Asn Gly Gly Ser Val Ser
465 470 475 480

Val Trp Val Lys Arg
485

<210> 4
<211> 483
<212> PRT
<213> Bacillus sp.

<400> 4

His His Asp Gly Thr Asn Gly Thr Ile Met Gln Tyr Phe Glu Trp Asn
1 5 10 15

Val Pro Asn Asp Gly Gln His Trp Asn Arg Leu His Asn Asn Ala Gln
20 25 30

Asn Leu Lys Asn Ala Gly Ile Thr Ala Ile Trp Ile Pro Pro Ala Trp
35 40 45

Lys Gly Thr Ser Gln Asn Asp Val Gly Tyr Gly Ala Tyr Asp Leu Tyr
50 55 60

Asp Leu Gly Glu Phe Asn Gln Lys Gly Thr Val Arg Thr Lys Tyr Gly
65 70 75 80

Thr Lys Ala Glu Leu Glu Arg Ala Ile Arg Ser Leu Lys Ala Asn Gly
85 90 95

```
Ile Gln Val Tyr Gly Asp Val Val Met Asn His Lys Gly Gly Ala Asp
            100                 105                 110

Phe Thr Glu Arg Val Gln Ala Val Glu Val Asn Pro Gln Asn Arg Asn
        115                 120                 125

Gln Glu Val Ser Gly Thr Tyr Gln Ile Glu Ala Trp Thr Gly Phe Asn
    130                 135                 140

Phe Pro Gly Arg Gly Asn Gln His Ser Ser Phe Lys Trp Arg Trp Tyr
145                 150                 155                 160

His Phe Asp Gly Thr Asp Trp Asp Gln Ser Arg Gln Leu Ala Asn Arg
                165                 170                 175

Ile Tyr Lys Phe Arg Gly Lys Ala Trp Asp Trp Glu Val Asp Thr Glu
            180                 185                 190

Asn Gly Asn Tyr Asp Tyr Leu Met Tyr Ala Asp Val Asp Met Asp His
        195                 200                 205

Pro Glu Val Ile Asn Glu Leu Asn Arg Trp Gly Val Trp Tyr Ala Asn
    210                 215                 220

Thr Leu Asn Leu Asp Gly Phe Arg Leu Asp Ala Val Lys His Ile Lys
225                 230                 235                 240

Phe Ser Phe Met Arg Asp Trp Leu Gly His Val Arg Gly Gln Thr Gly
                245                 250                 255

Lys Asn Leu Phe Ala Val Ala Glu Tyr Trp Lys Asn Asp Leu Gly Ala
            260                 265                 270

Leu Glu Asn Tyr Leu Ser Lys Thr Asn Trp Thr Met Ser Ala Phe Asp
        275                 280                 285

Val Pro Leu His Tyr Asn Leu Tyr Gln Ala Ser Asn Ser Ser Gly Asn
    290                 295                 300

Tyr Asp Met Arg Asn Leu Leu Asn Gly Thr Leu Val Gln Arg His Pro
305                 310                 315                 320

Ser His Ala Val Thr Phe Val Asp Asn His Asp Thr Gln Pro Gly Glu
                325                 330                 335

Ala Leu Glu Ser Phe Val Gln Gly Trp Phe Lys Pro Leu Ala Tyr Ala
            340                 345                 350
```

Thr Ile Leu Thr Arg Glu Gln Gly Tyr Pro Gln Val Phe Tyr Gly Asp
355 360 365

Tyr Tyr Gly Ile Pro Ser Asp Gly Val Pro Ser Tyr Arg Gln Gln Ile
370 375 380

Asp Pro Leu Leu Lys Ala Arg Gln Gln Tyr Ala Tyr Gly Arg Gln His
385 390 395 400

Asp Tyr Phe Asp His Trp Asp Val Ile Gly Trp Thr Arg Glu Gly Asn
405 410 415

Ala Ser His Pro Asn Ser Gly Leu Ala Thr Ile Met Ser Asp Gly Pro
420 425 430

Gly Gly Ser Lys Trp Met Tyr Val Gly Arg Gln Lys Ala Gly Glu Val
435 440 445

Trp His Asp Met Thr Gly Asn Arg Ser Gly Thr Val Thr Ile Asn Gln
450 455 460

Asp Gly Trp Gly His Phe Phe Val Asn Gly Gly Ser Val Ser Val Trp
465 470 475 480

Val Lys Arg

<210> 5
<211> 485
<212> PRT
<213> Bacillus sp.

<400> 5

His His Asn Gly Thr Asn Gly Thr Met Met Gln Tyr Phe Glu Trp Tyr
1 5 10 15

Leu Pro Asn Asp Gly Asn His Trp Asn Arg Leu Asn Ser Asp Ala Ser
20 25 30

Asn Leu Lys Ser Lys Gly Ile Thr Ala Val Trp Ile Pro Pro Ala Trp
35 40 45

Lys Gly Ala Ser Gln Asn Asp Val Gly Tyr Gly Ala Tyr Asp Leu Tyr
50 55 60

Asp Leu Gly Glu Phe Asn Gln Lys Gly Thr Val Arg Thr Lys Tyr Gly
65 70 75 80

```
Thr Arg Ser Gln Leu Gln Ala Ala Val Thr Ser Leu Lys Asn Asn Gly
                85                  90                  95

Ile Gln Val Tyr Gly Asp Val Val Met Asn His Lys Gly Gly Ala Asp
            100                 105                 110

Ala Thr Glu Met Val Arg Ala Val Glu Val Asn Pro Asn Asn Arg Asn
        115                 120                 125

Gln Glu Val Thr Gly Glu Tyr Thr Ile Glu Ala Trp Thr Arg Phe Asp
    130                 135                 140

Phe Pro Gly Arg Gly Asn Thr His Ser Ser Phe Lys Trp Arg Trp Tyr
145                 150                 155                 160

His Phe Asp Gly Val Asp Trp Asp Gln Ser Arg Arg Leu Asn Asn Arg
                165                 170                 175

Ile Tyr Lys Phe Arg Gly His Gly Lys Ala Trp Asp Trp Glu Val Asp
            180                 185                 190

Thr Glu Asn Gly Asn Tyr Asp Tyr Leu Met Tyr Ala Asp Ile Asp Met
        195                 200                 205

Asp His Pro Glu Val Val Asn Glu Leu Arg Asn Trp Gly Val Trp Tyr
    210                 215                 220

Thr Asn Thr Leu Gly Leu Asp Gly Phe Arg Ile Asp Ala Val Lys His
225                 230                 235                 240

Ile Lys Tyr Ser Phe Thr Arg Asp Trp Ile Asn His Val Arg Ser Ala
                245                 250                 255

Thr Gly Lys Asn Met Phe Ala Val Ala Glu Phe Trp Lys Asn Asp Leu
            260                 265                 270

Gly Ala Ile Glu Asn Tyr Leu Gln Lys Thr Asn Trp Asn His Ser Val
        275                 280                 285

Phe Asp Val Pro Leu His Tyr Asn Leu Tyr Asn Ala Ser Lys Ser Gly
    290                 295                 300

Gly Asn Tyr Asp Met Arg Asn Ile Phe Asn Gly Thr Val Val Gln Arg
305                 310                 315                 320

His Pro Ser His Ala Val Thr Phe Val Asp Asn His Asp Ser Gln Pro
```

```
                325                 330                 335

Glu Glu Ala Leu Glu Ser Phe Val Glu Glu Trp Phe Lys Pro Leu Ala
            340                 345                 350

Tyr Ala Leu Thr Leu Thr Arg Glu Gln Gly Tyr Pro Ser Val Phe Tyr
        355                 360                 365

Gly Asp Tyr Tyr Gly Ile Pro Thr His Gly Val Pro Ala Met Arg Ser
    370                 375                 380

Lys Ile Asp Pro Ile Leu Glu Ala Arg Gln Lys Tyr Ala Tyr Gly Lys
385                 390                 395                 400

Gln Asn Asp Tyr Leu Asp His His Asn Ile Ile Gly Trp Thr Arg Glu
                405                 410                 415

Gly Asn Thr Ala His Pro Asn Ser Gly Leu Ala Thr Ile Met Ser Asp
            420                 425                 430

Gly Ala Gly Gly Ser Lys Trp Met Phe Val Gly Arg Asn Lys Ala Gly
        435                 440                 445

Gln Val Trp Ser Asp Ile Thr Gly Asn Arg Thr Gly Thr Val Thr Ile
    450                 455                 460

Asn Ala Asp Gly Trp Gly Asn Phe Ser Val Asn Gly Gly Ser Val Ser
465                 470                 475                 480

Ile Trp Val Asn Lys
                485
```

<210> 6
<211> 485
<212> PRT
<213> Bacillus sp.

<400> 6

```
His His Asn Gly Thr Asn Gly Thr Met Met Gln Tyr Phe Glu Trp Tyr
1               5                   10                  15

Leu Pro Asn Asp Gly Asn His Trp Asn Arg Leu Arg Ser Asp Ala Ser
            20                  25                  30

Asn Leu Lys Asp Lys Gly Ile Ser Ala Val Trp Ile Pro Pro Ala Trp
        35                  40                  45

Lys Gly Ala Ser Gln Asn Asp Val Gly Tyr Gly Ala Tyr Asp Leu Tyr
```

```
    50                  55                  60

Asp Leu Gly Glu Phe Asn Gln Lys Gly Thr Ile Arg Thr Lys Tyr Gly
65                  70                  75                  80

Thr Arg Asn Gln Leu Gln Ala Ala Val Asn Ala Leu Lys Ser Asn Gly
                85                  90                  95

Ile Gln Val Tyr Gly Asp Val Val Met Asn His Lys Gly Gly Ala Asp
            100                 105                 110

Ala Thr Glu Met Val Arg Ala Val Glu Val Asn Pro Asn Asn Arg Asn
        115                 120                 125

Gln Glu Val Ser Gly Glu Tyr Thr Ile Glu Ala Trp Thr Lys Phe Asp
    130                 135                 140

Phe Pro Gly Arg Gly Asn Thr His Ser Asn Phe Lys Trp Arg Trp Tyr
145                 150                 155                 160

His Phe Asp Gly Val Asp Trp Asp Gln Ser Arg Lys Leu Asn Asn Arg
                165                 170                 175

Ile Tyr Lys Phe Arg Gly Asp Gly Lys Gly Trp Asp Trp Glu Val Asp
            180                 185                 190

Thr Glu Asn Gly Asn Tyr Asp Tyr Leu Met Tyr Ala Asp Ile Asp Met
        195                 200                 205

Asp His Pro Glu Val Val Asn Glu Leu Arg Asn Trp Gly Val Trp Tyr
    210                 215                 220

Thr Asn Thr Leu Gly Leu Asp Gly Phe Arg Ile Asp Ala Val Lys His
225                 230                 235                 240

Ile Lys Tyr Ser Phe Thr Arg Asp Trp Ile Asn His Val Arg Ser Ala
                245                 250                 255

Thr Gly Lys Asn Met Phe Ala Val Ala Glu Phe Trp Lys Asn Asp Leu
            260                 265                 270

Gly Ala Ile Glu Asn Tyr Leu Asn Lys Thr Asn Trp Asn His Ser Val
        275                 280                 285

Phe Asp Val Pro Leu His Tyr Asn Leu Tyr Asn Ala Ser Lys Ser Gly
    290                 295                 300
```

Gly Asn Tyr Asp Met Arg Gln Ile Phe Asn Gly Thr Val Val Gln Arg
305 310 315 320

His Pro Met His Ala Val Thr Phe Val Asp Asn His Asp Ser Gln Pro
325 330 335

Glu Glu Ala Leu Glu Ser Phe Val Glu Glu Trp Phe Lys Pro Leu Ala
340 345 350

Tyr Ala Leu Thr Leu Thr Arg Glu Gln Gly Tyr Pro Ser Val Phe Tyr
355 360 365

Gly Asp Tyr Tyr Gly Ile Pro Thr His Gly Val Pro Ala Met Lys Ser
370 375 380

Lys Ile Asp Pro Ile Leu Glu Ala Arg Gln Lys Tyr Ala Tyr Gly Arg
385 390 395 400

Gln Asn Asp Tyr Leu Asp His His Asn Ile Ile Gly Trp Thr Arg Glu
405 410 415

Gly Asn Thr Ala His Pro Asn Ser Gly Leu Ala Thr Ile Met Ser Asp
420 425 430

Gly Ala Gly Gly Asn Lys Trp Met Phe Val Gly Arg Asn Lys Ala Gly
435 440 445

Gln Val Trp Thr Asp Ile Thr Gly Asn Arg Ala Gly Thr Val Thr Ile
450 455 460

Asn Ala Asp Gly Trp Gly Asn Phe Ser Val Asn Gly Gly Ser Val Ser
465 470 475 480

Ile Trp Val Asn Lys
485

<210> 7
<211> 501
<212> PRT
<213> Bacillus sp.

<400> 7

Met Lys Arg Trp Val Val Ala Met Leu Ala Val Leu Phe Leu Phe Pro
1 5 10 15

Ser Val Val Val Ala Asp Gly Leu Asn Gly Thr Met Met Gln Tyr Tyr
20 25 30

```
Glu Trp His Leu Glu Asn Asp Gly Gln His Trp Asn Arg Leu His Asp
        35                  40                  45

Asp Ala Glu Ala Leu Ser Asn Ala Gly Ile Thr Ala Ile Trp Ile Pro
    50                  55                  60

Pro Ala Tyr Lys Gly Asn Ser Gln Ala Asp Val Gly Tyr Gly Ala Tyr
65                  70                  75                  80

Asp Leu Tyr Asp Leu Gly Glu Phe Asn Gln Lys Gly Thr Val Arg Thr
                85                  90                  95

Lys Tyr Gly Thr Lys Ala Gln Leu Glu Arg Ala Ile Gly Ser Leu Lys
            100                 105                 110

Ser Asn Asp Ile Asn Val Tyr Gly Asp Val Val Met Asn His Lys Leu
        115                 120                 125

Gly Ala Asp Phe Thr Glu Ala Val Gln Ala Val Gln Val Asn Pro Ser
    130                 135                 140

Asn Arg Trp Gln Asp Ile Ser Gly Val Tyr Thr Ile Asp Ala Trp Thr
145                 150                 155                 160

Gly Phe Asp Phe Pro Gly Arg Asn Asn Ala Tyr Ser Asp Phe Lys Trp
                165                 170                 175

Arg Trp Phe His Phe Asn Gly Val Asp Trp Asp Gln Arg Tyr Gln Glu
            180                 185                 190

Asn His Leu Phe Arg Phe Ala Asn Thr Asn Trp Asn Trp Arg Val Asp
        195                 200                 205

Glu Glu Asn Gly Asn Tyr Asp Tyr Leu Leu Gly Ser Asn Ile Asp Phe
    210                 215                 220

Ser His Pro Glu Val Gln Glu Glu Leu Lys Asp Trp Gly Ser Trp Phe
225                 230                 235                 240

Thr Asp Glu Leu Asp Leu Asp Gly Tyr Arg Leu Asp Ala Ile Lys His
                245                 250                 255

Ile Pro Phe Trp Tyr Thr Ser Asp Trp Val Arg His Gln Arg Ser Glu
            260                 265                 270

Ala Asp Gln Asp Leu Phe Val Val Gly Glu Tyr Trp Lys Asp Asp Val
        275                 280                 285
```

```
Gly Ala Leu Glu Phe Tyr Leu Asp Glu Met Asn Trp Glu Met Ser Leu
    290                 295                 300

Phe Asp Val Pro Leu Asn Tyr Asn Phe Tyr Arg Ala Ser Lys Gln Gly
305                 310                 315                 320

Gly Ser Tyr Asp Met Arg Asn Ile Leu Arg Gly Ser Leu Val Glu Ala
                325                 330                 335

His Pro Ile His Ala Val Thr Phe Val Asp Asn His Asp Thr Gln Pro
            340                 345                 350

Gly Glu Ser Leu Glu Ser Trp Val Ala Asp Trp Phe Lys Pro Leu Ala
        355                 360                 365

Tyr Ala Thr Ile Leu Thr Arg Glu Gly Gly Tyr Pro Asn Val Phe Tyr
    370                 375                 380

Gly Asp Tyr Tyr Gly Ile Pro Asn Asp Asn Ile Ser Ala Lys Lys Asp
385                 390                 395                 400

Met Ile Asp Glu Leu Leu Asp Ala Arg Gln Asn Tyr Ala Tyr Gly Thr
                405                 410                 415

Gln His Asp Tyr Phe Asp His Trp Asp Ile Val Gly Trp Thr Arg Glu
            420                 425                 430

Gly Thr Ser Ser Arg Pro Asn Ser Gly Leu Ala Thr Ile Met Ser Asn
        435                 440                 445

Gly Pro Gly Gly Ser Lys Trp Met Tyr Val Gly Gln Gln His Ala Gly
    450                 455                 460

Gln Thr Trp Thr Asp Leu Thr Gly Asn His Ala Ala Ser Val Thr Ile
465                 470                 475                 480

Asn Gly Asp Gly Trp Gly Glu Phe Phe Thr Asn Gly Gly Ser Val Ser
                485                 490                 495

Val Tyr Val Asn Gln
            500
```

<210> 8
<211> 269
<212> PRT
<213> Bacillus lentus

<400> 8

```
Ala Gln Ser Val Pro Trp Gly Ile Ser Arg Val Gln Ala Pro Ala Ala
1               5                   10                  15

His Asn Arg Gly Leu Thr Gly Ser Gly Val Lys Val Ala Val Leu Asp
            20                  25                  30

Thr Gly Ile Ser Thr His Pro Asp Leu Asn Ile Arg Gly Gly Ala Ser
        35                  40                  45

Phe Val Pro Gly Glu Pro Ser Thr Gln Asp Gly Asn Gly His Gly Thr
    50                  55                  60

His Val Ala Gly Thr Ile Ala Ala Leu Asn Asn Ser Ile Gly Val Leu
65                  70                  75                  80

Gly Val Ala Pro Ser Ala Glu Leu Tyr Ala Val Lys Val Leu Gly Ala
                85                  90                  95

Ser Gly Ser Gly Ser Val Ser Ser Ile Ala Gln Gly Leu Glu Trp Ala
            100                 105                 110

Gly Asn Asn Gly Met His Val Ala Asn Leu Ser Leu Gly Ser Pro Ser
        115                 120                 125

Pro Ser Ala Thr Leu Glu Gln Ala Val Asn Ser Ala Thr Ser Arg Gly
    130                 135                 140

Val Leu Val Val Ala Ala Ser Gly Asn Ser Gly Ala Gly Ser Ile Ser
145                 150                 155                 160

Tyr Pro Ala Arg Tyr Ala Asn Ala Met Ala Val Gly Ala Thr Asp Gln
                165                 170                 175

Asn Asn Asn Arg Ala Ser Phe Ser Gln Tyr Gly Ala Gly Leu Asp Ile
            180                 185                 190

Val Ala Pro Gly Val Asn Val Gln Ser Thr Tyr Pro Gly Ser Thr Tyr
        195                 200                 205

Ala Ser Leu Asn Gly Thr Ser Met Ala Thr Pro His Val Ala Gly Ala
    210                 215                 220

Ala Ala Leu Val Lys Gln Lys Asn Pro Ser Trp Ser Asn Val Gln Ile
225                 230                 235                 240

Arg Asn His Leu Lys Asn Thr Ala Thr Ser Leu Gly Ser Thr Asn Leu
                245                 250                 255
```

```
Tyr Gly Ser Gly Leu Val Asn Ala Glu Ala Ala Thr Arg
            260                 265
```

<210> 9
<211> 382
<212> PRT
<213> Bacillus sp.

<400> 9

```
Asp Val Val Met Asn His Lys Gly Gly Ala Asp Ala Thr Glu Met Val
1               5                   10                  15

Arg Ala Val Glu Val Asn Pro Asn Asn Arg Asn Gln Glu Val Thr Gly
            20                  25                  30

Glu Tyr Thr Ile Glu Ala Trp Thr Arg Phe Asp Phe Pro Gly Arg Gly
        35                  40                  45

Asn Thr His Ser Ser Phe Lys Trp Arg Trp Tyr His Phe Asp Gly Val
    50                  55                  60

Asp Trp Asp Gln Ser Arg Arg Leu Asn Asn Arg Ile Tyr Lys Phe Arg
65                  70                  75                  80

Gly Lys Ala Trp Asp Trp Glu Val Asp Thr Glu Asn Gly Asn Tyr Asp
                85                  90                  95

Tyr Leu Met Tyr Ala Asp Ile Asp Met Asp His Pro Glu Val Val Asn
            100                 105                 110

Glu Leu Arg Asn Trp Gly Val Trp Tyr Thr Asn Thr Leu Gly Leu Asp
        115                 120                 125

Gly Phe Arg Ile Asp Ala Val Lys His Ile Lys Tyr Ser Phe Thr Arg
    130                 135                 140

Asp Trp Ile Asn His Val Arg Ser Ala Thr Gly Lys Asn Met Phe Ala
145                 150                 155                 160

Val Ala Glu Phe Trp Lys Asn Asp Leu Gly Ala Ile Glu Asn Tyr Leu
                165                 170                 175

Gln Lys Thr Asn Trp Asn His Ser Val Phe Asp Val Pro Leu His Tyr
            180                 185                 190

Asn Leu Tyr Asn Ala Ser Lys Ser Gly Gly Asn Tyr Asp Met Arg Asn
        195                 200                 205
```

```
Ile Phe Asn Gly Thr Val Val Gln Arg His Pro Ser His Ala Val Thr
    210                 215                 220

Phe Val Asp Asn His Asp Ser Gln Pro Glu Glu Ala Leu Glu Ser Phe
225                 230                 235                 240

Val Glu Glu Trp Phe Lys Pro Leu Ala Tyr Ala Leu Thr Leu Thr Arg
                245                 250                 255

Glu Gln Gly Tyr Pro Ser Val Phe Tyr Gly Asp Tyr Tyr Gly Ile Pro
            260                 265                 270

Thr His Gly Val Pro Ala Met Arg Ser Lys Ile Asp Pro Ile Leu Glu
        275                 280                 285

Ala Arg Gln Lys Tyr Ala Tyr Gly Pro Gln His Asp Tyr Leu Asp His
    290                 295                 300

Pro Asp Val Ile Gly Trp Thr Arg Glu Gly Asp Ser Ser His Pro Lys
305                 310                 315                 320

Ser Gly Leu Ala Thr Leu Ile Thr Asp Gly Pro Gly Gly Ser Lys Arg
                325                 330                 335

Met Tyr Ala Gly Leu Lys Asn Ala Gly Glu Thr Trp Tyr Asp Ile Thr
            340                 345                 350

Gly Asn Arg Ser Asp Thr Val Lys Ile Gly Ser Asp Gly Trp Gly Glu
        355                 360                 365

Phe His Val Asn Asp Gly Ser Val Ser Ile Tyr Val Gln Lys
    370                 375                 380
```

<210> 10
<211> 480
<212> PRT
<213> Bacillus sp.

<400> 10

```
Asp Gly Leu Asn Gly Thr Met Met Gln Tyr Tyr Glu Trp His Leu Glu
1               5                   10                  15

Asn Asp Gly Gln His Trp Asn Arg Leu His Asp Asp Ala Ala Ala Leu
            20                  25                  30

Ser Asp Ala Gly Ile Thr Ala Ile Trp Ile Pro Pro Ala Tyr Lys Gly
        35                  40                  45
```

```
Asn Ser Gln Ala Asp Val Gly Tyr Gly Ala Tyr Asp Leu Tyr Asp Leu
    50                  55                  60

Gly Glu Phe Asn Gln Lys Gly Thr Val Arg Thr Lys Tyr Gly Thr Lys
65                  70                  75                  80

Ala Gln Leu Glu Arg Ala Ile Gly Ser Leu Lys Ser Asn Asp Ile Asn
                85                  90                  95

Val Tyr Gly Asp Val Val Met Asn His Lys Met Gly Ala Asp Phe Thr
            100                 105                 110

Glu Ala Val Gln Ala Val Gln Val Asn Pro Thr Asn Arg Trp Gln Asp
        115                 120                 125

Ile Ser Gly Ala Tyr Thr Ile Asp Ala Trp Thr Gly Phe Asp Phe Ser
    130                 135                 140

Gly Arg Asn Asn Ala Tyr Ser Asp Phe Lys Trp Arg Trp Phe His Phe
145                 150                 155                 160

Asn Gly Val Asp Trp Asp Gln Arg Tyr Gln Glu Asn His Ile Phe Arg
                165                 170                 175

Phe Ala Asn Thr Asn Trp Asn Trp Arg Val Asp Glu Glu Asn Gly Asn
            180                 185                 190

Tyr Asp Tyr Leu Leu Gly Ser Asn Ile Asp Phe Ser His Pro Glu Val
        195                 200                 205

Gln Asp Glu Leu Lys Asp Trp Gly Ser Trp Phe Thr Asp Glu Leu Asp
    210                 215                 220

Leu Asp Gly Tyr Arg Leu Asp Ala Ile Lys His Ile Pro Phe Trp Tyr
225                 230                 235                 240

Thr Ser Asp Trp Val Arg His Gln Arg Asn Glu Ala Asp Gln Asp Leu
                245                 250                 255

Phe Val Val Gly Glu Tyr Trp Lys Asp Asp Val Gly Ala Leu Glu Phe
            260                 265                 270

Tyr Leu Asp Glu Met Asn Trp Glu Met Ser Leu Phe Asp Val Pro Leu
        275                 280                 285

Asn Tyr Asn Phe Tyr Arg Ala Ser Gln Gln Gly Gly Ser Tyr Asp Met
```

```
    290                 295                 300

Arg Asn Ile Leu Arg Gly Ser Leu Val Glu Ala His Pro Met His Ala
305                 310                 315                 320

Val Thr Phe Val Asp Asn His Asp Thr Gln Pro Gly Glu Ser Leu Glu
                325                 330                 335

Ser Trp Val Ala Asp Trp Phe Lys Pro Leu Ala Tyr Ala Thr Ile Leu
            340                 345                 350

Thr Arg Glu Gly Gly Tyr Pro Asn Val Phe Tyr Gly Asp Tyr Tyr Gly
        355                 360                 365

Ile Pro Asn Asp Asn Ile Ser Ala Lys Lys Asp Met Ile Asp Glu Leu
    370                 375                 380

Leu Asp Ala Arg Gln Asn Tyr Ala Tyr Gly Thr Gln His Asp Tyr Phe
385                 390                 395                 400

Asp His Trp Asp Val Val Gly Trp Thr Arg Glu Gly Ser Ser Ser Arg
                405                 410                 415

Pro Asn Ser Gly Leu Ala Thr Ile Met Ser Asn Gly Pro Gly Gly Ser
            420                 425                 430

Lys Trp Met Tyr Val Gly Arg Gln Asn Ala Gly Gln Thr Trp Thr Asp
        435                 440                 445

Leu Thr Gly Asn Asn Gly Ala Ser Val Thr Ile Asn Gly Asp Gly Trp
    450                 455                 460

Gly Glu Phe Phe Thr Asn Gly Gly Ser Val Ser Val Tyr Val Asn Gln
465                 470                 475                 480
```

<210> 11
<211> 485
<212> PRT
<213> Bacillus sp.

<400> 11

```
His His Asn Gly Thr Asn Gly Thr Met Met Gln Tyr Phe Glu Trp His
1               5                   10                  15

Leu Pro Asn Asp Gly Asn His Trp Asn Arg Leu Arg Asp Asp Ala Ala
            20                  25                  30

Asn Leu Lys Ser Lys Gly Ile Thr Ala Val Trp Ile Pro Pro Ala Trp
```

35 40 45

Lys Gly Thr Ser Gln Asn Asp Val Gly Tyr Gly Ala Tyr Asp Leu Tyr
50 55 60

Asp Leu Gly Glu Phe Asn Gln Lys Gly Thr Val Arg Thr Lys Tyr Gly
65 70 75 80

Thr Arg Ser Gln Leu Gln Gly Ala Val Thr Ser Leu Lys Asn Asn Gly
85 90 95

Ile Gln Val Tyr Gly Asp Val Val Met Asn His Lys Gly Gly Ala Asp
100 105 110

Gly Thr Glu Met Val Asn Ala Val Glu Val Asn Arg Ser Asn Arg Asn
115 120 125

Gln Glu Ile Ser Gly Glu Tyr Thr Ile Glu Ala Trp Thr Lys Phe Asp
130 135 140

Phe Pro Gly Arg Gly Asn Thr His Ser Asn Phe Lys Trp Arg Trp Tyr
145 150 155 160

His Phe Asp Gly Thr Asp Trp Asp Gln Ser Arg Gln Leu Gln Asn Lys
165 170 175

Ile Tyr Lys Phe Arg Gly Thr Gly Lys Ala Trp Asp Trp Glu Val Asp
180 185 190

Ile Glu Asn Gly Asn Tyr Asp Tyr Leu Met Tyr Ala Asp Ile Asp Met
195 200 205

Asp His Pro Glu Val Ile Asn Glu Leu Arg Asn Trp Gly Val Trp Tyr
210 215 220

Thr Asn Thr Leu Asn Leu Asp Gly Phe Arg Ile Asp Ala Val Lys His
225 230 235 240

Ile Lys Tyr Ser Tyr Thr Arg Asp Trp Leu Thr His Val Arg Asn Thr
245 250 255

Thr Gly Lys Pro Met Phe Ala Val Ala Glu Phe Trp Lys Asn Asp Leu
260 265 270

Ala Ala Ile Glu Asn Tyr Leu Asn Lys Thr Ser Trp Asn His Ser Val
275 280 285

Phe Asp Val Pro Leu His Tyr Asn Leu Tyr Asn Ala Ser Asn Ser Gly
290 295 300

Gly Tyr Phe Asp Met Arg Asn Ile Leu Asn Gly Ser Val Val Gln Lys
305 310 315 320

His Pro Ile His Ala Val Thr Phe Val Asp Asn His Asp Ser Gln Pro
325 330 335

Gly Glu Ala Leu Glu Ser Phe Val Gln Ser Trp Phe Lys Pro Leu Ala
340 345 350

Tyr Ala Leu Ile Leu Thr Arg Glu Gln Gly Tyr Pro Ser Val Phe Tyr
355 360 365

Gly Asp Tyr Tyr Gly Ile Pro Thr His Gly Val Pro Ser Met Lys Ser
370 375 380

Lys Ile Asp Pro Leu Leu Gln Ala Arg Gln Thr Tyr Ala Tyr Gly Thr
385 390 395 400

Gln His Asp Tyr Phe Asp His His Asp Ile Ile Gly Trp Thr Arg Glu
405 410 415

Gly Asp Ser Ser His Pro Asn Ser Gly Leu Ala Thr Ile Met Ser Asp
420 425 430

Gly Pro Gly Gly Asn Lys Trp Met Tyr Val Gly Lys His Lys Ala Gly
435 440 445

Gln Val Trp Arg Asp Ile Thr Gly Asn Arg Ser Gly Thr Val Thr Ile
450 455 460

Asn Ala Asp Gly Trp Gly Asn Phe Thr Val Asn Gly Gly Ala Val Ser
465 470 475 480

Val Trp Val Lys Gln
485

<210> 12
<211> 485
<212> PRT
<213> Bacillus sp.

<400> 12

His His Asn Gly Thr Asn Gly Thr Met Met Gln Tyr Phe Glu Trp Tyr
1 5 10 15

```
Leu Pro Asn Asp Gly Asn His Trp Asn Arg Leu Arg Ser Asp Ala Ser
            20                  25                  30

Asn Leu Lys Asp Lys Gly Ile Thr Ala Val Trp Ile Pro Pro Ala Trp
        35                  40                  45

Lys Gly Ala Ser Gln Asn Asp Val Gly Tyr Gly Ala Tyr Asp Leu Tyr
    50                  55                  60

Asp Leu Gly Glu Phe Asn Gln Lys Gly Thr Val Arg Thr Lys Tyr Gly
65                  70                  75                  80

Thr Arg Asn Gln Leu Gln Ala Ala Val Thr Ala Leu Lys Ser Asn Gly
                85                  90                  95

Ile Gln Val Tyr Gly Asp Val Val Met Asn His Lys Gly Gly Ala Asp
            100                 105                 110

Ala Thr Glu Trp Val Arg Ala Val Glu Val Asn Pro Ser Asn Arg Asn
        115                 120                 125

Gln Glu Val Ser Gly Asp Tyr Thr Ile Glu Ala Trp Thr Lys Phe Asp
    130                 135                 140

Phe Pro Gly Arg Gly Asn Thr His Ser Asn Phe Lys Trp Arg Trp Tyr
145                 150                 155                 160

His Phe Asp Gly Val Asp Trp Asp Gln Ser Arg Gln Leu Gln Asn Arg
                165                 170                 175

Ile Tyr Lys Phe Arg Gly Asp Gly Lys Gly Trp Asp Trp Glu Val Asp
            180                 185                 190

Thr Glu Asn Gly Asn Tyr Asp Tyr Leu Met Tyr Ala Asp Ile Asp Met
        195                 200                 205

Asp His Pro Glu Val Val Asn Glu Leu Arg Asn Trp Gly Val Trp Tyr
    210                 215                 220

Thr Asn Thr Leu Gly Leu Asp Gly Phe Arg Ile Asp Ala Val Lys His
225                 230                 235                 240

Ile Lys Tyr Ser Phe Thr Arg Asp Trp Leu Thr His Val Arg Asn Thr
                245                 250                 255

Thr Gly Lys Asn Met Phe Ala Val Ala Glu Phe Trp Lys Asn Asp Ile
            260                 265                 270
```

Gly Ala Ile Glu Asn Tyr Leu Ser Lys Thr Asn Trp Asn His Ser Val
275 280 285

Phe Asp Val Pro Leu His Tyr Asn Leu Tyr Asn Ala Ser Arg Ser Gly
290 295 300

Gly Asn Tyr Asp Met Arg Gln Ile Phe Asn Gly Thr Val Val Gln Arg
305 310 315 320

His Pro Thr His Ala Val Thr Phe Val Asp Asn His Asp Ser Gln Pro
325 330 335

Glu Glu Ala Leu Glu Ser Phe Val Glu Glu Trp Phe Lys Pro Leu Ala
340 345 350

Cys Ala Leu Thr Leu Thr Arg Asp Gln Gly Tyr Pro Ser Val Phe Tyr
355 360 365

Gly Asp Tyr Tyr Gly Ile Pro Thr His Gly Val Pro Ala Met Lys Ser
370 375 380

Lys Ile Asp Pro Ile Leu Glu Ala Arg Gln Lys Tyr Ala Tyr Gly Lys
385 390 395 400

Gln Asn Asp Tyr Leu Asp His His Asn Met Ile Gly Trp Thr Arg Glu
405 410 415

Gly Asn Thr Ala His Pro Asn Ser Gly Leu Ala Thr Ile Met Ser Asp
420 425 430

Gly Pro Gly Gly Asn Lys Trp Met Tyr Val Gly Arg Asn Lys Ala Gly
435 440 445

Gln Val Trp Arg Asp Ile Thr Gly Asn Arg Ser Gly Thr Val Thr Ile
450 455 460

Asn Ala Asp Gly Trp Gly Asn Phe Ser Val Asn Gly Gly Ser Val Ser
465 470 475 480

Ile Trp Val Asn Asn
485

<210> 13
<211> 485
<212> PRT
<213> Bacillus sp.

<400> 13

His His Asp Gly Thr Asn Gly Thr Ile Met Gln Tyr Phe Glu Trp Asn
1 5 10 15

Val Pro Asn Asp Gly Gln His Trp Asn Arg Leu His Asn Asn Ala Gln
20 25 30

Asn Leu Lys Asn Ala Gly Ile Thr Ala Ile Trp Ile Pro Pro Ala Trp
35 40 45

Lys Gly Thr Ser Gln Asn Asp Val Gly Tyr Gly Ala Tyr Asp Leu Tyr
50 55 60

Asp Leu Gly Glu Phe Asn Gln Lys Gly Thr Val Arg Thr Lys Tyr Gly
65 70 75 80

Thr Lys Ala Glu Leu Glu Arg Ala Ile Arg Ser Leu Lys Ala Asn Gly
85 90 95

Ile Gln Val Tyr Gly Asp Val Val Met Asn His Lys Gly Gly Ala Asp
100 105 110

Phe Thr Glu Arg Val Gln Ala Val Glu Val Asn Pro Gln Asn Arg Asn
115 120 125

Gln Glu Val Ser Gly Thr Tyr Glu Ile Glu Ala Trp Thr Gly Phe Asn
130 135 140

Phe Pro Gly Arg Gly Asn Gln His Ser Ser Phe Lys Trp Arg Trp Tyr
145 150 155 160

His Phe Asp Gly Thr Asp Trp Asp Gln Ser Arg Gln Leu Ser Asn Arg
165 170 175

Ile Tyr Lys Phe Arg Gly Asp Gly Lys Ala Trp Asp Trp Glu Val Asp
180 185 190

Thr Glu Asn Gly Asn Tyr Asp Tyr Leu Met Tyr Ala Asp Val Asp Met
195 200 205

Asn His Pro Glu Val Ile Asn Glu Leu Asn Arg Trp Gly Val Trp Tyr
210 215 220

Ala Asn Thr Leu Asn Leu Asp Gly Phe Arg Leu Asp Ala Val Lys His
225 230 235 240

Ile Gln Phe Ser Phe Met Arg Asn Trp Leu Gly His Val Arg Gly Gln
245 250 255

Thr Gly Lys Asn Leu Phe Ala Val Ala Glu Tyr Trp Lys Asn Asp Leu
260 265 270

Gly Ala Leu Glu Asn Tyr Leu Ser Lys Thr Asn Trp Thr Met Ser Ala
275 280 285

Phe Asp Val Pro Leu His Tyr Asn Leu Tyr Gln Ala Ser Asn Ser Gly
290 295 300

Gly Asn Tyr Asp Met Arg Asn Leu Leu Asn Gly Thr Leu Val Gln Arg
305 310 315 320

His Pro Ser His Ala Val Thr Phe Val Asp Asn His Asp Thr Gln Pro
325 330 335

Gly Glu Ala Leu Glu Ser Phe Val Gln Gly Trp Phe Lys Pro Leu Ala
340 345 350

Tyr Ala Thr Ile Leu Thr Arg Glu Gln Gly Tyr Pro Gln Val Phe Tyr
355 360 365

Gly Asp Tyr Tyr Gly Ile Pro Ser Asp Gly Val Pro Ser Tyr Arg Gln
370 375 380

Gln Ile Asp Pro Leu Leu Lys Ala Arg Gln Gln Tyr Ala Tyr Gly Arg
385 390 395 400

Gln His Asp Tyr Phe Asp His Trp Asp Val Ile Gly Trp Thr Arg Glu
405 410 415

Gly Asn Ala Ser His Pro Asn Ser Gly Leu Ala Thr Ile Met Ser Asp
420 425 430

Gly Pro Gly Gly Ser Lys Trp Met Tyr Val Gly Arg Gln Lys Ala Gly
435 440 445

Glu Val Trp His Asp Ile Thr Gly Asn Arg Ser Gly Thr Val Thr Ile
450 455 460

Asn Gln Asp Gly Trp Gly Gln Phe Phe Val Asn Gly Gly Ser Val Ser
465 470 475 480

Val Trp Val Lys Arg
485